# EUROPEAN PATENT SPECIFICATION

(11) **EP 0 354 297 B1**
(45) Date of publication and mention of the grant of the patent: **05.08.1992**
(21) Application number: 89105133.6
(22) Date of filing: 22.03.1989
(51) Int. Cl.: C07D 285/34, C07D 417/12, A01N 43/88

(54) **Thiadiazines, process for production thereof, and insecticidal and acaricidal agents comprising the thiadiazines**
Thiadiazine, Verfahren zu ihrer Herstellung und diese enthaltende insektizide und acarizide Mittel
Thiadiazines, procédé pour leur préparation et agents insecticides et acaricides les contenant

(30) Priority: 08.07.1988 JP 168997/88
(43) Date of publication of application: 14.02.1990
(73) Proprietor: MITSUI TOATSU CHEMICALS, Inc., Chiyoda-Ku Tokyo 100 (JP)
(72) Inventor: Nakaya, Michihiko, Zushi-shi Kanagawa-ken (JP); Fukushi, Yukiharu, Nishi 13, Kita-ku, Sapporo-shi, Hokkaido (JP); Shiraishi, Shirou, Totsuka-ku, Yokohama-shi, Kanagawa-ken (JP); Nakamura, Masahiko, Sakae-ku, Yokohama-shi, Kanagawa-ken (JP); Numata, Satoshi, Totsuka-ku, Yokohama-shi, Kanagawa-ken (JP); Kodaka, Kenji, Sakae-ku, Yokohama-shi, Kanagawa-ken (JP); Ooka, Masayuki, c/o Sogokenkyujo, Sakae-ku, Yokohama-shi, Kanagawa-ken (JP)
(74) Representative: Zumstein, Fritz, Dr.

(56) References cited:
- EP-A- 0 308 961
- DE-A- 2 824 126
- FR-A- 2 512 023
- CHEMICAL ABSTRACTS, vol. 93, no. 9, 1st September 1980, page 193, column 2, abstract no. 90206n, Columbus, Ohio, USA
- CHEMICAL ABSTRACTS, vol. 97, no. 1, 5th July 1982, page 239, column 1, abstract no. 2276w, Columbus, Ohio, USA

## Description

This invention relates to tetrahydro-1,3,5-thiadiazin-4-ones of general formula (I) as explained below or salts thereof; a process for production thereof; and to an insecticidal and acaricidal composition comprising at least one of these compounds as an active ingredient.

The compounds of this invention are useful in various industrial fields, and particularly in the agricultural field as an insecticidal and acaricidal agent.

Japanese Laid-Open Patent Publications Nos. 3083/1979, 12890/1979 and 154780/1979 state that tet- rahydro-1,3,5-thiadiazin-4-ones have insecticidal and acaricidal activities.

Among them, 2-tertiary butylimino-3-isopropyl-5-phenyl-tetrahydro-1,3,5-thiadiazin-4-one (common name: Buprofezin) represented by the following formula (IV) 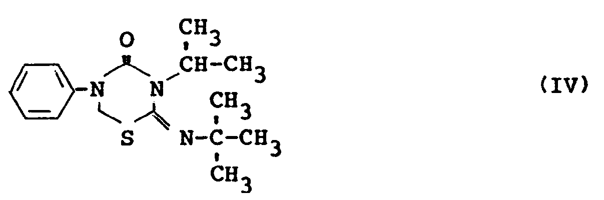 has been put to practical use as an insecticide.

Japanese Laid-Open Patent Publication No. 140577/1986 discloses that tetrahydro-1,3,5-thiadiazin-4- ones in which the 2-imino group or at least one of the 3- and 5-positions is substituted by a certain substituted phenylalkyl group are novel compounds having insecticidal and acaricidal activities, and particularly, tetrahyd- ro-1,3,5-thiadiazin-4-ones in which at least one of the 2-imino group and the 3-position is substituted by a substituted phenylalkyl group have marked insecticidal and acaricidal activities over the known compound Buprofezin. These insecticidal and acaricidal compounds, however, have no sufficient insecticidal activity on lepidopterous pests although they do have insecticidal activity on hemipterous and coleopterous insect pests. It has been desired therefore to develop a novel agent having similar activity to these insecticidal and acaricidal compounds and outstanding insecticidal and acaricidal activities on lepidopterous pests as well. Further, tet- rahydro-1,3,5-thiadizin-4-one derivatives with insecticidal or acaricidal activity are disclosed in DE-A-28 24 126, FR-A-2 512 023, CA 93 (1980) 90206u, CA 97 (1982) 2276w and EP-A-308 961 (to be considered unter Article 54(3) EPC).

It is an object of this invention to provide an excellent insecticidal and acaricidal compound having a new structure, a broad insecticidal spectrum and high insecticidal and acaricidal activities and being free from the problems of the prior art, an insecticidal and acaricidal composition , and a simple process for producing the insecticidal compound.

The present inventors made extensive investigations on tetrahydro-1,3,5-thiadiazin-4-ones in order to achieve the above object, and have now found that 2-(2,2,2-trifluoroethylimino)-tetrahydro-1,3,5-thiadiazin-4- ones or salts thereof have a broad insecticidal spectrum and exhibit high insecticidal activity also on lepidopterous pests on which known analogous compounds do not show sufficient insecticidal activity.

The present invention provides tetrahydro-1,3,5-thiadiazin-4-ones of the following general formula (I) 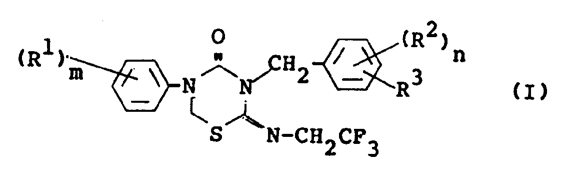 wherein each of R¹ and R² represents a halogen atom or an alkyl group having 1 to 4 carbon atoms, R³ represents a haloalkyloxy group having 1 to 4 carbon atoms, a haloalkenyloxy group having 2 to 4 carbon atoms, a haloalkyltio group having 1 to 4 carbon atoms, a haloalkyl group having 2 to 8 carbon atoms, a haloalkenyl group having 2 to 8 carbon atoms, a alkyloxycarbonyl group having 2 to 8 carbon atoms, phenoxycarbonyl, 2-chlorophenoxycarbonyl, 3-chlorophenoxycarbonyl, 4-chlorophenoxycarbonyl, 2-fluorophenoxycarbonyl, 3-fluorophenoxycarbonyl, 4-fluorophenoxycarbonyl, 2,4-dichlorophenoxycarbonyl, 3,4-dichlorophenoxycarbonyl, 3,5-dichlorophenoxycarbonyl, 2,4-difluorophenoxycarbonyl, 2,6-difluorophenoxycarbonyl, 4-trifiuoromethyi- phenoxycarbonyl, 4-trifluoromethoxyphenoxycarbonyl, 4-methoxyphenoxycarbonyl, 2-methylphenoxycarbonyl, 3-methylphenoxycarbonyl, 4-methylphenoxycarbonyl, 2,4-dimethylphenoxycarbonyl, 4-t-butylphenoxycarbonyl, 3-chloro-pyridyloxy, 3,5-dichloro-2-pyridyloxy, 5-trifluoromethyl-pyridyloxy and 3-chloro-5-tri-fluoromethyl-pyridyloxy groups at 4-position m is 0, 1, 2 or 3, and n is 0, 1, 2 or 3, or a salt thereof.

The present invention also provides a process for producing the tetrahydro-1,3,5-thiadiazin-4-ones of formula (I) or salts thereof, which comprises reacting a compound of the following general formula (II) 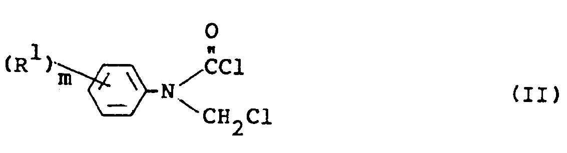 wherein R¹ and m are as defined above, with a compound of the following general formula (III) 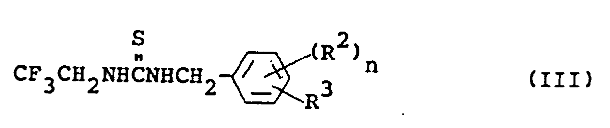 wherein R², R³ and n are as defined above.

The invention additionally provides an insecticidal and acaricidal composition comprising at least one tet- rahydro-1,3,5-thiadiazin-4-one of formula (I) or a salt thereof as an active ingredient.

The tetrahydro-1,3,5-thiadiazin-4-ones of general formula (I) and their salts provided by this invention are not described in the literature, and are novel compounds.

In the compounds of this invention represented by general formula (I), when the benzyl group at the 3- position of the thiadiazine ring has two substituents, they are preferably substituted at the 3,4-positions of the benzyl group. The 3-position substituent is preferably a halogen atom, particularly a fluorine or chlorine atom.

When the 4-position substituent is a haloalkyloxy group, the halogen atom is preferably a bromine, chlorine or fluorine atom. The fluorine atom is especially preferred. Preferred as the haloalkyloxy group are dif- luoromethoxy, trifluoromethoxy, chlorodifluoromethoxy, bromodifluoromethoxy, 2,2,2-trifluoroethoxy, 1,1,2,2-tetrafluoroethoxy, 2,2,3,3-tetrafluoropropyloxy and 2,2,3,3,4,4,4-hexafluorobutoxy groups. The trifluoromethoxy group is especially preferred.

When the 4-position substituent is a haloalkenyloxy group, the halogen atom is preferably a bromine, chlorine or fluorine atom. The fluorine and chlorine atoms are especially preferred. Examples of preferred haloalkenyloxy groups include 1,2,2-trichlorovinyloxy, 2,2-dichloro-1-fluorovinyloxy, 2-chloro-2-propeneoxy, 2-bromo-2-propeneoxy, 2,3-dichloro-2-propeneoxy, 3-chloro-2-n-buteneoxy and 3-chloro-3-buteneoxy groups. The 1,2,2-trichlorovinyloxy and 2-chloro-2-propeneoxy groups are especially preferred.

When the 4-position substituent is a haloalkylthio group, the halogen atom is preferably a bromine, chlorine or fluorine atom, and the fluorine atom is especially preferred. Examples of preferred haloalkylthio groups include difluoromethylthio, trifluoromethylthio, chlorodifluoromethylthio, bromodifluoromethyltbio, 2,2,2-trif- luoroethylthio, 1,1,2,2-tetrafluoroethylthio, 2,2,3,3-tetrafluoropropylthio and 2,2,3,3,4,4,4-hexafluorobutylthio groups. The trifluoromethylthio group is especially preferred.

When the 4-position substituent is a haloalkyl group, the halogen atom is preferably a bromine, chlorine or fluorine atom, the fluorine atom being especially preferred. Examples of preferred haloalkyl groups include pentafluoroethyl, n-heptafluoropropyl, n-tridecafluorohexyl, 2,2,2-trifluoroethyl and 2,2-bis-(trifluoromethyl)-3,3,4,4,5,5,5-heptafluoropentyl groups. The pentafluoroethyl group is especially preferred.

When the 4-position substituent is an alkyloxycarbonyl group, examples of preferred alkyloxycarbonyl groups include methoxycarbonyl, ethoxycarbonyl, n-propoxycarbonyl, iso-propoxycarbonyl, n-butoxycarbonyl, iso-butoxycarbonyl, sec-butoxycarbonyl, t-butoxycarbonyl, n-pentyloxycarbonyl, iso-pentyloxycarbonyl, n-hexyloxycarbonyl, 2-methylpentyloxycarbonyl, 3-methylpentyloxycarbonyl, n-heptyloxycarbonyl, t-heptyloxycarbonyl and n-octyloxycarbonyl groups. The t-butoxycarbonyl group is especially preferred.

When the 4-position substituent is one of the above mentioned substituted phenoxycarbonyl groups, the 2,4-dichlorophenoxycarbonyl group is especially preferred.

When the 4-position is one of the above mentioned substituted pyridyloxy groups, the 3-chloro-5-trifluoromethyl-pyridyloxy group is especially preferred.

When the phenyl group at the 5-position of the thiadiazine ring has one substituent, it may be a halogen atom which is preferably a bromine, chlorine or fluorine atom, especially preferably the fluorine atom. The fluorine is preferably substituted at the 2-position. The substituent may also be an alkyl group, preferably a methyl, ethyl, isopropyl or t-butyl group, especially preferably a methyl group. The methyl group is preferably substituted at the 3- or 4-position.

When the phenyl group at the 5-position has two substituents, they may be halogen atoms, preferably a bromine, chlorine orfluorine atom, especially preferably the fluorine atom, and/oralkyl groups such as a methyl, ethyl, isopropyl or t-butyl group, especially preferably the methyl group. The two substituents at the phenyl group are preferably substituted at the 2,4-positions, 3,4-positions or 2,6-positions. The 2-position substituent is preferably a fluorine atom. The 3-position substituent is preferably a methyl group. The 4-position substituent is preferably a methyl group. The 6-position substituent is preferably a fluorine atom. When the 5-position phenyl group has 3 substituents, they may be halogen atoms, preferably a bromine, chlorine or fluorine atom, especially preferably the fluorine atom, and/or alkyl groups such as a methyl, ethyl, isopropyl or t-butyl group, especially preferably the methyl group. The 3 substituents are preferably substituted at the 2,4,6-positions or 3,4,6-positions of the phenyl group. The 2-position substituent is preferably a fluorine atom. The 3-position substituent is preferably a methyl group. The 4-position substituent is preferably a methyl group. The 6-position substituent is preferably a fluorine atom.

Examples of the salts of the compounds of the invention represented by general formula (I) include their inorganic acid salts such as hydrochlorides, hydrobromides, hydroiodides, hydrofluorides, sulfates, hydrogen- sulfates, nitrates, chlorates, perchlorates, phosphates, hydrogen phosphates, dihydrogenphosphates, thiocyanates and tetrafluoroborates, and organic acid salts such as formates, acetates, trichlroacetates, trifluoroacetates, citrates, lactates, oxalates, glycollates, malonates, succinates, malates, dodecylbenzenesulfo- nates, benzoates, salicylates and nicotinates.

Typical examples of the compounds of the invention represented by general formula (I) are shown in Table 1 without any intention of limiting the invention thereto.

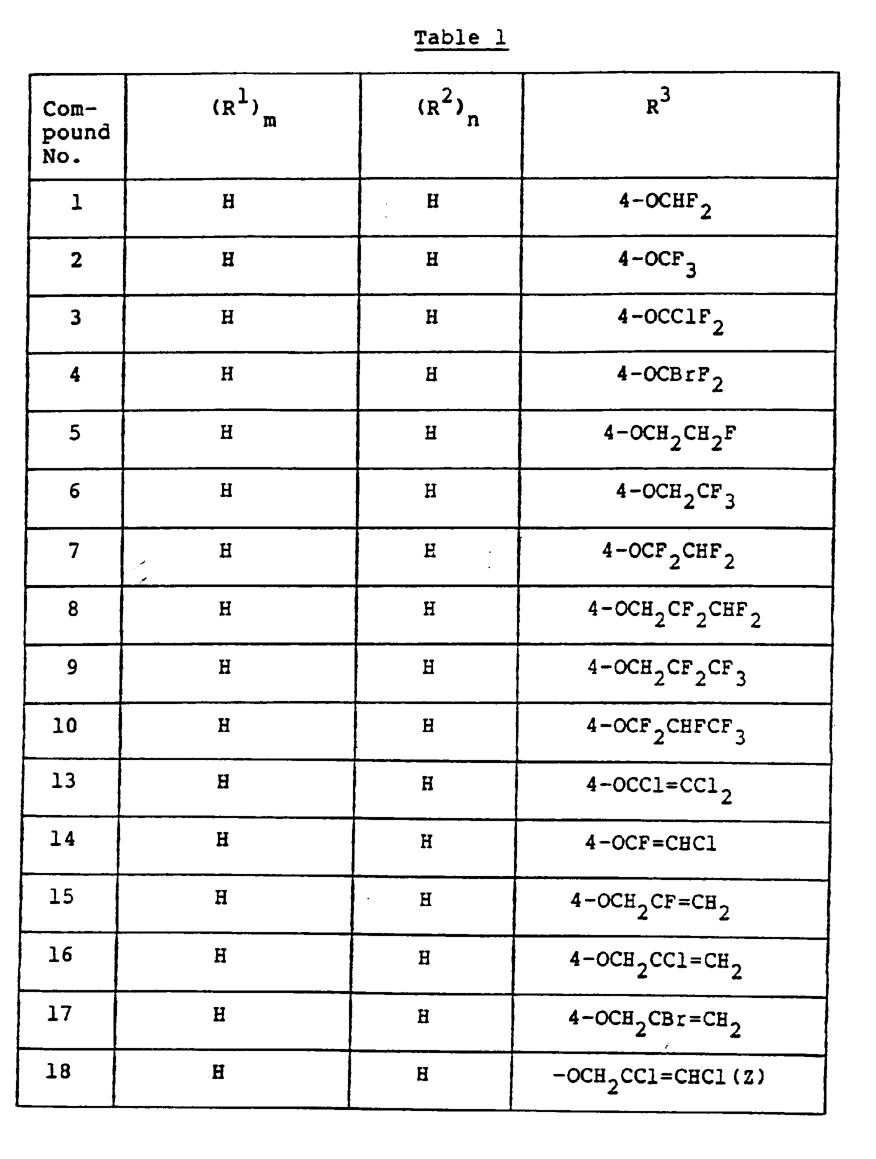

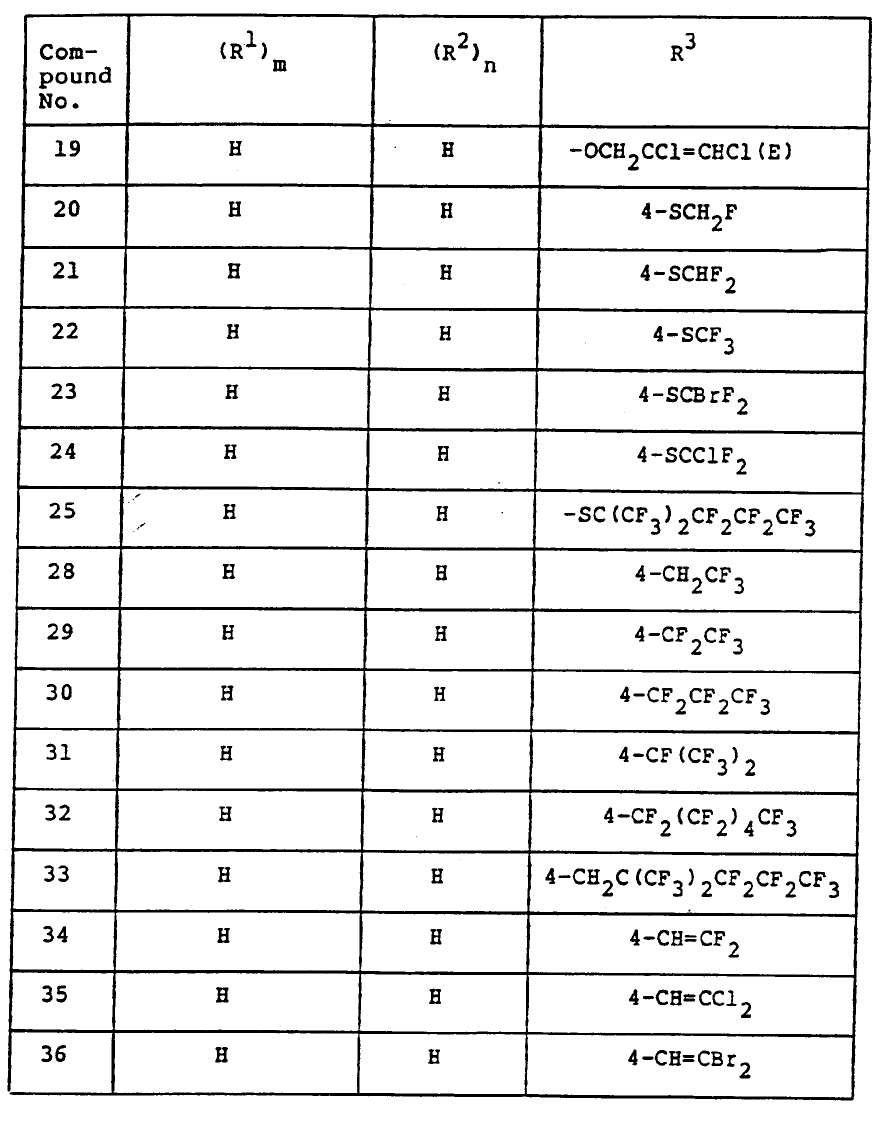

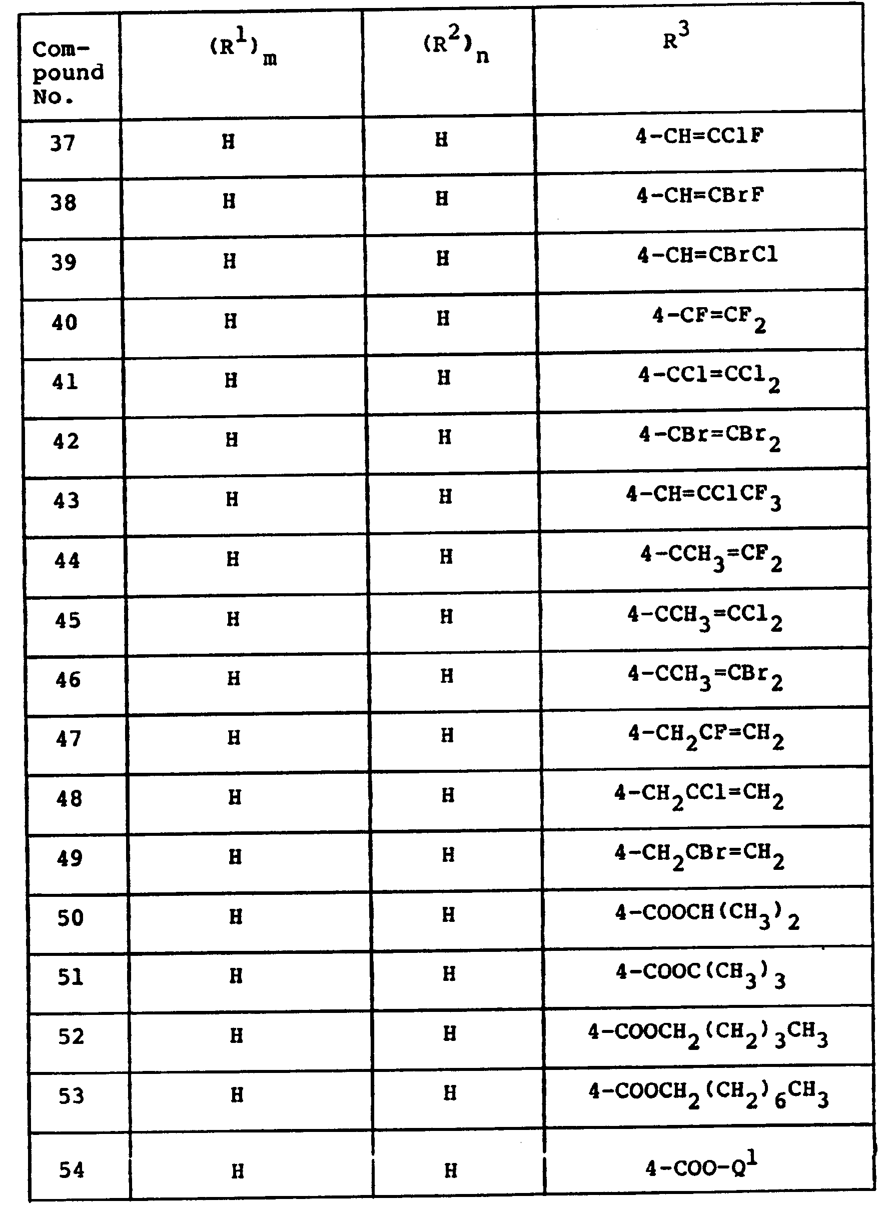

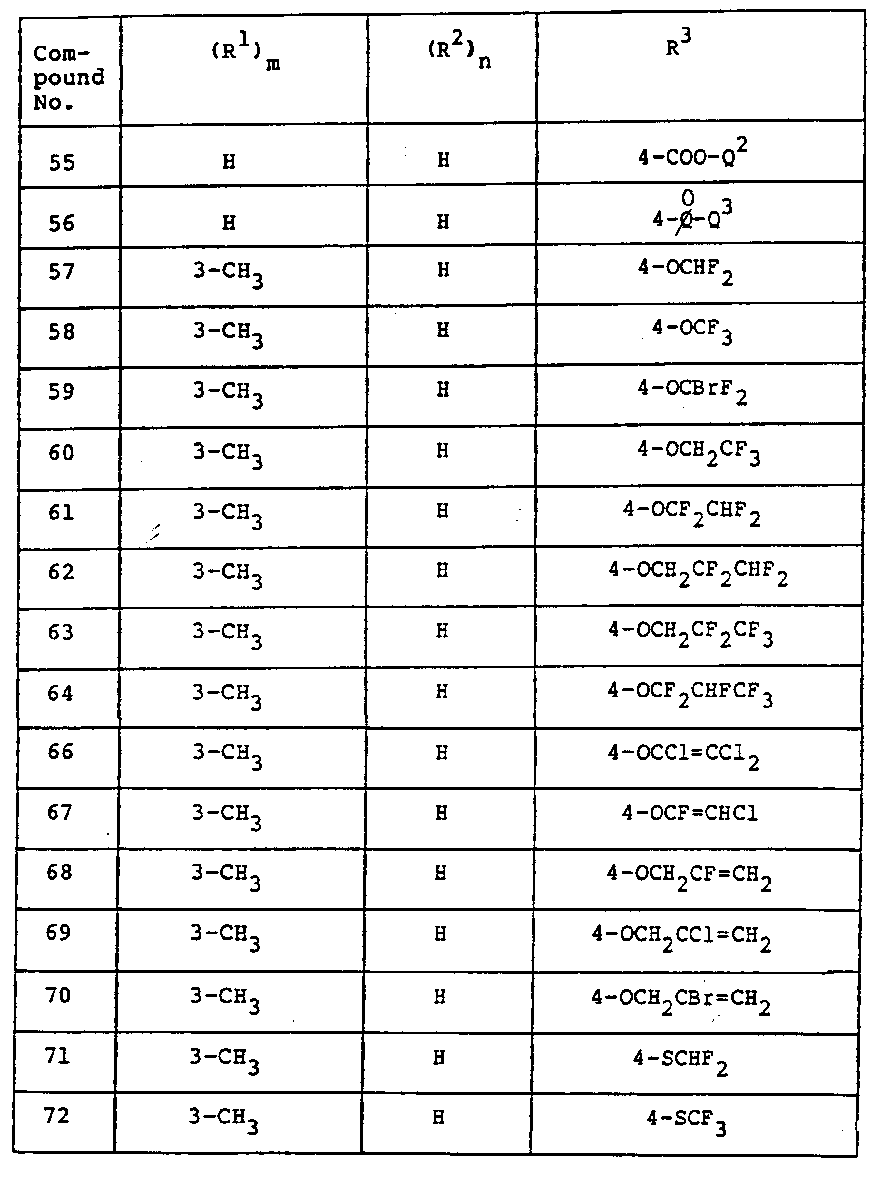

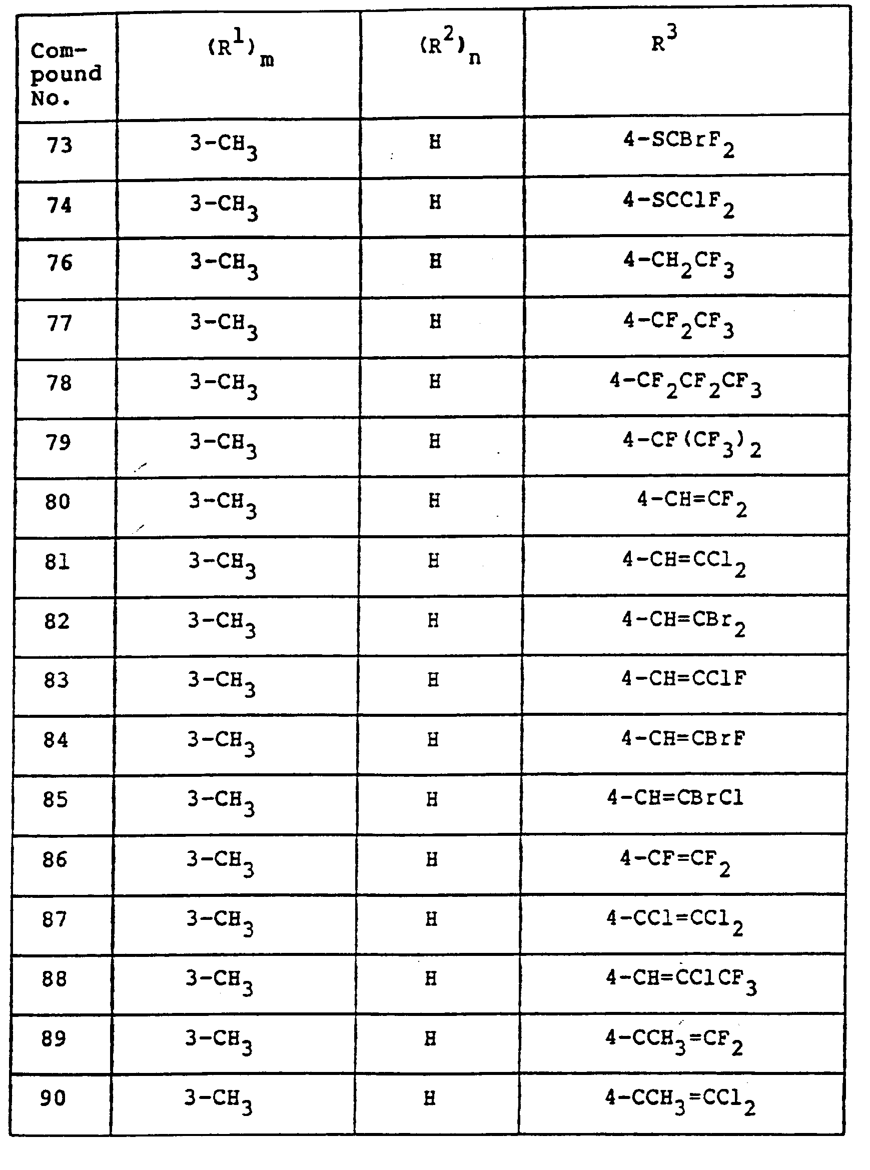

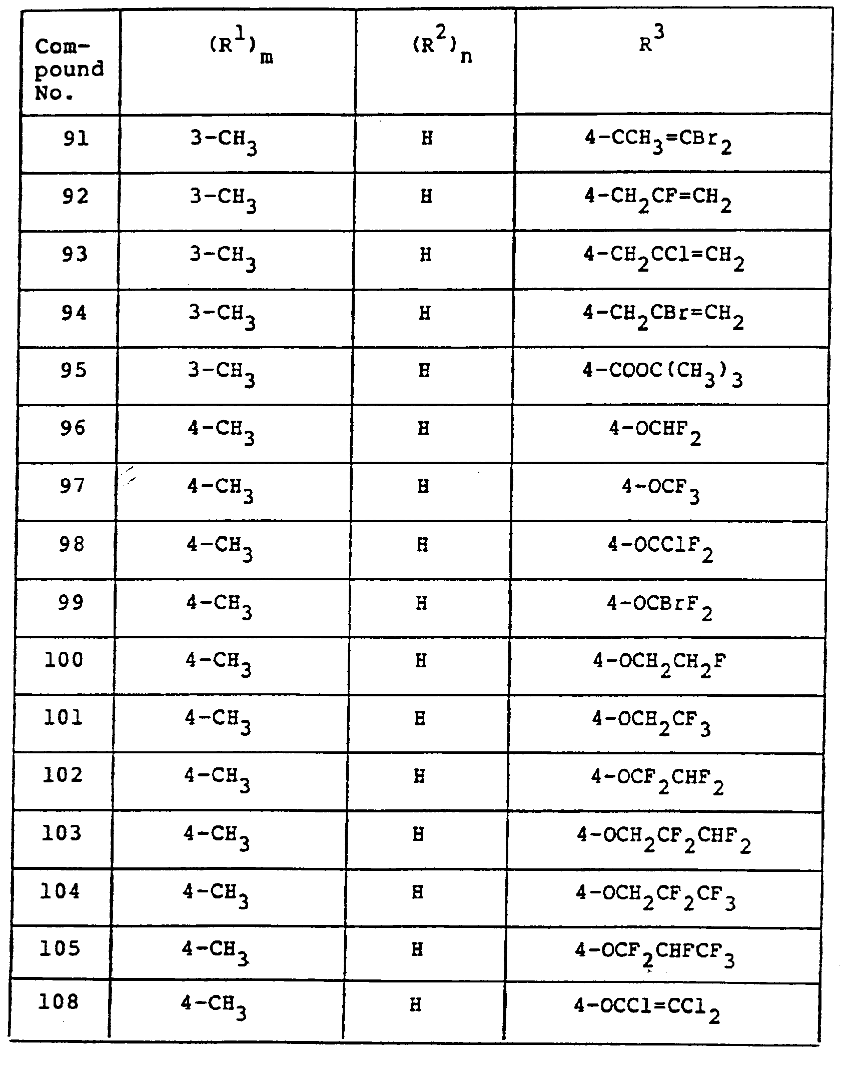

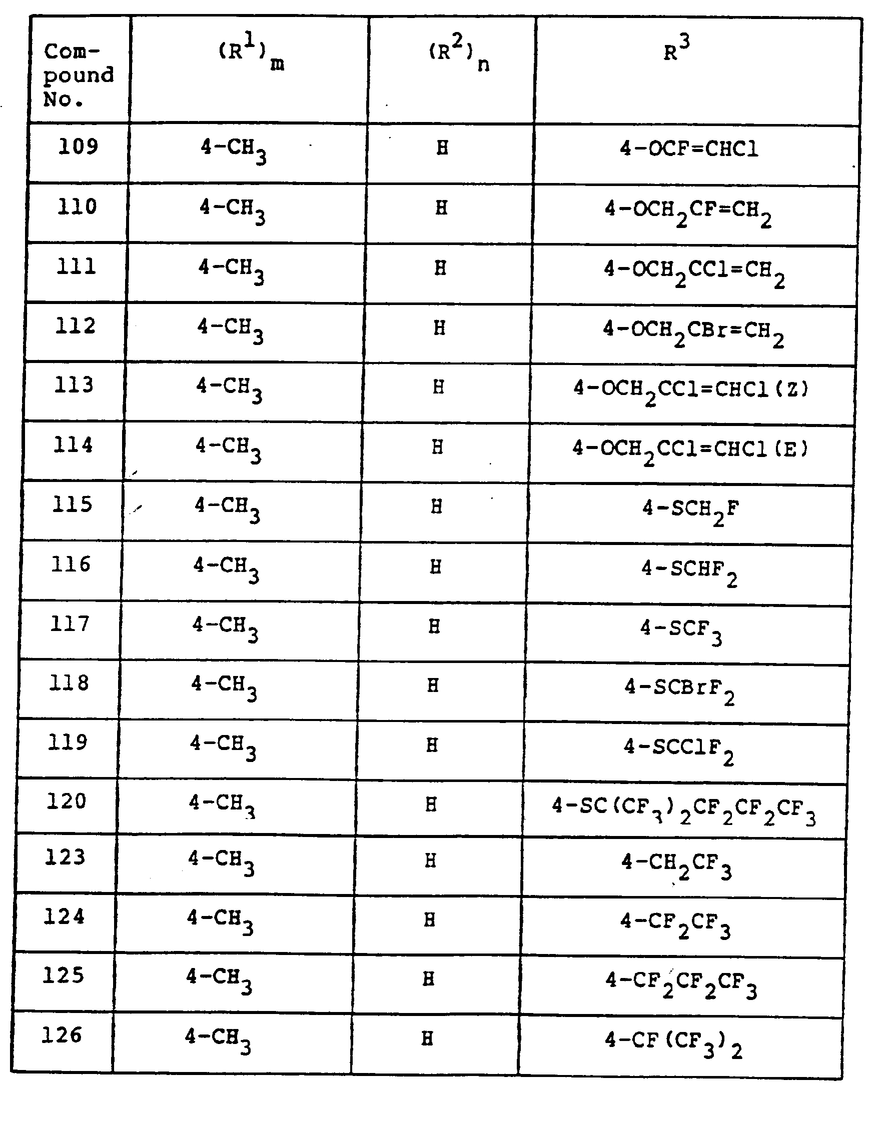

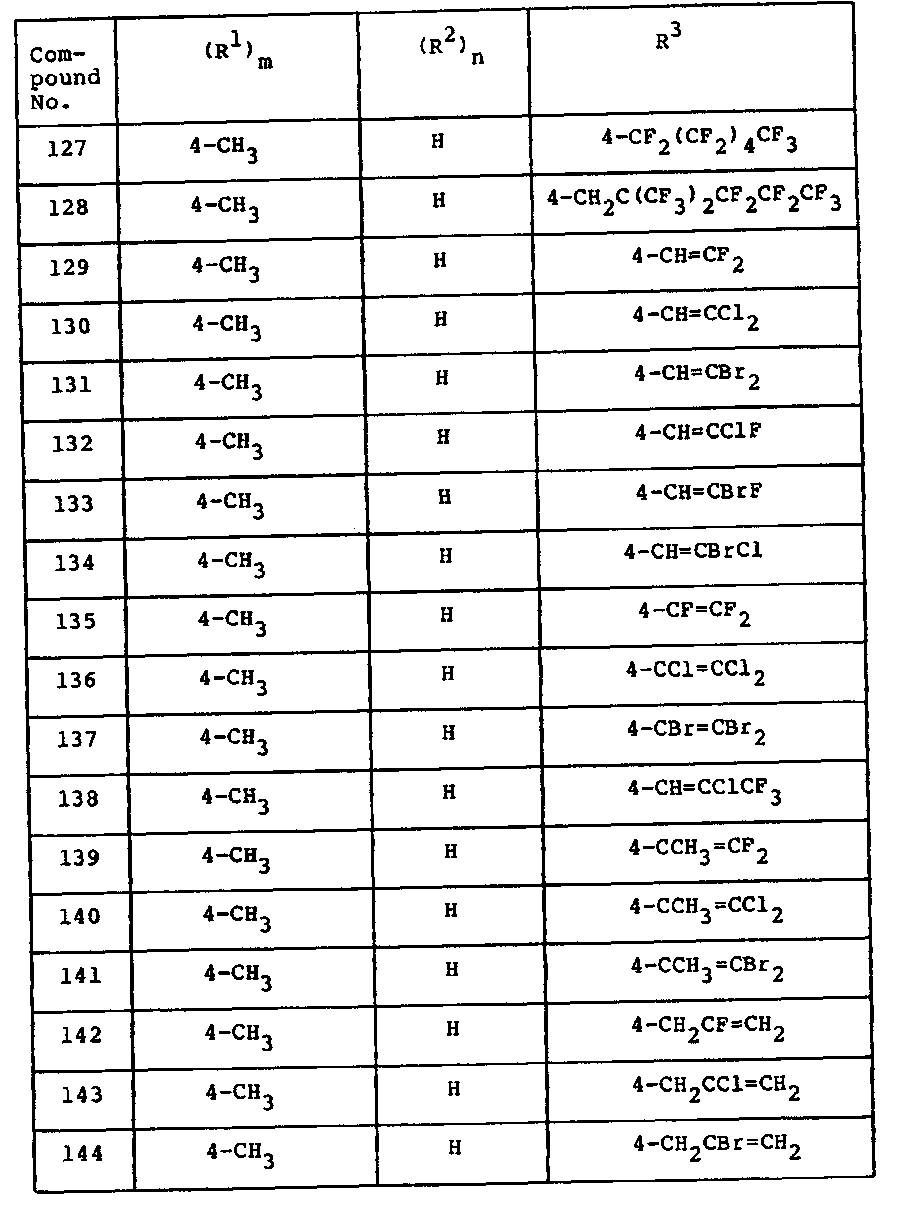

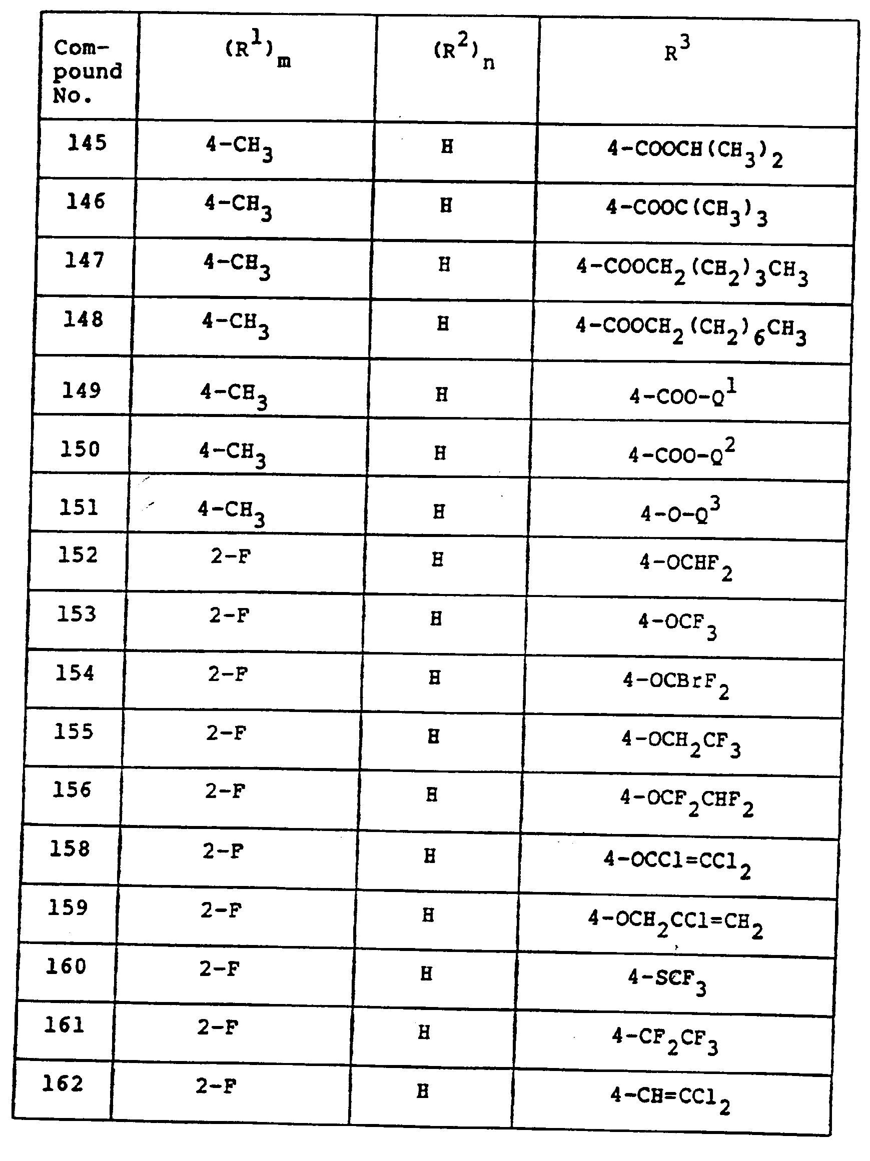

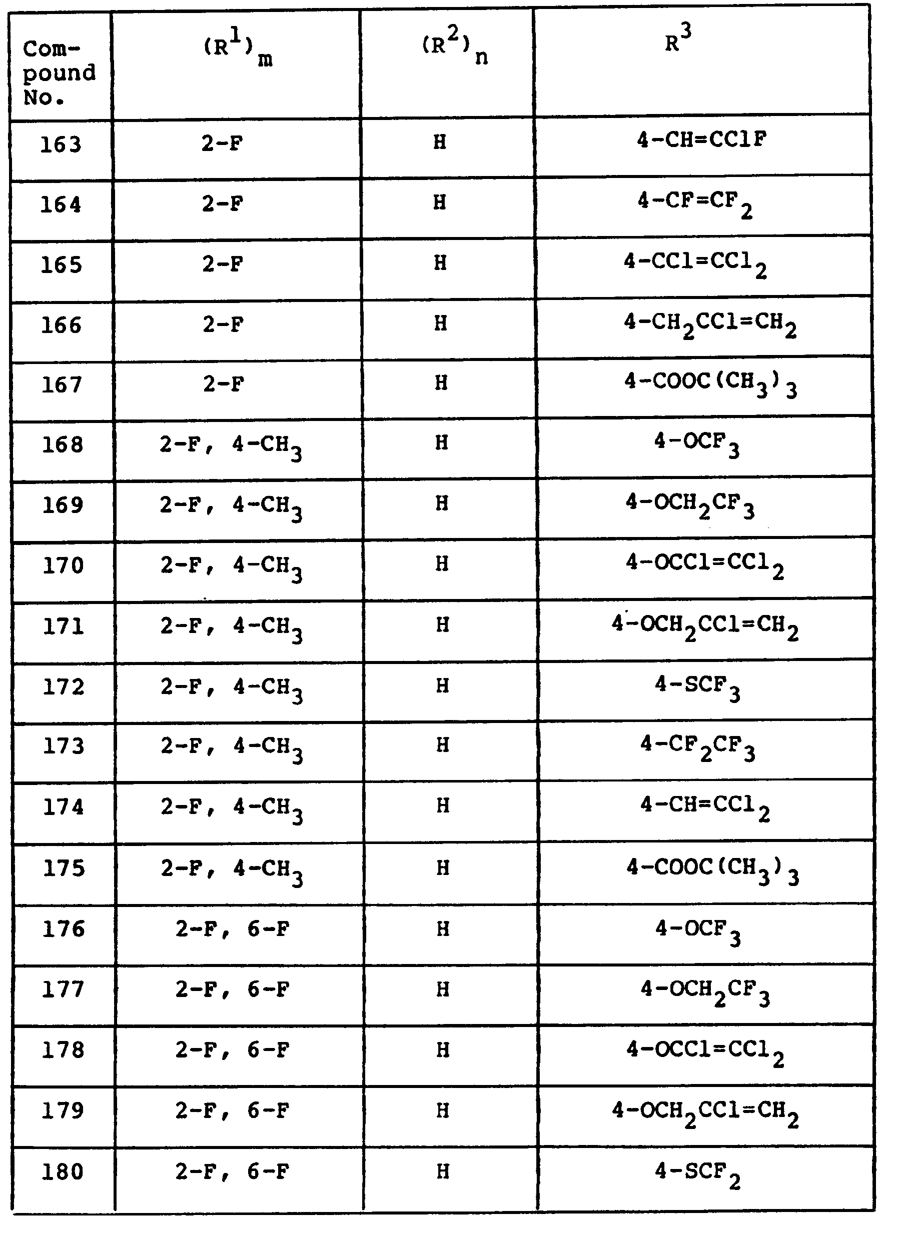

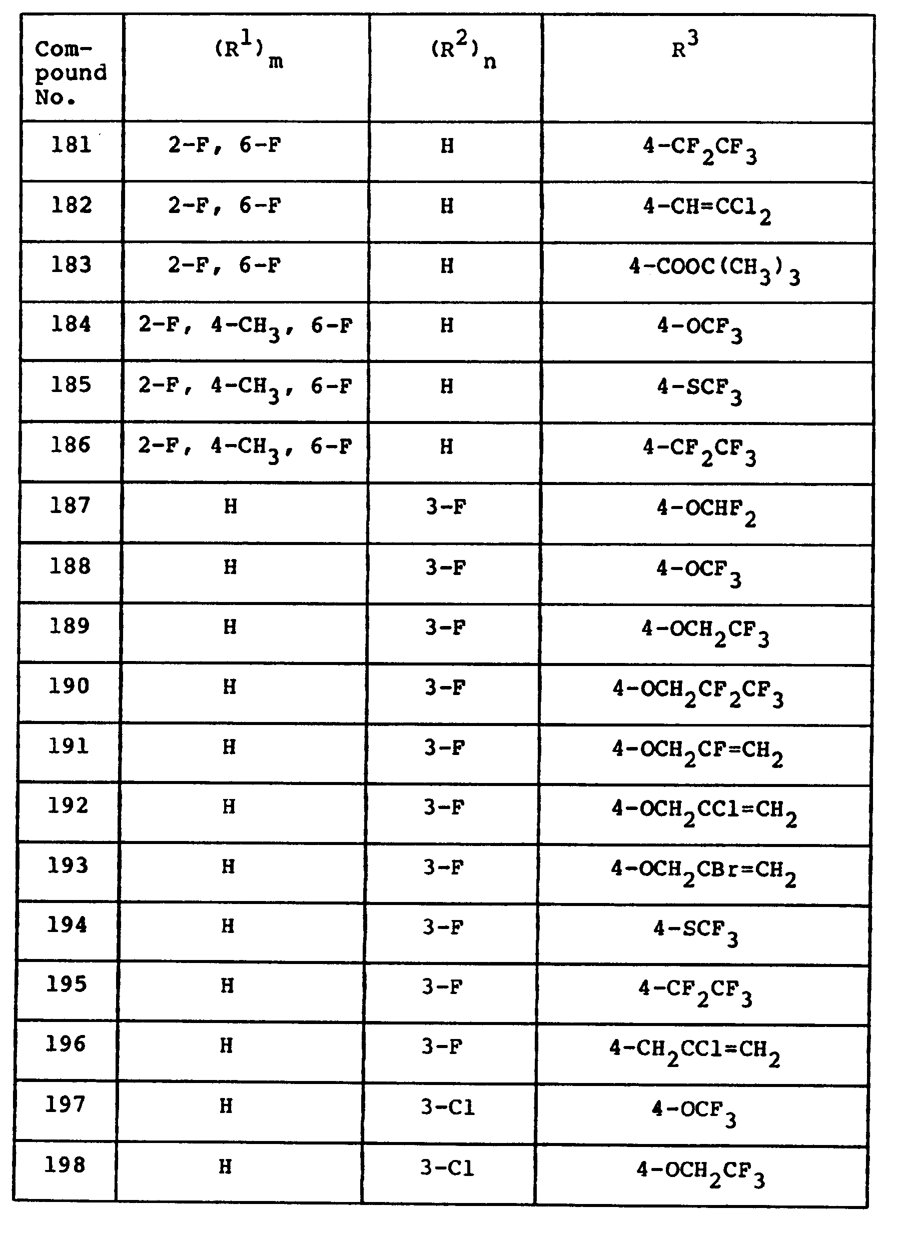

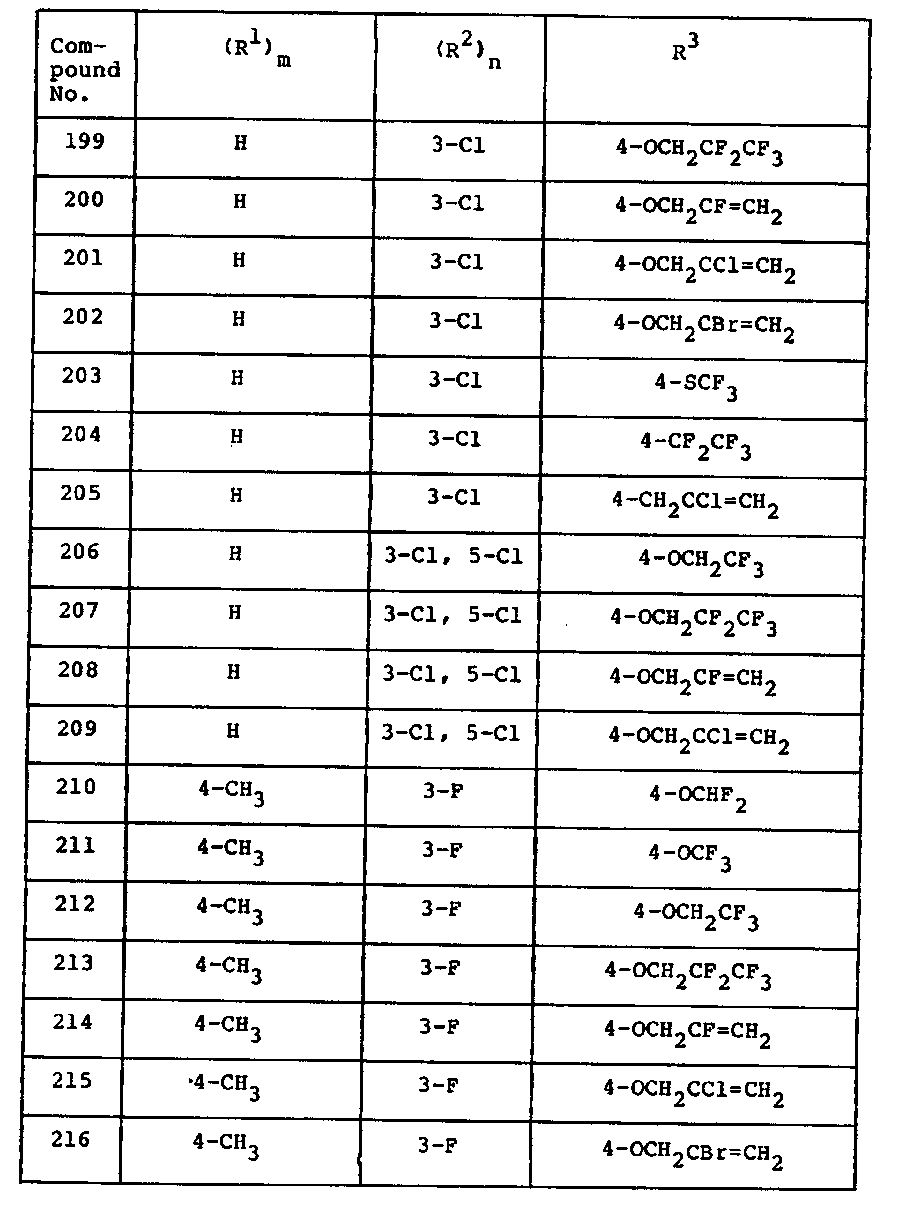

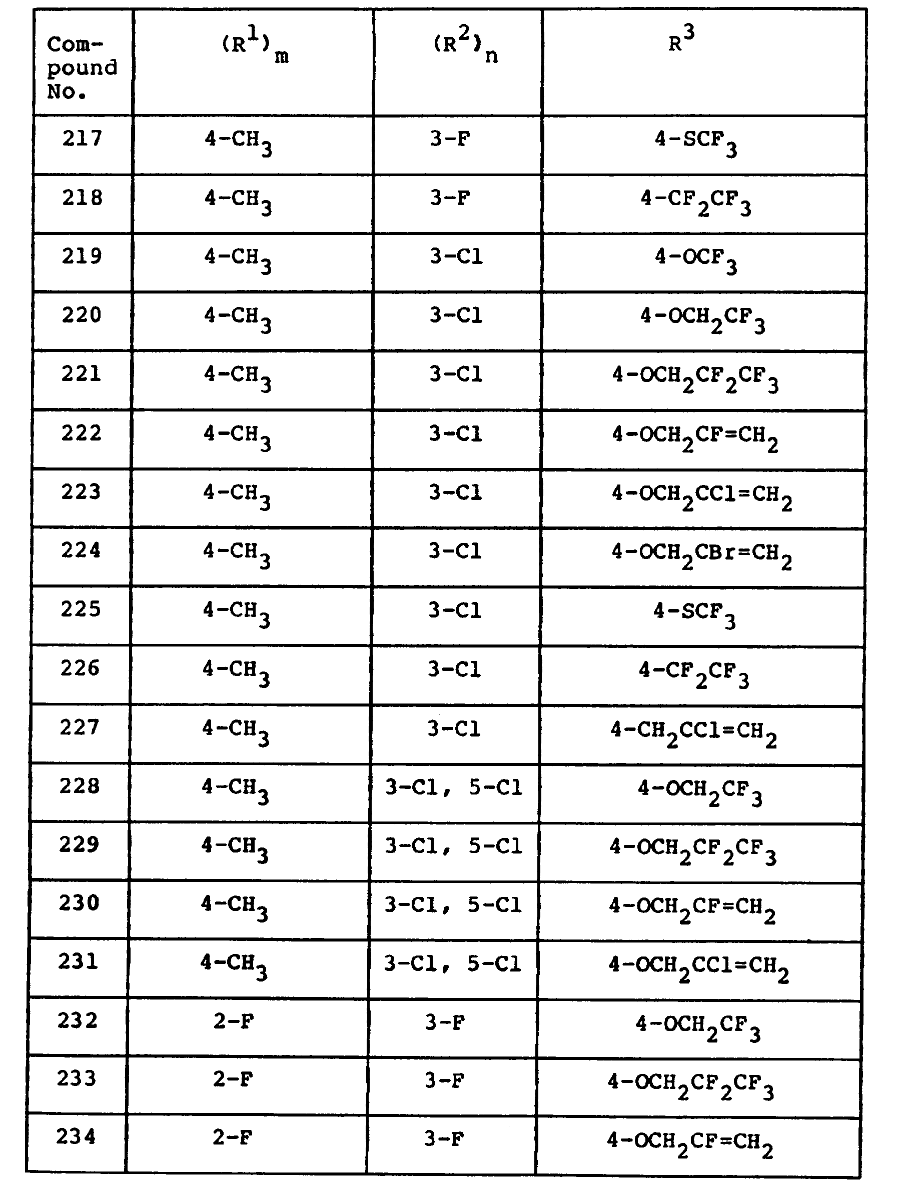

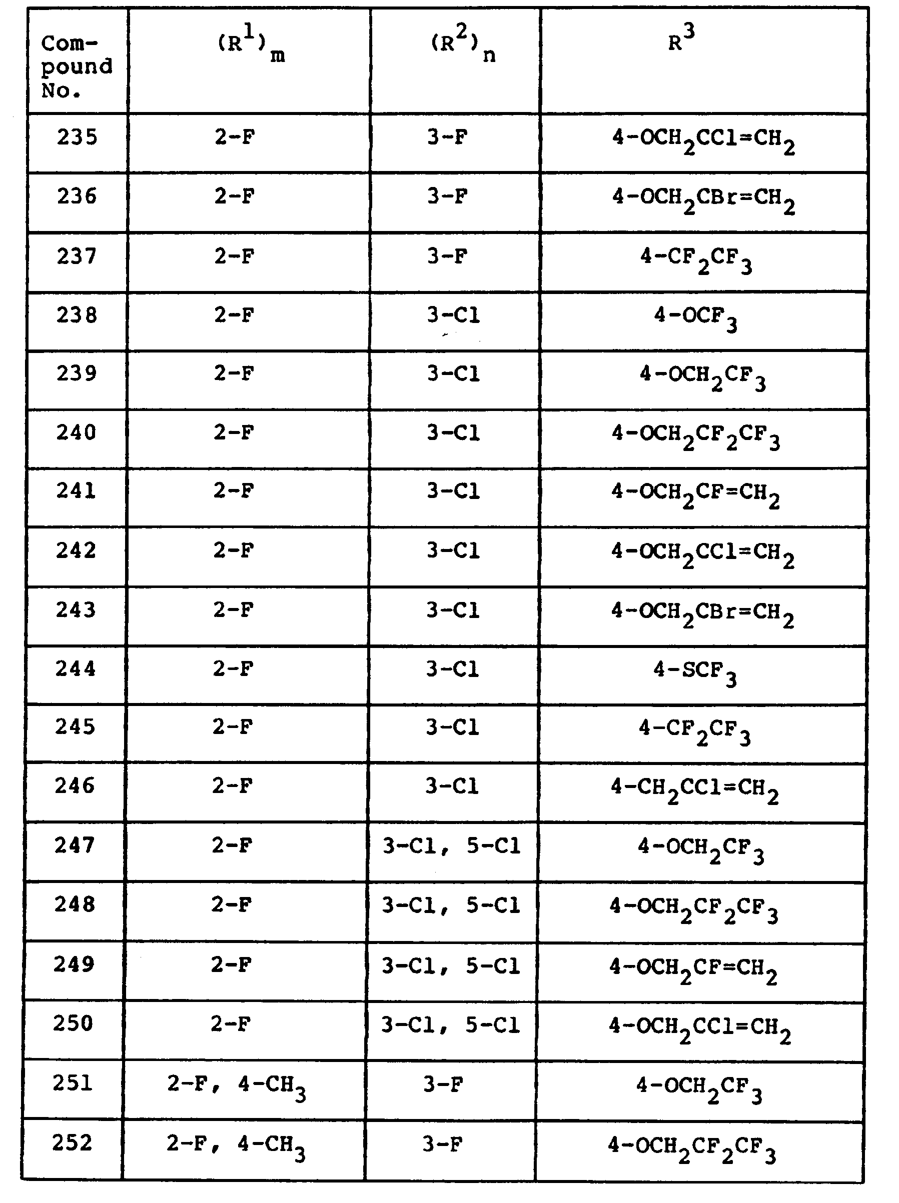

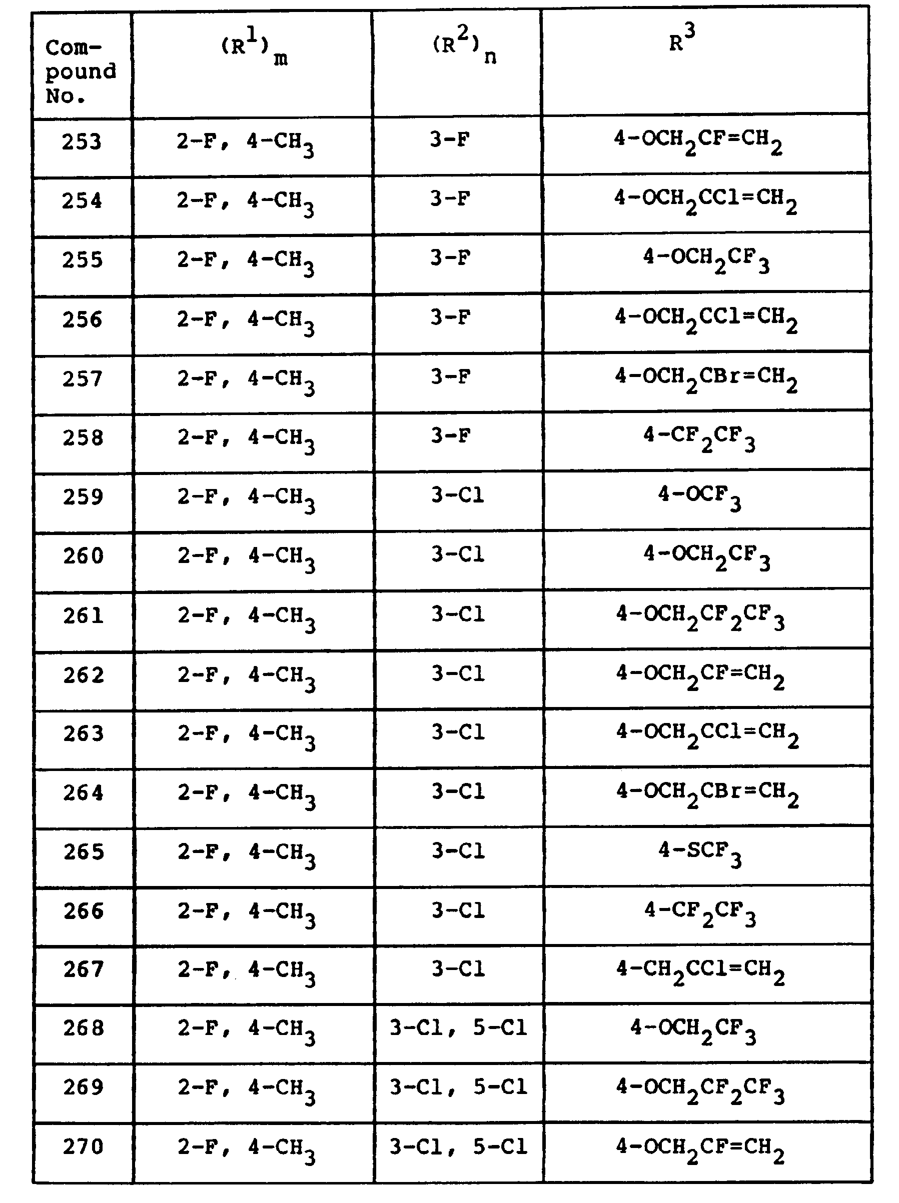

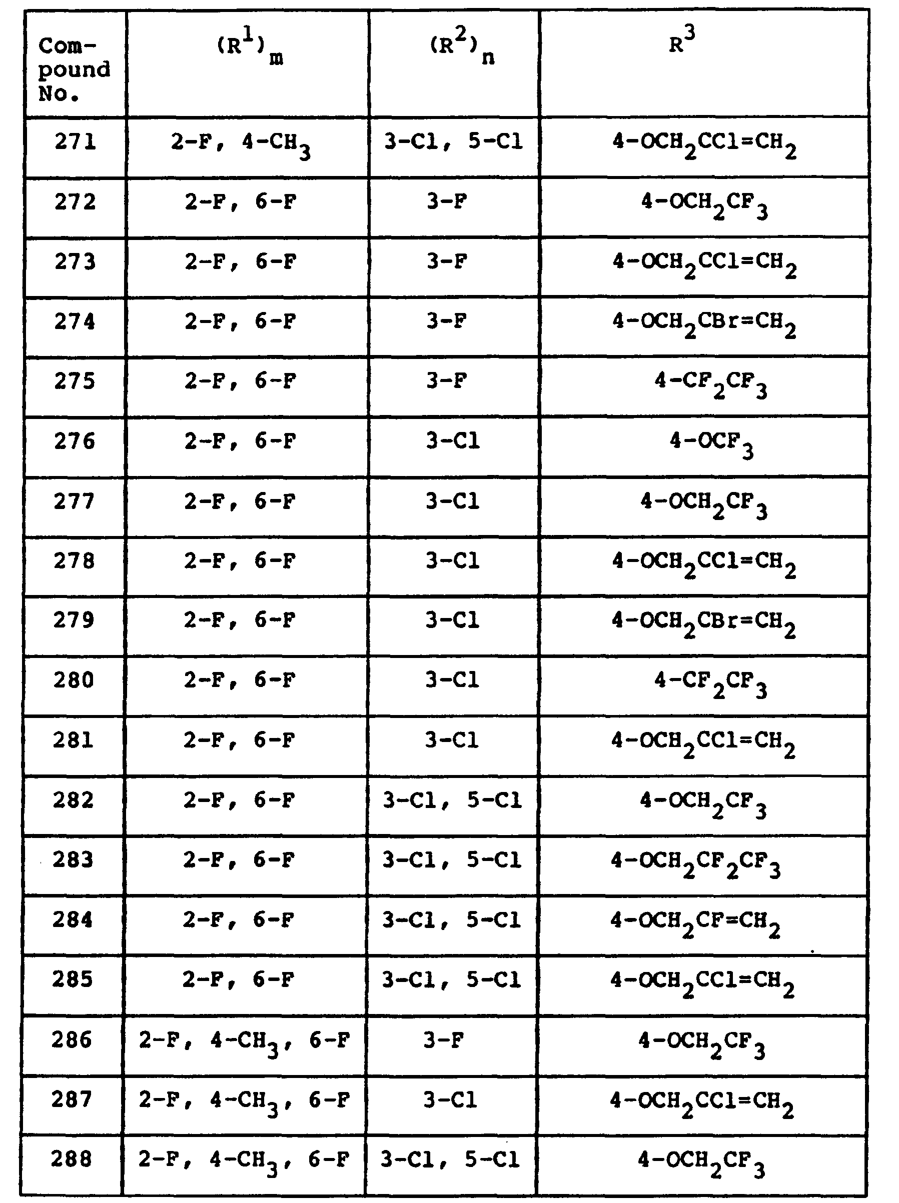

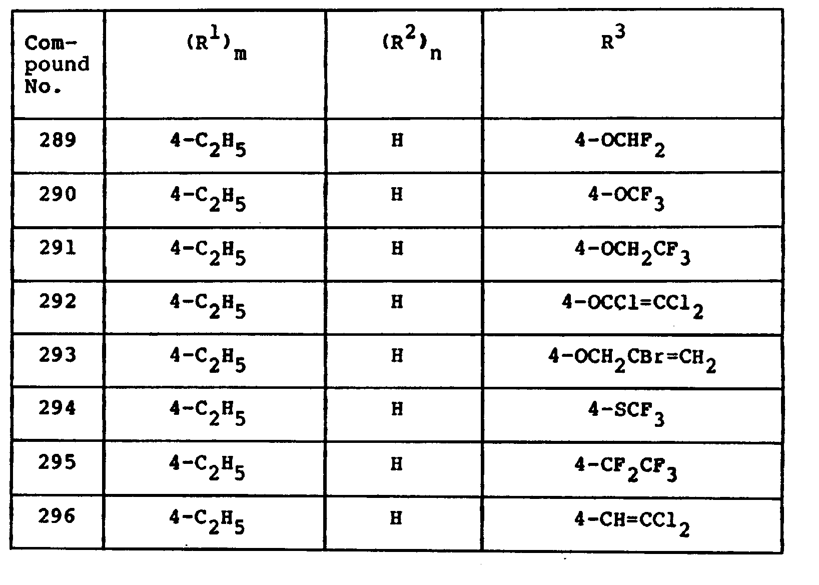

Q¹, Q² and Q³ in Table 1 show the following structural formulae.

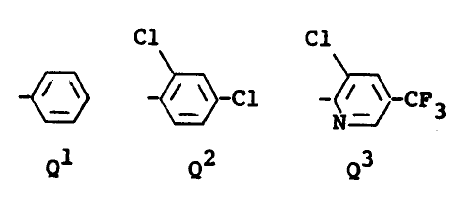

The compounds of formula (I) provided by this invention can be produced by the following method.

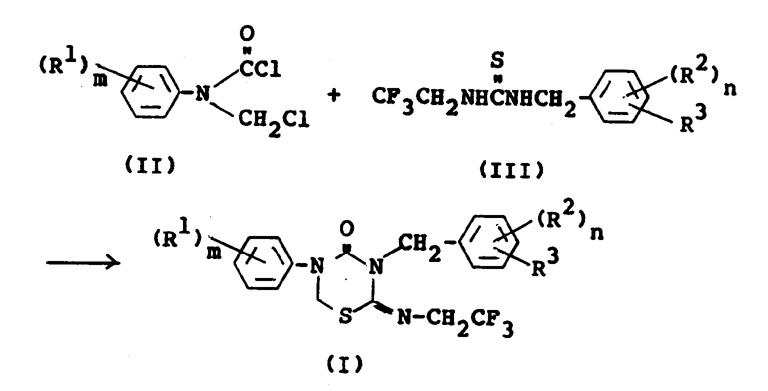

(In the formulae, R^{l}, R², R³, m and n are as defined above.)

Specifically, the compounds of the invention can be obtained by reacting a carbamoyl chloride derivative of general formula (II) with a thiourea derivative of general formula (III) in the presence or absence of a solvent, preferably in the presence of a solvent. Examples of suitable solvents are acetone, methyl ethyl ketone, cyclohexanone, tetrahydrofuran, dioxane, ethyl ether, benzene, toluene, acetonitrile, ethanol, propanol, dichloromethane, chloroform, carbon tetrachloride, dimethylformamide, dimethylacetamide, dimethyl sulfoxide, 1,3-dimethyi-2-imidazoiidinone, water and other solvents which do not affect the reaction.

The reaction is carried out under heating or in the presence of a base. In the case of performing the reaction under heating, the reaction temperature can be varied over a wide range depending upon the starting material. Generally, it is 30 to 250 °C, preferably 40 to 150 °C. The reaction time is 0.1 to 30 hours, preferably 0.5 to 24 hours.

Suitable bases that may be used in performing the reaction include, for example, potassium hydroxide, sodium hydroxide, potassium carbonate, sodium carbonate, sodium hydrogen carbonate, triethylamine, pyridine, N,N-dimethylaniline, 1,8-diazabicycio-[5,4,0]-7-undecene. The reaction temperature and the reaction time may be varied over broad ranges depending upon the starting material. Generally, the reaction temperature is -10 to 200 °C, preferably room temperature to 150 °C, and the reaction time is 0.1 to 30 hours, preferably 0.5 to 24 hours.

In performing the above reaction, the carbamoyl chloride derivative of general formula (II) and the thiourea derivative of general formula (III) may be used in equimolar proportions, or one of them may be used in slight excess. In the case of carrying out the reaction using the base and obtaining the compound of general formula (I) in a free form, it is preferred to use the base in an amount of 2 moles per mole of the carbamoyl chloride derivative of general formula (11), or in a slightly excessive molar proportion with respect to the compound (II).

The starting carbamoyl chloride derivative of general formula (II) may be synthesized by a known method [Journal of Organic Chemistry, vol. 39, page 2897 (1974)]. The thiourea of formula (III) may also be synthesized by a known method.

The salts of the compounds of the invention represented by general formula (I) can be produced by a known method. Specifically, the salts can be obtained by treating the compounds of general formula (I) with an inorganic acid such as hydrochloric acid, hydrobromic acid, hydroiodic acid, hydrofluoric acid, sulfuric acid, nitric acid, chloric acid, perchloric acid, phosphoric acid, thiocyanic acid and tetrafluoroboric acid, or an organic acid such as formic acid, acetic acid, trichloroacetic acid, trifluoroacetic acid, citric acid, lactic acid, oxalic acid, glycollic acid, malonic acid, succinic acid, malic acid, dodecylbenzenesulfonic acid, benzoic acid, salicylic acid and nicotinic acid.

The compounds of the present invention represented by general formula (I) and their salts can be used to protect plants from many kinds of noxious arthropods encountered in various fields, for example in agriculture, forestry and horticulture.

For example, the compounds of formula (I) are effective against hemipterous insect pests such as small brown planthopper, brown planthopper, whitebacked planthopper, green rice leafhopper, zig-zag rice leafhopper, tea green leafhopper, jumping plantlice, westwood-greenhouse whitefly, citrus spiny whitefly, green peach aphid, cotton aphid, cabbage aphid, spiraea aphid, lace bug, bean bug, cletus punctiger Dallas, rice bug, white-spotted bug, southern green stink bug, arrowhead scale, San jose scale, and white peach scale; lepidopterous insect pests such as rice stem borer, rice leafroller, oriental corn borer, rice skipper, green rice caterpillar, apple leafminer, beet semi-looper, black cutworm, cutworm, summer fruit tortrix, apple leafroller, peach fruit moth, citrus leafminer, pear leafminer, cherry treeborer, gypsy moth, fall webworm, cabbage moth, rice armyworm, cabbage armyworm, tobacco cutworm and common cabbageworm; coleopterous insect pests such as cupreous chafer, soybean beetle, Japanese beetle, citrus flower chafer, rice water weevil, rice plant weevil and sugarcane wireworm; dipterous insect pests such as rice crane fly, soybean pod gall midge, melon fly, oriental fruit fly, rice leafminer, stone leek leafminer, bryony leafminer, onion maggot and seedcorn maggot; and thrips such as yellow tea thrips, Thrips palmi Karny and onion thrips; and mites such as two-spotted spider mite, Kanzaa spider mite, carmine mite, European red mite, citrus red mite, hawthorn spider mite, and broad mite.

They are also effective against pests which cause various damages to man and domestic animals, for example, the transmission of epidemics, blood sucking, stinging and biting and skin inflammation, such as house mosquito, Culex pipiens molestus, Culex tritaeniorhyncus, Aedes albopictus, house flies, Boettcherisca peregrina Robineau-Desvoidy, Calliphora lata Coquillett, Phormia regina Meigen, Drosophila melanogaster, American cockroach, German cockroach, smokybrown cockroach, Periplaneta brunnea Burmeister, Japanese cockroach, Ornithonyssus bacoti Hirst, human louse, Pediculus humanus humanus De Geer, Climex lectularus Linne, human flea, dog flea, cat flea, oriental tussock moth, tea tussock moth, Scolopendra subspinipes japonica, rove beetle, and Xanthochroa waterhousei Harold; pests which damage foods or stored grains, such as mold mite, bread beetle, confused flour beetle, maize weevil, azuki bean weevil, common hide beetle and Indian meal moth; pests which damages furniture, building materials, books and apparel such as Reticulitermes speratus Kolbe, Formosan subterranean termite, powderpost beetle, Gastrallus immarginatus Mullerr, casemaking clothes moth and black carpet beetle; and so-called "unpleasant pests", such as Telmatoscopus albipunctatus Williston, Chironomus plumosus Linnaeus, midges, camel crickets, brown marmorated stink bug, Thereuronema hilgendorfi Verhoeff, Oxidus gracilis C. L. Koch, pillbug and Porcellio scaber Latreille.

The compounds of this invention show much higher insecticidal activity on lepidopterous insect pests than known compounds.

In actual application, the compound of the invention may be used singly without other components, but to make it easy to use as a control agent, it is generally applied as a mixture with a carrier.

Formulation of the compound of the invention requires no particular conditions, and it may be prepared in any desired form such as an emulsifiable concentrate, a wettable powder, a dust, granules, a pulverulent agent, an oil, an aerosol, a fumigant or a bait by methods well known to those skilled in the art in accordance with the formulation of general agricultural chemicals.

The carrier, as used herein, denotes a synthetic or natural inorganic or organic material which is incorporated in order to aid in the arrival of the active ingredient at a site to be treated orfacilitate storage, transportation and handling of the active ingredient compound.

Suitable solid carriers include, for example, clays such as montmorillonite and kaolinite; inorganic materials such as diatomaceous earth, terra alba, talc, vermiculite, gypsum, calcium carbonate, silica gel and ammonium sulfate, organic plant materials such as soybean meal, sawdust and wheat flour; and urea.

Suitable liquid carriers include, for example, aromatic hydrocarbons such as toluene, xylene and cumene, paraffinic hydrocarbons such as kerosene and mineral oils, ketones such as acetone and methyl ethyl ketone, ethers such as dioxane and tetrahydrofuran, alcohols such as methanol, ethanol, propanol and ethylene glycol, dimethylformamide, dimethyl sulfoxide, and water.

To enhance the efficacy of the compounds of this invention, various adjuvants, either singly or in combination, may be combined with the compounds of the invention according to the formulation of the compounds, the situation in which they are applied, etc.

For the purpose of emulsification, dispersion, spreading, wetting, binding and stabilization, there may be used anionic surface-active agents such as ligno-sulfonates, alkylbenzenesulfonates and alkylsulfates; nonionic surface-active agents such as polyoxyalkylene alkyl ethers, polyoxyalkylene alkyl aryl ethers, polyoxyalkylene alkylamines, polyoxyalkylene alkylamides, polyoxyalkylene alkyl thioethers, polyoxyalkylene fatty acid esters, glycerin fatty acid esters, sorbitan fatty acid esters, polyoxyalkylene sorbitan fatty acid esters and polyoxypropylene polyoxyethylene block polymers; lubricants such as calcium stearate and waxes; stabilizers such as isopropyl hydrogenphosphate; and methyl cellulose, carboxymethyl cellulose, casein and gum arabic. These examples, however, are not limitative.

Better insecticidal and acaricidal activities may be obtained by using two or more compounds of this invention in combination. Furthermore, multipurpose compositions having a better efficacy may be prepared by mixing the compounds of the invention with other insecticides or acaricides, fungicides, nematocides, herbicides, plant growth regulating agents, fertilizers, and other agricultural chemicals. Synergistic effects can be expected from such compositions. Examples of the other insecticides or acaricides include fenthion, fenitrotion, diazinon, chlorpyrifos, chlorpyrifos-methyl, methidathion, dichlorvos, thiometon, acephate, trichlorphon, isoxathion, pyridafenthion, salithion, prothiofos, propaphos, EPN, sulprofos, NAC, MTMC, MIPC, BPMC, PHC, MPMC, XMC, pirimicarb, carbosulfan, benfuracarb, methomyl, oxamyl, pyrethrin, tetramethrin, phthalthrin, vaporthrin, allethrin, resmethrin, fenvalerate, esfenvalerate, permethrin, cypermethrin, fluvalinate, ethofenprox, flucythrinate, cyhalothrin, bifenthrin, diflubenzuron, chlorfluazuron, teflubenzuron, flufenoxuron, cypromazine, buprofezin, fenoxycarb, benzoepin, nereistoxin, bensultap, thiocyclam, avermectin, dicofol, amitraz, polynactins, fenbuta- tin oxide, cyhexatin, hexythiazox, flubenzamine, triarathene, clofentezine and milbemycin.

The compounds of this invention are stable to light, heat and oxidation. If required, however, suitable amounts of stabilizers, for example antioxidants or ultraviolet absorbers such as phenol derivatives [e.g., BHT (2,6-di-t-butyl-4-methylphenol) and BHA (butylhydroxyanisole)], bisphenol derivatives, arylamines (e.g., phenyl-a-naphthylamine, phenyl-β-naphthylamine, or a condensate of phenetidine and acetone), and benzophenone compounds may be added. This can give a composition having a more stabilized efficacy.

In the insecticidal and acaricidal agent of this invention, 0.1 to 95 % by weight, preferably 0.3 to 50 % by weight, of the compound of formula (I) or its salt is included as an active ingredient. In applying the insecticidal and acaricidal agent of this invention, the active ingredient is desirably used in a concentration of 0.01 to 5000 ppm, preferably 0.1 to 1000 ppm. The rate of application per 10 a is generally 1 g to 300 g as the active ingredient.

The following examples illustrate the present invention more specifically.

### EXAMPLE 1

Synthesis of 2-(2,2,2-trifluoroethylimino)-3-(4-trifluoromethoxybenzyl)-5-phenyl-tetrahydro-1,3,5-thiadia- zin-4-one (compound No. 2):-
0.61 g of N-chloromethyl-N-phenylcarbamoyl chloride and 1.00 g of 1-(4-trifluoromethoxybenzyl)-3-(2,2,2-trifluoroethyl)-thiourea were dissolved in 30 ml of benzene, and the solution was heated under refluxfor4 hours. After the reaction, benzene was evaporated under reduced pressure. The resulting oily product was purified by column chromatography [silica gel; developing solvent: hexane/ethyl acetate (10:1)] to give 0.68 g of the captioned compound.
melting point: 54.0-56.0 °C.
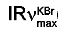 (cm¹): 1675, 1665, 1610, 1500, 1450, 1395, 1260, 1140, 1120, 930, 840, 765.
¹H NmR 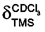 (ppm): 3.78(2H, q, J=9Hz), 4.84 (2H, s), 5.25(2H, s), 7.04-7.91(9H, m).

As an isomer, 0.17 g of 2-(4-trifluoromethoxybenzylimino)-3-(2,2,2-trifluoroethyl)-5-phenyl-tetrahydro-1,3,5-thiadiazin-4-one was obtained.
Melting point: 84.0-87.0 °C.
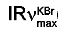 (cm⁻¹): 1690, 1615, 1505, 1495, 1440, 1390, 1260, 1170, 1120, 835, 820, 755.
¹H NMR 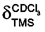 (ppm): 4.52(2H, s), 4.69(2H, s), 5.09(2H, q, J=9Hz), 7.11-7.36(9H, m).

### EXAMPLE 2

Synthesis of 2-(2,2,2-trifluoroethylimino)-3-(4-difluoromethoxybenzyl)-5-(4-methylphenyl)-tetrahydro-1,3,5-thiadiazin-4-one (compound No. 96):-
0.69 g of N-chloromethyl-N-(4-methylphenyl)-carbamoyl chloride and 1.00 g of 1-(4-difluoromethoxyben- zyl)-3-(2,2,2-trifluoroethyl)thiourea were dissolved in 30 ml of toluene, and the solution was heated under reflux for 4 hours. After the reaction, toluene was evaporated under reduced pressure. The resulting oily product was purified by column chromatography [silica gel; developing solvent hexane/ethyl acetate (10:1)] to give 0.80 g of the captioned compound.
Refractive index 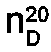 : 1.5461.
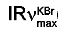 (cm⁻¹): 1680, 1620, 1515, 1450, 1400, 1270, 1130, 1090, 1045, 940, 850, 820, 755.
¹H NMR 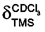 (ppm): 2.33(3H, s), 3.75(2H, q, J=9Hz), 4.75(2H, s), 5.18(2H, s), 6.39(1 H, t, J=74Hz), 6.90-7.44(8H, m).

As an isomer, 0.19 g of 2-(4-difluoromethoxybenzylimino)-3-(2,2,2-trifluoroethyl)-5-(4-methylphenyl)-tet- rahydro-1,3,5-thiadiazin-4-one was obtained.
Melting point: 88.5-90.0 °C.
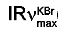 (cm-¹): 1710, 1685, 1620, 1520, 1445, 1390, 1265, 1175, 1120, 1050, 830.
¹H NMR 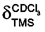 (ppm): 2.36(3H, s), 4.46(2H, s), 4.79(2H, s), 5.04(2H, q, J=9Hz), 6.33(1 H, t, J=74Hz), 6.97-7.28(8H, m).

### EXAMPLE 3

Synthesis of 2-(2,2,2-trifluoroethylimino)-3-(4-trifluoromethylthiobenzyl)-5-phenyl-tetrahydro-1,3,5-thiadiazin-4-one (compound No. 22):-
A solution of 1.77 g of trichloromethyl chloroformate in 10 ml of benzene was added dropwise to a solution of 1.89 g of 1,3,5-triphenyl-hexahydro-s-triazine with stirring at room temperature under a nitrogen current. The reaction solution was stirred at room temperature for 1.5 hours. Then, 3.0 g of 1-(4-trifluoromethylthiobenzyl)-3-(2,2,2-trifluoroethyl)-thiourea was added to the solution at room temperature with stirring, and subsequently, 1.5 ml of triethylamine was added. The mixture was stirred at room temperature for 12 hours. Aqueous ammonia (30 ml) was added to the reaction solution, and the mixture was extracted with 150 ml of ethyl acetate. The ethyl acetate solution was washed with water and dried, and ethyl acetate was evaporated under reduced pressure. The resulting oily product was purified by column chromatography [silica gel; developing solvent hexane/ethyl acetate (10:1)] to give 0.47 g of the captioned compound.
Melting point: 89.5-90.0 °C.
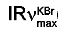 (cm⁻¹): 1660, 1605, 1500, 1490, 1430, 1395, 1285, 1270, 1215, 1140, 1120, 1085, 930, 840, 750, 715.
¹H NMR 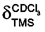 (ppm): 3.80(2H, q, J=9Hz), 4.84 (2H, s), 5.32(2H, s), 7.20-7.60(9H, m).

As an isomer, 0.11 g of 2-(4-trifluoromethylthiobenzylimino)-3-(2,2,2-trifluoroethyl)-5-phenyltetrahydro-1,3,5-thiadiazin-4-one was obtained as a semisolid.
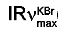 (cm⁻¹): 1695, 1615, 1490, 1440, 1390, 1335, 1255, 1170, 1150, 1120, 1080, 810, 755, 715.
¹H NMR 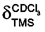 (ppm): 4.28(2H, s), 4.78(2H, s), 5.01(2H, q, J=9Hz), 7.10-7.49(9H, m).

### EXAMPLE 4

Synthesis of 2-(2,2,2-trifluoroethylimino)-3-[4-(2,2,2-trifluoroethoxy)benzyl]-5-phenyl-tetrahydro-1,3,5-thia- diazin-4-one (compound No. 6):-
A solution of 1.00 g of trichloromethyl chloroformate in 10 ml of benzene was added dropwise to a solution of 1.08 g of 1,3,5-triphenyl-hexahydro-s-triazine in 20 ml of tetrahydrofuran with stirring at room temperature under a nitrogen current. The reaction solution was stirred at room temperature for 1.5 hours, and then 3.46 g of 1-[4-(2,2,2-trifluoroethoxy)benzyl]-3-(2,2,2-trifluoroethyl)thiourea was added at room temperature with stirring, and subsequently, 5.0 ml of triethylamine was added. The mixture was stirred at room temperature for 12 hours. Water (30 ml) was added to the reaction mixture, and the mixture was extracted with 150 ml of ethyl acetate. The ethyl acetate solution was washed with water and dried, and ethyl acetate was evaporated under reducd pressure. The resulting oily product was purified by column chromatography [silica gel; developing solvent hexane/ethyl acetate (10:1)] to give 0.56 g of the captioned compound.
Refractive index 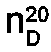 : 1.5324.
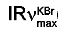 (cm⁻¹): ₁₆₇₀, ₁₆₀₅, ₁₅₀₅, ₁₄₅₀, ₁₄₄₀, ₁₃₈₀, ₁₃₅₀, ₁₂₆₀, ₁₂₅₀, ₁₂₂₅, ₁₂₀₀, ₁₁₇₀, ₁₁₅₀, ₁₁₃₀, 1110, 1080, 1070, 965.
¹H NMR 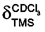 (ppm): 3.80(2H, d, J=9Hz), 4.31 (2H, d, J=8Hz), 4.80(2H, s), 5.27(2H, s), 6.88(2H, d, J=9Hz), 7.20-7.60(7H, m).

As an isomer, 0.51 g of2-[4-(2,2,2-trifluoroethoxy)benzylimino]-3-(2,2,2-trifluoroethyl)-5-phenyltetrahydro-1,3,5-thiadiazin-4-one was obtained.
Melting point: 103.0-104.5 °C.
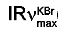 (cm-¹): 1675, 1605, 1500, 1430, 1375, 1350, 1285, 1260, 1230, 1190, 1160, 1150, 1135, 1100, 1060, 1035, 965.
¹H NMR 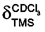 (ppm): 4.32(2H, q, J=8Hz), 4.47 (2H, s), 4.87(2H, s), 5.13(2H, d, J=10Hz), 6.94(2H, d, J=8Hz), 7.20-7.60(7H, m).

### EXAMPLE 5

Synthesis of 2-(2,2,2-trifluoroethylimino)-3-[4-(1,1,2,2-tetrafluoroethoxy)benzyl]-5-(4-methylphenyl)-tetrahyd- ro-1,3,5-thiadiazin-4-one (compound No. 102):-
A solution of 0.65 g of trichloromethyl chloroformate in 20 ml of benzene was added dropwise to a solution of 0.76 g of 1,3,5-tris(p-tolyl)hexahydro-s-triazine in 10 ml of tetrahydrofuran at room temperature with stirring under a nitrogen current. The reaction solution was stirred at room temperature for 1 hour and then 2.20 g of 1-[4-(1,1,2,2-tetrafluoroethoxy)benzyl]-3-(2,2,2-trifluoroethyl)thiourea was added at room temperature with stirring, and then 4 ml of triethylamine was added. The mixture was stirred at room temperature for 6 hours. Water (50 ml) was added to the reaction mixture, and the mixture was extracted with 200 ml of ethyl acetate. The ethyl acetate solution was washed with water and dried, and ethyl acetate was evaporated under reduced pressure. The resulting oily product was purified by column chromatography [silica gel; developing solvent hexane/ethyl acetate (10:1)] to give 0.23 g of the captioned compound.
Refractive index 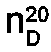 : 1.5242.
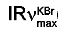 (cm⁻¹): 1680, 1615, 1510, 1450, 1390, 1360, 1285, 1270, 1265, 1210, 1190, 1145, 1130.
¹H NMR 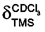 (ppm): 2.37(3H, s), 3.80(2H, q, J=9Hz), 4.80(2H, s), 5.28(2H, s), 5.89(1 H, tm, J=52Hz), 7.0-7.4(6H, m), 7.30(2H, d, J=8Hz).

As an isomer, 0.32 g of 2-[4-(1,1,2,2-tetrafluoroethoxy)benzylimino]-3-(2,2,2-trifluoroethyl)-5-(4-methylphenyl)tetrahydro-1,3,5-thiadiazin-4-one was obtained.
Melting point: 89.0-90.5 °C.
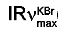 (cm-¹): 1690, 1620, 1515, 1505, 1440, 1425, 1410, 1395, 1300, 1265, 1260, 1205, 1180, 1160, 1110.
¹H NMR 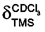 (ppm): 2.40(3H, s), 4.52(2H, s), 4.86(2H, s), 5.15(2H, q, J=10Hz), 5.93(1 H, tm, J=52Hz), 7.10-7.50(8H, m).

### EXAMPLE 6

Synthesis of 2-(2,2,2-trifluoroethylimino)-3-(4-pentafluoroethylbenzyl)-5-(4-methylphenyl)-tetrahydro-1,3,5-thiadiazin-4-one (compound No. 124):-
0.71 g of N-chloromethyl-N-(4-methylphenyl)carbamoyl chloride and 1.20 g of 1-(4-pentafluoroethylbenzyl)-3-(2,2,2-trifluoroethyl)thiourea were dissolved in 30 ml of toluene, and the solution was heated under reflux for 4 hours. After the reaction, toluene was evaporated under reduced pressure. The resulting oily product was purified by column chromatography [silica gel; developing solvent hexane/ethyl acetate (10:1)] to give 0.72 g of the captioned compound.
Refractive index 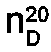 : 1.5053.
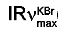 (cm⁻¹): 3030, 2930, 1680, 1610, 1515, 1450, 1390, 1290, 1260, 1200, 1140, 1105, 1095, 1045, 975,940,890,845,815,750,720.
¹H NMR 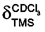 (ppm): 2.32(3H, s), 3.74(2H, q, J=8Hz), 4.74(2H, s), 5.24(2H, s), 7.17(4H, s), 7.28(2H, d, J_{AB}=2Hz), 7.38(2H, d, J_{AB}=2Hz).

As an isomer, 0.16 g of 2-(4-pentafluoroethylbenzylimino)-3-(2,2,2-trifluoroethyl)-5-(4-methylphenyl)-tet- rahydro-1,3,5-thiadiazin-4-one was obtained.
Melting point: 102.9-103.6 °C.
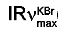 (cm⁻¹): 3030, 2980, 2800, 1700, 1625, 1510, 1450, 1395, 1335, 1290, 1260, 1210, 1170, 1120, 1050,970,950,815,750,730.
¹H NMR 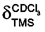 (ppm): 2.36(3H, s), 4.54(2H, s), 4.82(2H, s), 5.06(2H, q, J=8Hz), 7.18(4H, s), 7.44(2H, d, J_{AB}=8Hz), 7.60(2H, d, J_{AB}=8Hz).

### EXAMPLE 7

Synthesis of 2-(2,2,2-trifluoroethylimino)-2-[4-(2-chloro-2-propeneoxy)benzyl)-5-(4-methylphenyl)tetrahydro-1,3,5-thiadiazin-4-one (compound No. 111):-
0.65 g of N-chloromethyl-N-(4-methylphenyl)carbamoyl chloride and 1.0 g of 1-[4-(2-chloro-2-propene- oxy)benzyl]-3-(2,2,2-trifluoroethyl)thiourea were dissolved in 30 ml of toluene, and the solution was heated under reflux for 3 hours. After the reaction, toluene was evaporated under reduced pressure. The resulting oily product was purified by column chromatography [silica gel; developing solvent hexane/ethyl acetate (10:1)] to give 0.82 g of the captioned compound.
Refractive index 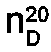 : 1.5641.
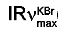 (cm⁻¹): 1680, 1610, 1510, 1450, 1390, 1360, 1290, 1265, 1240, 1210, 1170, 1140, 1090, 1045, 940,890,845,820,760,745,720.
¹H NMR 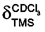 (ppm): 2.32(3H, s), 3.77(2H, q, J=10Hz), 4.52(2H, s), 4.72(2H, s), 5.20(2H, s), 5.40(1H, s), 5.54(1 H, s), 6.86(2H, d, J_{AB}=9Hz), 7.10(4H, s), 7.48(2H, d, J_{AB}=9Hz).

As an isomer, 0.14 g of 2-[4-(2-chloro-2-propeneoxy)benzylimino)-3-(2,2,2-trifluoroethyl)-5-(4-methylphenyl)-tetrahydro-1,3,5-thiadiazin-4-one was obtained as a semisolid.
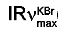 (cm⁻¹): 1690, 1615, 1510, 1440, 1390, 1300, 1260, 1220, 1160, 1115, 1160, 1115, 1045, 890, 820, 765, 720.
¹H NMR 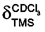 (ppm): 2.36(3H, s), 4.46(2H, s), 4.60(2H, s), 4.80(2H, s), 5.07(2H, q, J=10Hz), 5.42(1H, s), 5.57(1 H, s), 6.94(2H, d, J_{AB}=10Hz), 7.22(4H, s), 7.26(2H, d, J_{A}ε=10Hz).

### EXAMPLE 8

Synthesis of 2-(2,2,2-trifluoroethylimino)-3-(4-t-butoxycarbonylbenzyl)-5-(4-methylphenyl)-tetrahydro-1,3,5-thiadiazin-4-one (compound No. 146):-
0.31 g of N-chloromethyl-N-(4-methylphenyl)carbamoyl chloride and 0.49 g of 1-(4-t-butoxycarbonylben- zyl)-3-(2,2,2-trifluoroethyl)thiourea were dissolved in 30 ml of benzene, and the solution was heated under reflux for 5 hours. After the reaction, benzene was evaporated under reduced pressure. The resulting oily product was purified by column chromatography [silica gel; developing solvent hexane/ethyl acetate (9:1)] to obtain 0.34 g of the captioned compound.
Refractive index 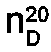 : 1.5329.
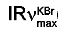 (cm-¹): 1700, 1670, 1605, 1505, 1440, 1380, 1360, 1305, 1290, 1260, 1200, 1160, 1140, 1110, 1085,1040,1010,990,930,840,810.
¹H NMR 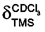 (ppm): 1.57(9H, s), 2.35(3H, s), 3.77(2H, q, J=10Hz), 4.80(2H, s), 5.27(2H, s), 7.21(4H, s), 7.48(2H, d, J_{AB}=8Hz), 7.94(2H, d, J_{AB}=8Hz).

As an isomer, 0.10 g of 2-(4-t-butoxycarbonylbenzylimino)-3-(2,2,2-trifluoroethyl)-5-(4-methylphenyl)-tet- rahydro-1,3,5-thiadiazin-4-one was obtained.
Melting point: 105.0-108.0 °C.
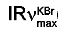 : 1690, 1610, 1505, 1440, 1390, 1360, 1290, 1265, 1200, 1155, 1110, 1070, 1040, 1010, 980,965,840,830,810,745.
¹H NMR 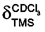 (ppm): 1.60(9H, s), 2.37(3H, s), 4.56(2H, s), 4.84(2H, s), 5.09(2H, q, J=10Hz), 7.24(4H, s), 7.43(2H, d, J_{AB}=9Hz), 8.04(2H, d, J_{AB}=9Hz).

### EXAMPLE 9

Synthesis of 2-(2,2,2-trifluoroethylimino)-3-(4-trifluoromethoxybenzyl)-5-(2-fluoro-4-methylphenyl)tetrahydro-1,3,5-thiadiazin-4-one (compound No. 168):-
0.60 g of N-chloromethyl-N-(2-fluoro-4-methylphenyl)carbamoyl chloride and 0.84 g of 1-(4-trifluoromethoxybenzyl)-3-(2,2,2-trifluoroethyl)thiourea were dissolved in 50 ml of toluene, and the solution was heated under reflux for 5 hours. After the reaction, toluene was evaporated under reduced pressure. The resulting oily product was purified by column chromatography [silica gel; developing solvent hexane/ethyl acetate (9:1)] to give 0.68 g of the captioned compound.
Refractive index 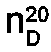 : 1.5180.
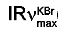 (cm⁻¹): 1685, 1615, 1510, 1450, 1390, 1360, 1260, 1220, 1140, 1100, 1080, 1040, 1015, 935, 840, 810,745.
¹H NMR 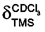 (ppm): 2.36(3H, s), 3.76(2H, q, J=9.6Hz), 4.70(2H, s), 5.20(2H, s), 6.92-7.24 (5H, m), 7.43(2H, d, J=9Hz).

As an isomer, 0.17 g of 2-(4-trifluoromethoxybenzylimino)-3-(2,2,2-trifluoroethyl)-5-(2-fluoro-4-methylphenyl)-tetrahydro-1,3,5-thiadiazin-4-one was obtained as a semisolid.
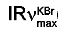 (cm⁻¹): 1710, 1615, 1590, 1510, 1445, 1390, 1360, 1330, 1270, 1250, 1220, 1150, 1105, 1035, 830,815,755.
¹H NMR 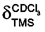 (ppm): 2.38(3H, s), 4.52(2H, s), 4.76(2H, s), 5.08(2H, d, J=9Hz), 6.91-7.37 (7H, m).

### EXAMPLE 10

Synthesis of 2-(2,2,2-trifluoroethylimino)-3-[3-fluoro-4-(2,2,2-trifluoroethoxy)benzyl)-5-(4-methylphenyl)-tet- rahydro-1,3,5-thiadiazin-4-one (compound No. 212):-
0.6 g of N-chloromethyl-N-(4-methylphenyl)carbamoyl chloride and 1.00 g of 1-[3-fluoro-4-(trifluoroethoxy)benzyl]-3-(2,2,2-trifluoroethyl)thiourea were dissolved in 50 ml of toluene, and the solution was heated under reflux for 3 hours. After the reaction, toluene was evaporated under reduced pressure. The resulting oily product was purified by column chromatography [silica gel; developing solvent hexane/ethyl acetate (4:1)] to give 0.63 g of the captioned compound.
Melting point: 94.1-95.6 °C.
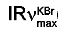 (cm⁻¹): 1665, 1605, 1515, 1465, 1440, 1425, 1410, 1315, 1290, 1265, 1170, 1145, 1125, 965, 935, 830, 765, 755.
¹H NMR 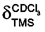 (ppm): 2.36(3H, s), 3.81(2H, q, J=9Hz), 4.37(2H, q, J=8Hz), 4.80(2H, s), 5.16(2H, s), 6.90-7.40(7H, m).

As an isomer, 0.04 g of 2-[3-fluoro-4-(2,2,2-trifluoroethoxy)benzylimino]-3-(2,2,2-trifluoroethyl)-5-(4-methylphenyl)-tetrahydro-1,3,5-thiadiazin-4-one was obtained.
Refractive index 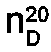 : 1.5196.
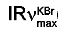 (cm⁻¹): 1690, 1620, 1515, 1445, 1395, 1290, 1265, 1215, 1165, 1115, 1055, 975, 865, 820, 775. ¹H NMR 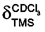 (ppm): 2.35(3H, s), 4.39(2H, q, J=8Hz), 4.44(2H, s), 4.82(2H, s), 5.06(2H, q, J=9Hz), 7.00-7.30(7H, m).

Table 2 below shows the ¹H NMR data, IR data and properties of compounds produced by methods substantially in accordance with the methods described in Examples 1 to 10.

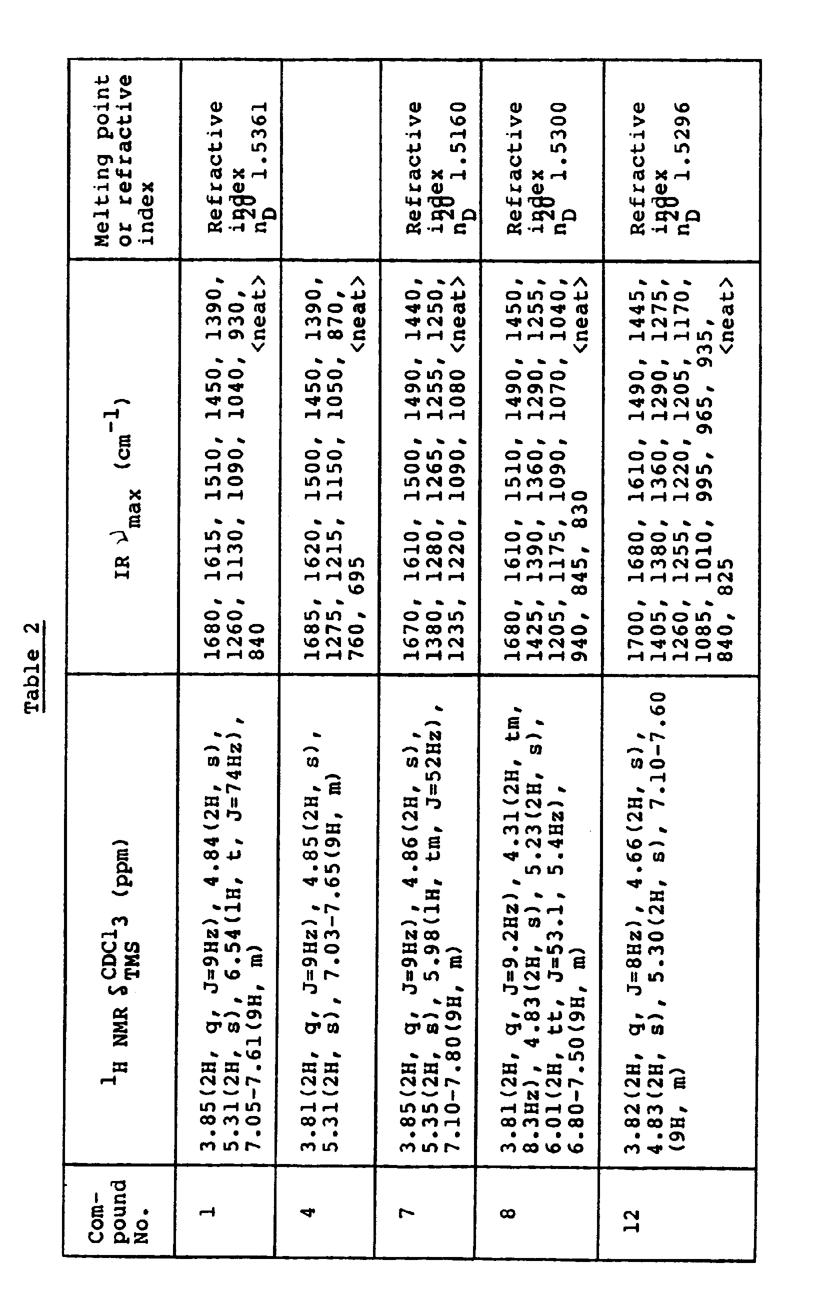

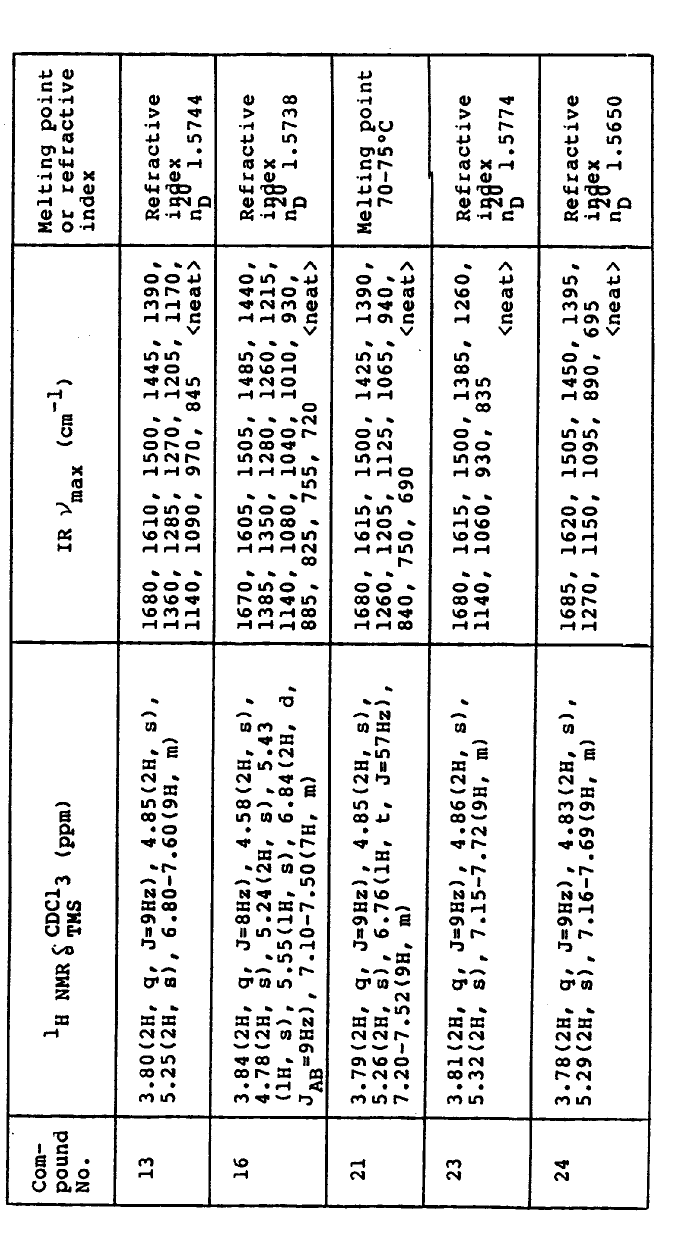

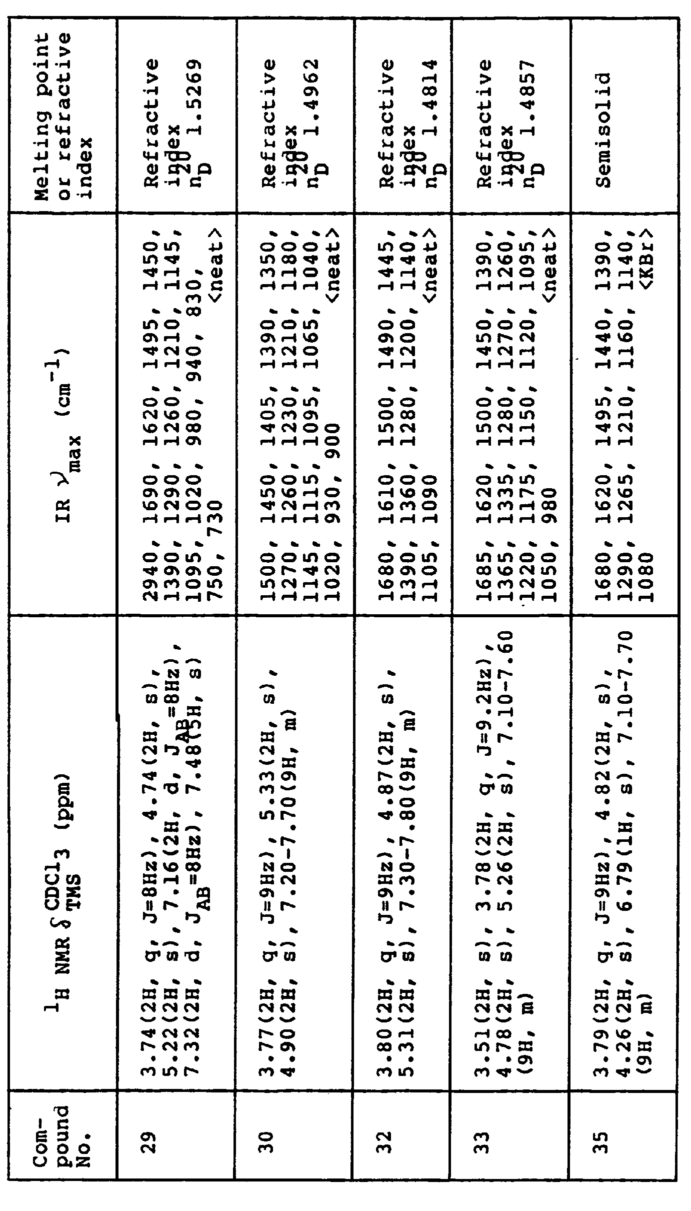

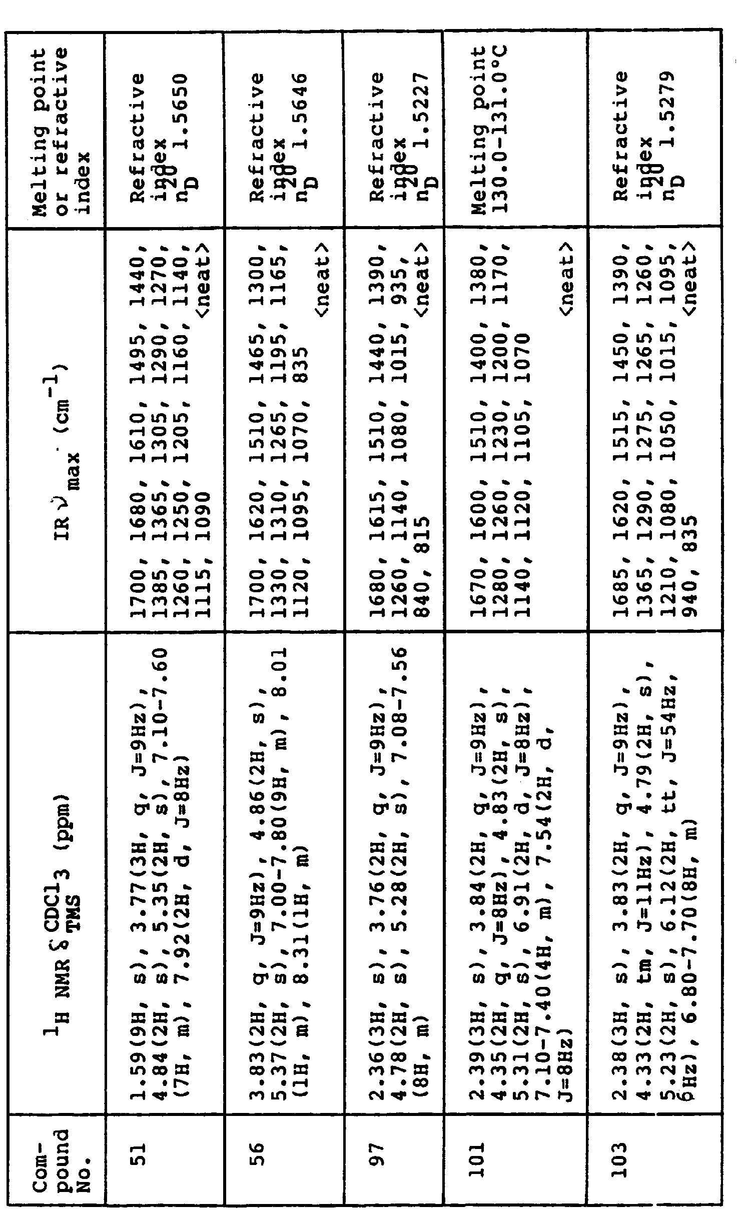

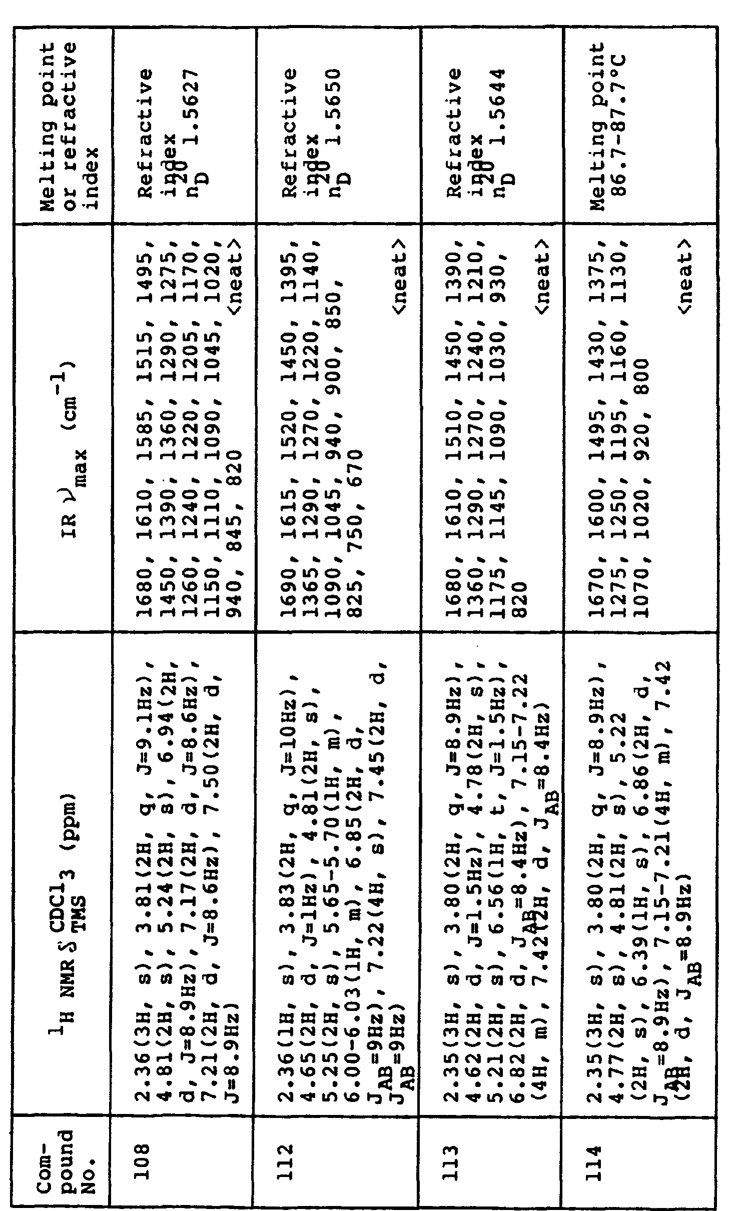

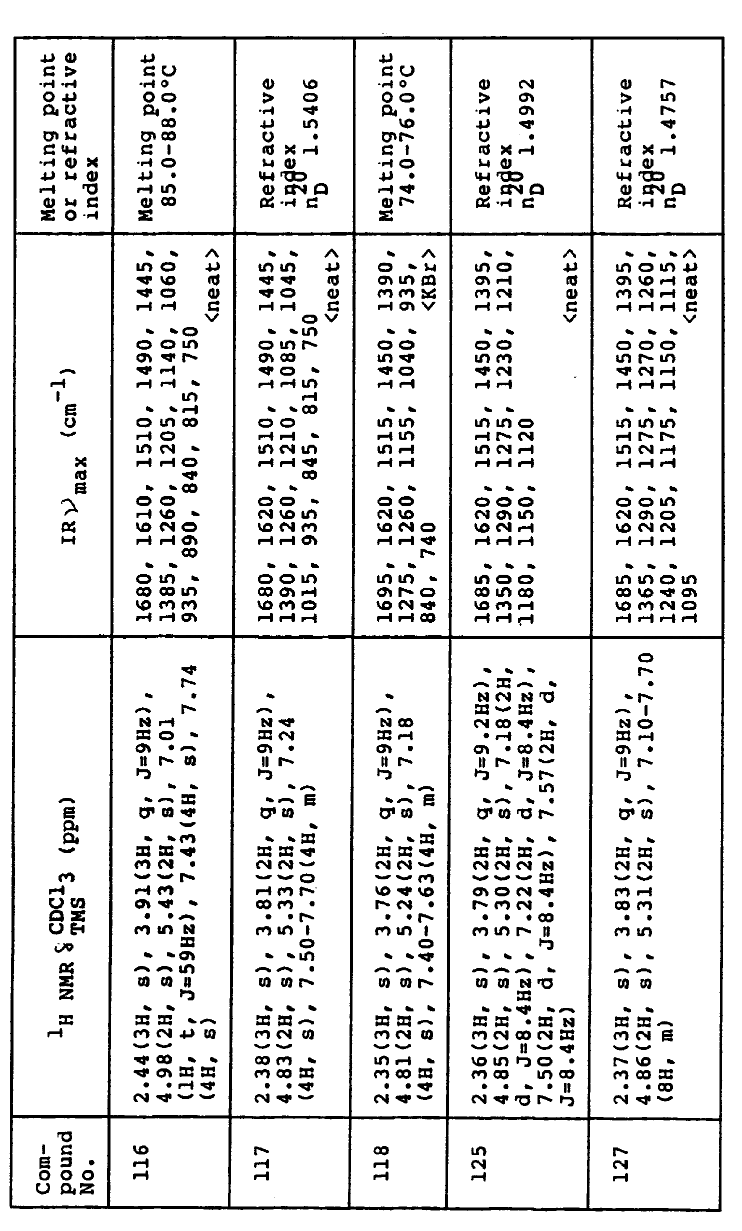

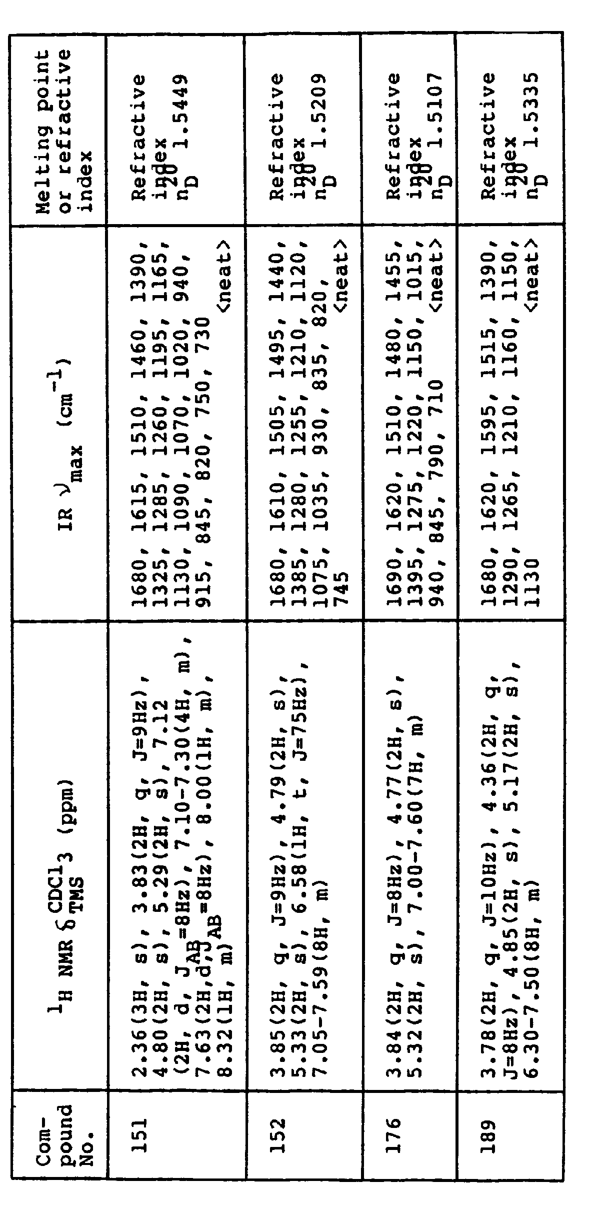

The following Referential Examples illustrate the production of the starting materials for the compounds of this invention.

### REFERENTIAL EXAMPLE 1

### Synthesis of 1-(4-trifluoromethoxybenzyl)-3-(2,2,2-trifluoroethyl)thiourea:-

### (1) N-(4-trifluoromethoxybenzyl)phthalimide

Phosphorus tribromide (5.17 g) was added dropwise to 10.0 g of 4-trifluoromethoxybenzyl alcohol at 10 °C over 10 minutes, and the mixture was stirred at room temperature for 2 hours. The reaction solution was poured into ice water, and extracted with 100 ml of hexane. The hexane solution was washed with water and dried, and hexane was evaporated under reduced pressure to give 13.13 g of white crystals. The crystals were dissolved in 60 ml of dimethylformamide, and 19.26 g of potassium phthalimide and 1 g of potassium iodide were added, and the mixture was heated at 100 °C for 3 hours with stirring. Water (50 ml) was added to the reaction solution, and the mixture was extracted with 200 ml of ethyl acetate. The ethyl acetate solution was washed with water and dried. Ethyl acetate was evaporated under reduced pressure. The resulting oily product was purified by column chromatography [silica gel; developing solvent hexane/ethyl acetate (5:1)] to give 14.17 g of the captioned compound.
Melting point: 82.1-83.6 °C.
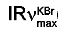 (cm⁻¹): 1690, 1620, 1515, 1505, 1440, 1425, 1410, 1395, 1300, 1265, 1260, 1205, 1180, 1160, 1110.
¹H NMR 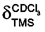 (ppm): 4.83(2H, s), 7.04-7.85(8H, m).

### (2) 4-Trifluoromethoxybenzyl isothiocyanate

12 g of N-(4-trifluoromethoxybenyl)phthalimide was dissolved in 50 ml of ethanol, and 2.34 g of hydrazine hydrate was added dropwise. Then, the mixture was heated under reflux for 4 hours and then allowed to cool. Concentric hydrochloric acid (10 ml) was added, and the mixture was filtered. The ethanol solution as the filtrate was evaporated under reduced pressure, and the residue was adjusted to pH 11 by adding an aqueous solution of sodium hydroxide. Ethyl acetate (100 ml) was added, and the solution was washed with water and dried. Ethyl acetate was evaporated under reduced pressure to give 6.55 g of an oily product. The resulting oily product (6.55 g) was added dropwise to a mixture of 10.32 g of dicyclohexylcarbodiimide (DCC), 20 ml of carbon disulfide and 100 ml of ethyl ether with stirring at-10 °C. The temperature was returned to room temperature, and the mixture was left to stand for 12 hours.

The reaction mixture was filtered, and the residue was washed with ethyl ether, and combined with the filtrate. The solvent was evaporated under reduced pressure. The resulting oily product was purified by column chromatography [silica gel; developing solvent hexane/ethyl acetate (19: 1)] to give 7.53 g of the captioned compound.
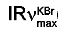 (cm⁻¹): 2080, 1510, 1440, 1340, 1260, 1215, 1160, 1015, 920.
¹H NMR 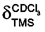 (ppm): 4.69(2H, s), 7.28(2H, d, J_{AB}=9Hz), 7.40(2H, d, J_{AB}=9Hz).
Reference: Angewandte Chemie International Edition, Vol. 6, page 174 (1967).

### (3) 1-(4-trifluoromethoxybenzyl)-3-(2,2,2-trifluoroethyl)thiourea

6.0 g of 4-trifluoromethoxybenzyl isothiocyanate obtained in (2) above and 3.06 g of 2,2,2-trifluoroethylamine were dissolved in 50 ml of ethyl acetate, and the solution was left to stand at room temperature for 24 hours. Ethyl acetate was evaporated under reduced pressure, and the resulting white crystals were recrystallized from hexane to give 6.05 g of the captioned compound.
Melting point: 94.7-96.4 °C.
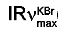 (cm⁻¹): 3240, 3060, 1570, 1510, 1300, 1250, 1180, 1170, 1150, 1110, 975, 920, 825, 680.
¹H NMR 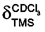 (ppm): 4.31(2H, d, J=9Hz), 4.66 (2H, s), 6.30(1H, m), 6.74(1H, m), 7.12-7.55 (9H, m).

### REFERENTIAL EXAMPLE 2

### Synthesis of 1-(4-trifluoromethylthiobenzyl)-3-(2,2,2-trifluoroethyl)thiourea:-

### (1) N-(4-trifluoromethylthiobenzyl)phthalimide

15.5 g of 4-trifluoromethylthiotoluene and 17.2 g of N-bromosuccinimide were added to 100 ml of benzene, and about 100 mg of 2,2'-azobisisobutyronitrile was added. The mixture was gradually heated. When the temperature reached about 86 °C, the reaction solution turned red yellow. It was stirred at the same temperature for 40 minutes. The reaction solution was poured into ice water and extracted with 200 ml of ethyl acetate. The ethyl acetate solution was washed with water and dried. Ethyl acetate was evaporated under reduced pressure to give 21 g of an oily product. This product was dissolved in 50 ml of dimethylformamide, and 14.9 g of potassium phthalimide and 1 g of potassium iodide were added. The mixture was stirred at 60 °C for 1 hour. Water (50 ml) was added to the reaction solution, and the mixture was extracted with 200 ml of toluene. The toluene solution was washed with water and dried, and toluene was evaporated under reduced pressure to give 21.0 g of the captioned compound.
Melting point: 130.0-131.5 °C.
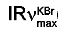 (cm⁻¹): 3180, 1765, 1710, 1600, 1465, 1430, 1390, 1340, 1325, 1150, 1120, 1080, 935, 735, 710.
¹H NMR 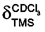 (ppm): 4.92(2H, s), 7.40-7.90(8H, m).

### (2) 4-Trifluoromethylthiobenzyl isothiocyanate

18 g of N-(4-trifluoromethylthiobenzyl)phthalimide was dissolved in 200 ml of ethanol, and 4 g of hydrazine hydrate was added dropwise. The mixture was then heated under reflux for 3 hours, and then allowed to cool. Concentrated hydrochloric acid (10 ml) was added, and the mixture was heated under reflux for 4 hours. The precipitate formed was separated by filtration, and the ethanol solution as the filtrate was evaporated under reduced pressure. The residue was dissolved in 100 ml of water. The solution was washed with 50 ml of hexane and adjusted to pH 11 with an aqueous solution of sodium hydroxide. Ethyl acetate (100 ml) was added, and the mixture was washed with water and dried. Ethyl acetate was evaporated under reduced pressure to give 8.0 g of an oily product. The resulting product (8.0 g) was added dropwise to a mixed solution of 7.9 g of DCC, 40 ml of carbon disulfide and 20 ml of ethyl ether with stirring and ice cooling to 25 °C or below. The temperature of the mixture was returned to room temperature, and it was left to stand for 12 hours. The reaction mixture was filtered, and the residue was washed with ethyl ether and combined with the filtrate. The solvent was evaporated under reduced pressure. The resulting oily product was purified by column chromatography (silica gel; developing solvent hexane) to give 6.0 g of 4-trifluoromethylthiobenzyl isothiocyanate.
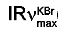 (cm⁻¹): 2120, 1500, 1440, 1340, 1305, 1160, 1120, 1085, 1020.
¹H NMR 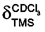 (ppm): 4.76(2H, s), 7.34(2H, d, J=9Hz), 7.66(2H, d, J=9Hz).

### (3) 1-(4-Trifluoromethylthiobenzyl)-3-(2,2,2-trifluoroethyl)thiourea

6.0 g of 4-trifluoromethylthiobenzyl isothiocyanate obtained in (2) above and 2.5 g of 2,2,2-trifluoroethylamine was dissolved in 20 ml of ethyl acetate, and the solution was left to stand at room temperature for 24 hours. Ethyl acetate was evaporated under reduced pressure. The resulting white crystals were recrystallized from hexane to give 6.66 g of the captioned compound.
Melting point: 113.5-114.5 °C.
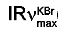 (cm⁻¹): 3260, 3070, 1565, 1380, 1300, 1255, 1140, 1120, 1090, 1020, 960, 855.
¹H NMR 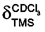 (ppm): 4.34(2H, dq, J=9Hz), 4.72(2H, d, J=8Hz), 6.00-6.20(1 H, m), 6.60-6.80(1 H, m), 7.38(2H, d, J=8Hz), 7.68(2H, d, J=8Hz).

### REFERENTIAL EXAMPLE 3

Synthesis of 1-[4-(2,2,2-trifluoroethoxy)benzyl)-3-(2,2,2-trifluoroethyl)thiourea:
Lithium aluminum hydride (2.0 g) was added to 100 ml of dry ethyl ether, and with ice cooling and stirring, a solution of 10.0 g of 4-(2,2,2-trifluoroethoxy)benzonitrile in 50 ml of dry ethyl ether was added dropwise over 30 minutes. The excess of lithium aluminum hydride was decomposed with methanol and water, and the reaction solution was extracted with 100 ml of ethyl ether. The ethyl ether solution was washed with water and dried, and ethyl ether was evaporated under reduced pressure to give 11.34 g of an oily product. A portion (5.63 g) of the resulting oily product was added dropwise to a mixture of 5.60 g of DCC, 13 ml of carbon disulfide and 30 ml of ethyl ether with stirring at -10 °C. The temperature of the mixture was returned to room temperature, and it was left to stand for 12 hours. The reaction solution was filtered, and the residue was washed with ethyl ether and combined with the filtrate. The solvent was evaporated under reduced pressure. The resulting oily product was purified by column chromatography [silica gel; developing solvent hexane/ethyl acetate (9:1)] to give 7.20 g of a purified product. This purified product and 4.00 g of 2,2,2-trifluoroethylamine were dissolved in 20 ml of ethyl acetate. The solution was left to stand at room temperature for 24 hours. Ethyl acetate was evaporated under reduced pressure. The resulting white crystals were recrystallized from hexane to give 9.54 g of the captioned compound.
Melting point: 119.0-120.5 °C.
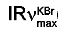 (cm⁻¹): 3320, 3300, 1610, 1560, 1515, 1460, 1365, 1290, 1250, 1240, 1180, 1165, 1130, 1080, 975.
¹H NMR 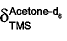 (ppm): 4.30-4.90(6H, m), 7.01 (2H, d, J=9Hz), 7.34(2H, d, J=9Hz), 7.20-7.80 (2H, br).

### REFERENTIAL EXAMPLE 4

### Synthesis of 1-(4-pentafluoroethylbenzyl)-3-(2,2,2-trifluoroethyl)thiourea:-

### (1) 4-pentafluoroethylbenzonitrile

15.0 g of sodium pentafluoropropionate and 15.8 g of copper iodide were dried under reduced pressure, and a solution of 4.9 g of 4-bromobenzonitrile in 80 ml of N-methylpyrrolidone was added. The reaction solution was stirred at 162 °C for 5 hours in a current of dry argon. The reaction mixture was allowed to cool, and then 300 ml of diethyl ether and 200 ml of water were added. The insoluble matter was removed by filtration. The filtrate was successively washed with water and a saturated aqueous solution of sodium chloride, and dried. Diethyl ether was evaporated under reduced pressure, and the resulting oily product was purified by column chromatography [slica gel; developing solvent hexane/ethyl acetate (9:1)] to give 3.2 g of the desired 4-pentafluoroethylbenzonitrile as an oily product (yield 53.4 %).
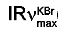 (cm⁻¹): 2215, 1410, 1345, 1330, 1280, 1205, 1155, 1100, 985, 960, 835, 740.
¹H NMR 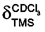 (ppm): 7.70(2H, d, J_{AB}=8Hz), 7.84(2H, d, J_{AB}=8Hz).

### (2) 4-Pentafluoroethylbenzylamine

0.79 g of lithium aluminum hydride was suspended in 50 ml of diethyl ether, and a solution of 4.6 g of 4-pentafluoroethylbenzonitrile in 30 ml of diethyl ether was added dropwise. After the addition, the mixture was stirred at room temperature for 30 minutes, and 5 ml of water was added dropwise over 30 minutes. The insoluble matter was removed by filtration. The ether layer was dried, and then diethyl ether was evaporated under reduced pressure to give 4.7 g of the desired 4-pentafluoroethylbenzylamine as an oil.
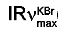 (cm⁻¹): 3350, 1610, 1575, 1470, 1415, 1340, 1285, 1200, 1140, 1090, 970, 805, 740.
¹H NMR 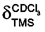 (ppm): 1.40(2H, b), 3.90(2H, b), 7.32 (2H, d, J_{AB}=8Hz).

### (3) 4-Pentafluoroethylbenzyl isothiocyanate

4.7 g of 4-pentafluoroethylbenzylamine was added dropwise to a mixture of 4.8 g of DCC, 20 ml of carbon disulfide and 50 ml of diethyl ether with stirring at -10 °C. The temperature of the mixture was returned to room temperature, and it was left to stand for 12 hours.

The reaction solution was filtered, and the residue was washed with diethyl ether and combined with the filtrate. The solvent was evaporated under reduced pressure, and the resulting oily product was purified by column chromatography (silica gel; developing solvent hexane) to give 2.1 g of the desired 4-pentafluoroethylbenzyl isothiocyanate as an oil.
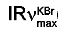 (cm⁻¹): 2920, 2190, 2080, 1615, 1415, 1340, 1285, 1200, 1145, 1095, 970, 805, 745.
¹H NMR 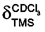 (ppm): 4.70(2H, s), 7.30(2H, d, J_{AB}=8Hz), 7.50(2H, d, J_{AB}=8Hz).

### 4) 1-(4-Pentafluoroethylbenzyl)-3-(2,2,2-trifluoroethylthiourea

2.1 g of 4-pentafluoroethylbenzyl isothiocyanate and 0.78 g of 2,2,2-trifluoroethylamine were dissolved in 20 ml of ethyl acetate, and the solution was left to stand at room temperature for 24 hours. Ethyl acetate was evaporated under reduced pressure. The resulting white crystals were recrystallized from hexane to give 2.6 g of the captioned compound.
Melting point: 97.3-98.8 °C.
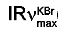 (cm⁻¹): 3240, 3070, 1570, 1395, 1290, 1250, 1215, 1200, 1165, 1130, 1090, 975, 935, 815, 745.
¹H NMR 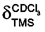 (ppm): 4.1-4.5(2H, m), 4.74(2H, d, J=6Hz), 5.70(1 H, b), 6.25(1 H, b), 7.34(2H, d, J_{AB}=8Hz), 7.52(2H, d, J_{AB}=8Hz).

### REFERENTIAL EXAMPLE 5

### Synthesis of 1-[4-(2-chloro-2-propeneoxy)benzyl]-3-(2,2,2-trifluoroethyl)thiourea:-

### 1) 4-(2-Chloro-2-propeneoxy)benzonitrile

10.0 g of 4-cyanophenol and 11.6 g of potassium carbonate were suspended in 100 ml of dimethylformamide, and 9.4 g of 2,3-dichloropropene was added dropwise. The reaction solution was stirred at 80 °C for 3 hours, allowed to cool, and poured into 200 ml of water, followed by extraction with ethyl acetate. The organic layer was washed with a saturated aqueous solution of sodium chloride and dried. Ethyl acetate was evaporated under reduced pressure. The resulting oily product was purified by column chromatography (silica gel; developing solvent hexane/ethyl acetate (9:1)] to give 15.2 g of the desired 4-(2-chloro-2-propeneoxy)benzonitrile as an oil.
Refractive index 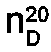 : 1.5607.
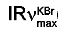 (cm⁻¹): 2210, 1630, 1600, 1570, 1505, 1450, 1300, 1255, 1225, 1165, 1040, 1020, 890, 830.
¹H NMR 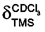 (ppm): 4.65(2H, s), 5.46(1 H, d, J=2Hz), 5.53(1 H d, J=2Hz), 6.96(2H, d, J_{AB}=10Hz), 7.56 (2H, d, J_{AB}=10Hz).

### (2) 4-(2-Chloro-2-propeneoxy)benzylamine

1.36 g of lithium aluminum hydride was suspended in 50 ml of diethyl ether, and a solution of 7.0 g of 4-(2-chloro-2-propeneoxy)benzonitrile in 40 ml of diethyl ether was added dropwise. After the addition, the mixture was stirred at room temperature for 30 minutes and then 10 ml of water was added dropwise for 30 minutes. The insoluble matter was removed by filtration. The ether layer was dried and diethyl ether was evaporated under reduced pressure to give 6.7 g of the desired 4-(2-chloro-2-propeneoxy)benzylamine as an oil.
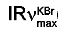 (cm⁻¹): 3360, 3260, 2920, 2860, 1635, 1605, 1580, 1505, 1450, 1385, 1300, 1240, 1215, 1170, 1045,890,820,715,690.
¹H NMR 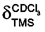 (ppm): 3.72(2H, b), 4.48(2H, s), 5.28 (1H d, J=2Hz), 5.40(1 H, d, J=2Hz), 6.72(2H, d, J_{AB}=9Hz), 7.06(2H, d, J_{AB}=9Hz).

### (3) 4-(2-Chloro-2-propeneoxy)benzyl isothiocyanate

6.7 g of 4-(2-chloro-2-propeneoxy)benzylamine was added dropwise to a mixture of 7.7 g of DCC, 20 ml of carbon disulfide and 100 ml of diethyl ether with stirring at -1 °C. The temperature of the mixture was returned to room temperature, and it was left to stand for 12 hours. The reaction solution was filtered, and the residue was washed with diethyl ether and combined with the filtrate. The solvent was evaporated under reduced pressure. The resulting oily product was purified by column chromatography (silica gel; developing solvent hexane) to give 6.6 g of the desired 4-(2-chloro-2-propeneoxy)benzyl isothiocyanate as an oil.
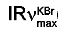 (cm⁻¹): 2160, 2070, 1690, 1640, 1610, 1510, 1450, 1445, 1350, 1300, 1245, 1220, 1170, 890, 850, 820.
¹H NMR 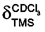 (ppm): 4.55(2H, s), 4.60(2H, s), 5.38(1 H, d, J=2Hz), 5.49(1 H, d, J=2Hz), 6.86 (2H, d, J_{AB}=9Hz), 7.17(2H, d,J_{AB}=9Hz).

### (4) 1-[4-(2-chloro-2-propenoxy)benzy]]-3-(2,2,2-trifluoroethyl)thiourea

6.6 g of 4-(2-chloro-2-propeneoxy)benzyl isothiocyanate and 2.7 g of 2,2,2-trifluoroethylamine were dissolved in 100 ml of ethyl acetate, and the solution was left to stand at room temperature for 24 hours. Ethyl acetate was evaporated under reduced pressure, and the resulting white crystals were recrystallized from hexane to give 5.9 g of the captioned compound.
Melting point: 83.4-84.9 °C.
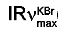 (cm⁻¹): 3240, 3070, 1605, 1565, 1505, 1380, 1350, 1295, 1250, 1225, 1165, 1115, 1040, 960, 900, 820.
¹H NMR 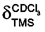 (ppm): 4.30(2H, dq, J=6Hz, 9Hz), 4.52(2H, d, J=6Hz), 4.54(2H, s), 5.40(1 H, d, J=2Hz), 5.51 (1 H d, J=2Hz), 5.94(1H, b), 6.56 (1 H, b), 6.94(2H, d, J_{AB}=9Hz), 7.24(2H, d, J_{AB}=9Hz).

### REFERENTIAL EXAMPLE 6

### Synthesis of 1-[3-fluoro-4-(2,2,2-trifluoroethoxy)benzyl]-3-(2,2,2-trifluoroethyl)thiourea:-

### 1) 3-Fluoro-4-(2,2,2-trifluoroethoxy)benzonitrile

2.2 g of oily sodium hydride (60 %) was suspended in 20 ml of dimethylformamide, and 5.50 g of trifluoroethanol was added dropwise at 20 °C. The mixture was stirred for 1 hour, and then, a solution of 6.96 g of 4-difluorobenzonitrile in 10 ml of dimethylformamide was added dropwise. The reaction temperature rose up to 30 °C. The reaction solution was stirred for 5 hours, and then 2 ml of acetic acid was added. The mixture was poured into 100 ml of ice water and extracted with toluene. The organic layer was dried and toluene was evaporated under reduced pressure. The resulting crude crystals were washed with hexane to give 10.30 g of the desired 3-fluoro-4-(2,2,2-trifluoroethoxy)benzonitrile.
Melting point: 62.0-67.0 °C.
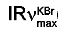 (cm⁻¹): 3040, 2220, 1605, 1580, 1515, 1505, 1455, 1420, 1315, 1275, 1250, 1220, 1180, 1150, 1120, 1050, 960.
¹H NMR 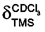 (ppm): 4.46(2H, q, J=8Hz), 7.00-7.60 (3H, m).

### (2) 3-Fluoro-4-(2,2,2-trifluoroethoxy)benzyl isothiocyanate

1.40 g of lithium aluminum hydride was suspended in 50 ml of diethyl ether, and a solution of 8.00 g of 3-fluoro-4-(2,2,2-trifluoroethoxy)benzonitrile in 10 ml of diethyl ether was added dropwise. After the addition, the mixture was stirred at room temperature for 30 minutes and subsequently, 10 ml of water was added dropwise over 30 minutes. The insoluble matter was removed by filtration. The ether layer was dried. Diethyl ether was evaporated under reduced pressure to give 7.54 g of crude 3-fluoro-4-(2,2,2-trifluoroethoxy)benzylamine as an oil.

The crude 3-fluoro-4-(2,2,2-trifluoroethoxy)benzylamine (7.54 g) was added dropwise to a mixture of 7.45 g of DCC, 15 ml of carbon disulfide and 30 ml of diethyl ether with stirring at -10 °C. The temperature of the mixture was returned to room temperature, and it was left to stand for 3 hours. The reaction mixture was filtered, and the residue was washed with ethyl ether and combined with the filtrate. The solvent was evaporated under reduced pressure. The resulting oily product was purified by column chromatography (silica gel; developing solvent hexane) to give 3.93 g of the desired 3-fluoro-4-(2,2,2-trifluoroethoxy)benzyl isothiocyanate.
Melting point: 38.0-40.0 °C.
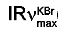 (cm-¹): 2160, 20₇0, 1₅0₅, 143₅, 142₅, 133₅, 1310, 1280, 12₇0, 12₅0, 1210, 11₇0, 114₅, 1120, 1055, 960, 850.
¹H NMR 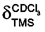 (ppm): 4.42(2H, q, J=8Hz), 4.65 (2H, s), 6.80-7.40(3H, m).

### (3) 1-[3-Fluoro-4-(2,2,2-trifluoroethoxy)benzyl]-3-(2,2,2-trifluoroethyl)thiourea

3.15 g of 3-fluoro-4-(2,2,2-trifluoroethoxy)benzyl isothiocyanate and 1.5 ml of 2,2,2-trifluoroethylamine were dissolved in 20 ml of ethyl acetate, and the solution was left to stand for 24 hours. Ethyl acetate was evaporated under reduced pressure. The resulting white crystals were recrystallized from hexane to give 3.92 g of the desired 1-[3-fluoro-4-(2,2,2-trifluoroethoxy)benzyl]-3-(2,2,2-trifluoroethyl)thiourea.
Melting point: 88.0-90.0 °C.
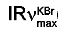 (cm⁻¹): 3260, 3080, 1600, 1570, 1525, 1440, 1390, 1350, 1310, 1290, 1270, 1255, 1195, 1180, 1165, 1135, 1125, 975.
¹H NMR 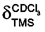 (ppm): 4.50(4H, q, J=9Hz), 4.81(2H, d, J=6Hz), 6.90-7.70(5H. m).

Table 3 shows the ¹H NMR data, IR data and properties of compounds produced by methods substantially in accordance with Referential Examples 1 to 6.

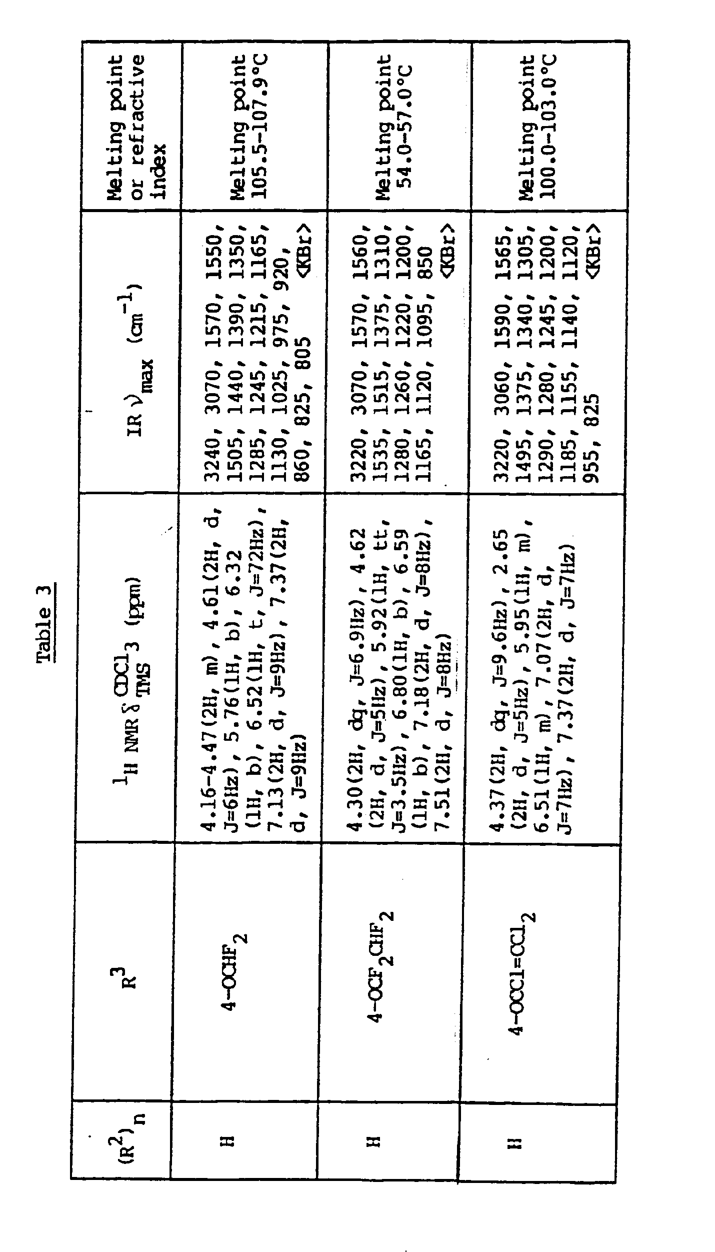

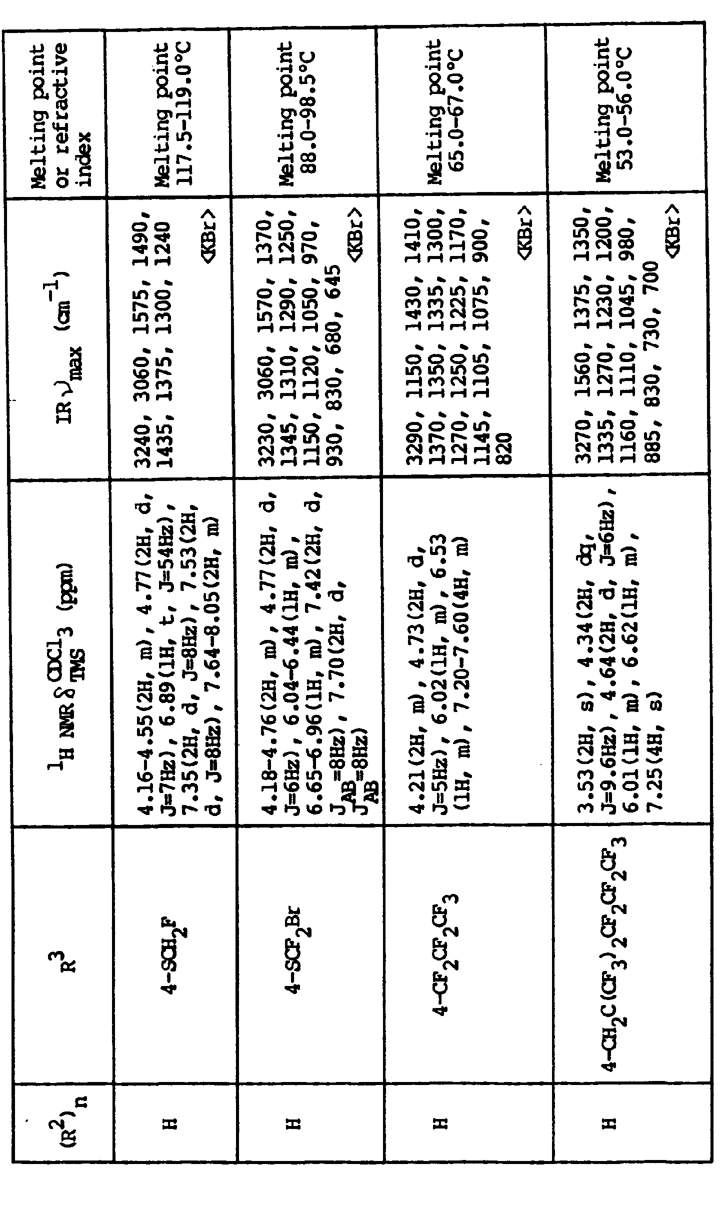

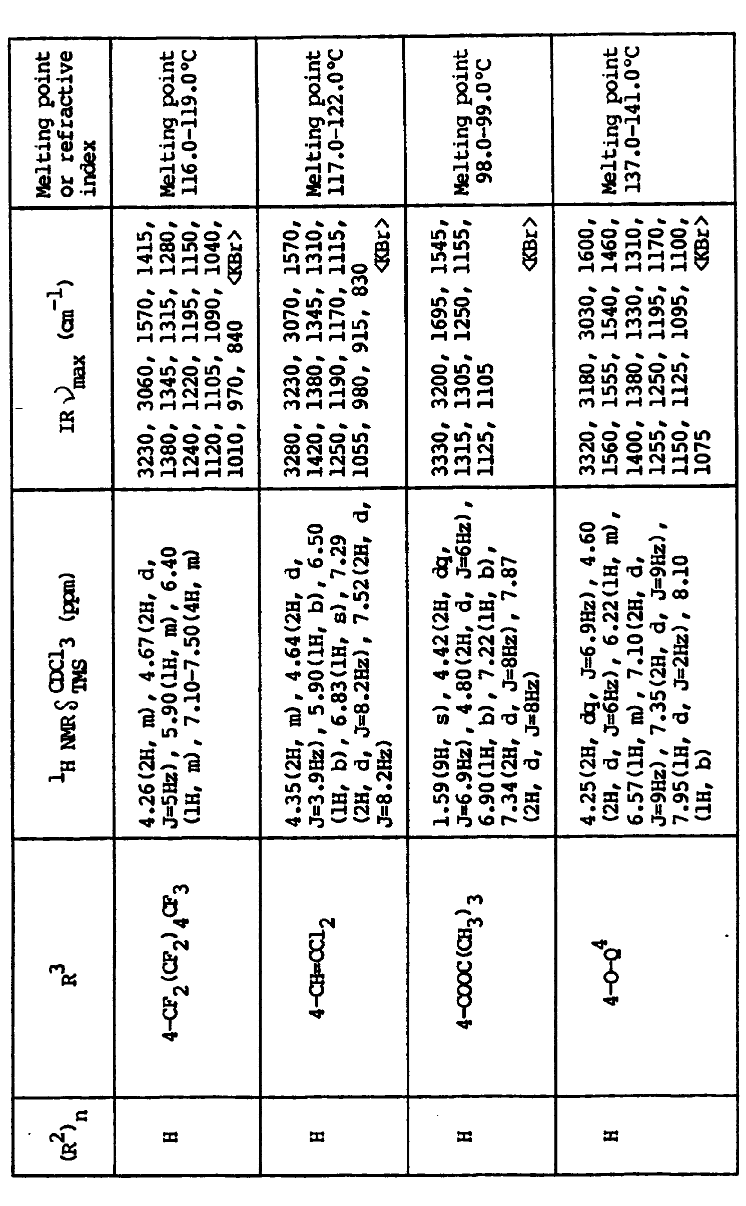

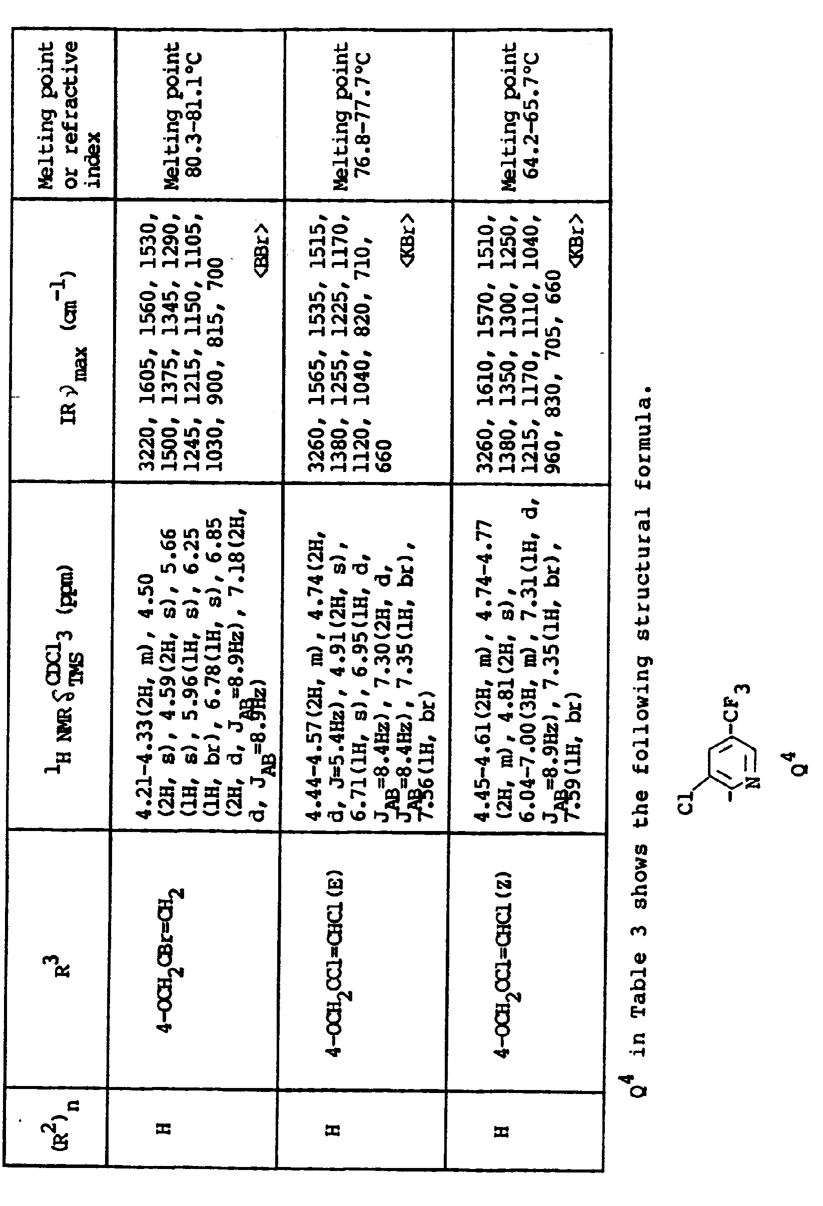

The following Formulation Examples illustrate agents comprising the compounds of geneal formula (I) produced by this invention as active ingredients and the method of producing them. The invention, however, is not limited to these examples.

### FORMULATION EXAMPLE 1

### Emulsifiable concentrate:-

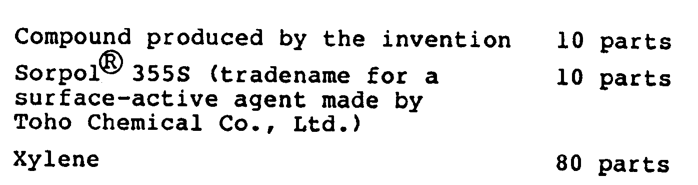

The above ingredients were mixed to form an emulsifiable concentrate.

### FORMULATION EXAMPLE 2

### Wettable powder:-

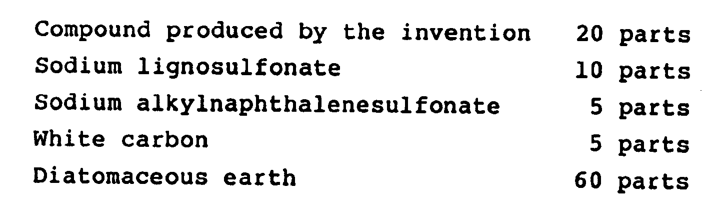

The above ingredients were mixed and pulverized uniformly to form a wettable powder.

### FORMULATION EXAMPLE 3

### Dust:-

Three parts of a compound in accordance with this invention was dissolved in 10 parts of acetone, and 97 parts of clay was added. Acetone was then evaporated to give a dust.

### FORMULATION EXAMPLE 4

### Granules:-

Three parts of a compound produced by the invention, 1 part of sodium lignosulfonate, 20 parts of talc and 76 parts of bentonite were mixed, and kneaded with a moderate amount of water. The mixture was granulated and dried to give granules.

### FORMULATION EXAMPLE 5

### Bait:-

One part of a compound in accordance with this invention, 5 parts of sugar, 50 parts of wheat bran, 20 parts of rice bran and 24 parts of wheat flour were mixed and kneaded with a moderate amount of water. The mixture was then granulated and dried to give a bait.

The following Test Examples illustrate the superior insecticidal activity of the compounds of this invention. All tests were conducted through three replicates, and the results were shown by averages of the results obtained.

### TEST EXAMPLE 1

### Effect against tobacco cutworm:-

An emulsifiable concentrate prepared in accordance with Formulation Example 1 was diluted with water to a concentration of 500 ppm and 50 ppm. Cabbage leaves were immersed in the emulsions, and then air-dried. The treated leaves were transferred to a plastic cup and ten 2nd-instar larvae of tobacco cutworm were caused to feed on the treated leaves. Five days later, the mortality (%) of the insects was examined. The results are shown in Table 4.

It is seen from Table 4 that the compounds of this invention have stronger insecticidal activity than known comparative compounds of a similar structure.

### TEST EXAMPLE 2

### Effect against cabbage moth:-

An emulsifiable concentrate prepared in accordance with Formulation Example 1 was diluted with water to a concentration of 500 ppm and 50 ppm, and sprayed by a hand sprayer onto cabbage seedlings (5- to 6-leaf stage) in pots to such an extent that the chemical lightly trickled over the leaves. After air drying, the leaves were cut off and put in a plastic cup. Ten 2nd-instar larvae of cabbage moth were caused to feed on the treated leaves, and five days later, the mortality of the insects was examined. The results are shown in Table 5.

Table 5 shows that the compounds of this invention have stronger insecticidal activity than known comparative compounds of a similar structure.

### TEST EXAMPLE 3

### Effect against small brown planthopper:-

An emulsifiable concentrate prepared in accordance with Formulation Example 1 was diluted with water to a concentration of 500 ppm and 50 ppm, and sprayed by a hand sprayer onto 5 to 6 rice seedlings (3-leaf stage) to such an extent that the chemical lightly trickled over the seedlings. After air drying, the rice seedlings were held in a plastic cylinder. Ten last-instar larvae of small brown planthopper about one day after ecdysis were inoculated in the rice seedlings. The cylinder was maintained at 25 °C for 16 hours under bright conditions and for 8 hours under dark conditions. Five days later, the mortality of the insects was examined. The results are shown in Table 6.

Table 6 shows that the compounds of this invention have equivalent or stronger insecticidal activity to or than the known comparative compounds of a similar structure.

### TEST EXAMPLE 4

### Effect against two-spotted spider mite:-

An emulsifiable concentrate prepared by Formulation Example 1 was diluted with water to a concentration of 50 ppm, and sprayed perpendicularly onto 30 to 50 larvae of two-spotted spider mites obtained by incubating spider mite eggs on kidney bean leaf discs having a diameter of 3 cm and placed on wet adsorbent cotton in such an amount that the amount of the chemical on the surfaces of the leaf discs corresponded to about 2 microliters/cm². The treated mite larvae were placed in an incubator at 25 °C, and four days later, the mortality of the insects was examined. The results are shown in Table 7.

It is seen from Table 7 that the compounds of this invention have equivalent or stronger acaricidal activity to or than the known comparative compounds having a similar structure.

The above Test Examples demonstrate that the compounds of this invention have stronger insecticidal activity on lepidopterous insect pests than the known comparative compounds of a similar structure, and equivalent or stronger insecticidal activity on hemipterous insect pests to or than the known comparative compounds of a similar structure; and also that the compounds of this invention have stronger acaricidal activity against mites than these comparavive compounds.

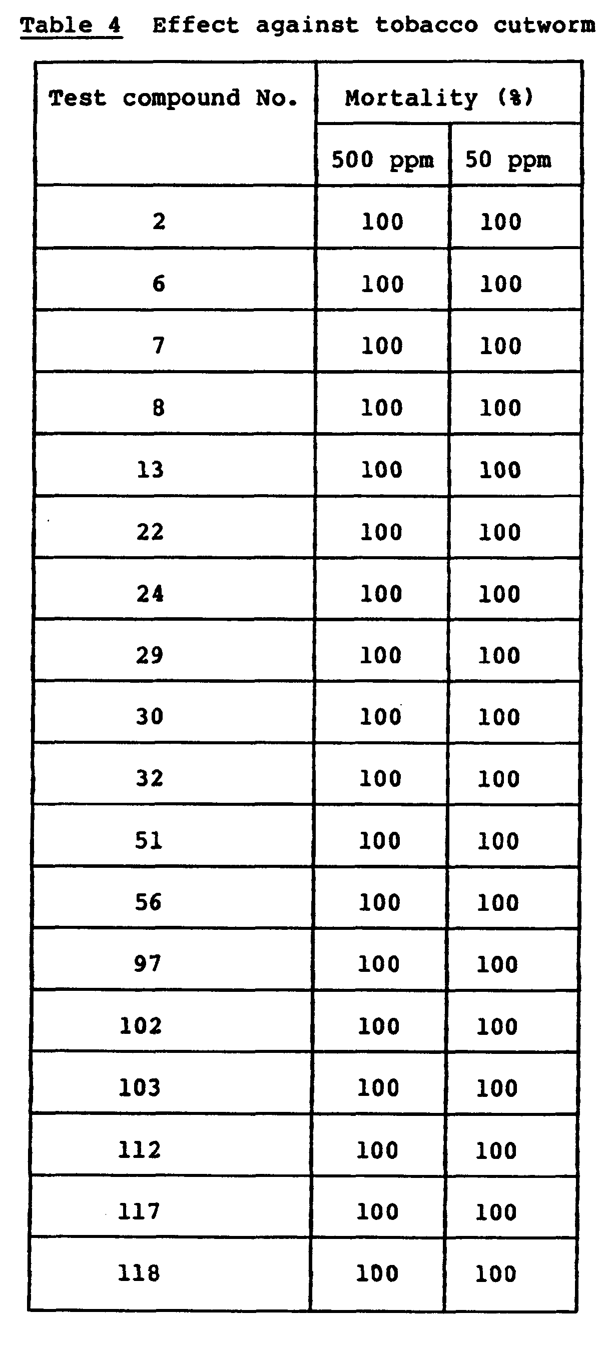

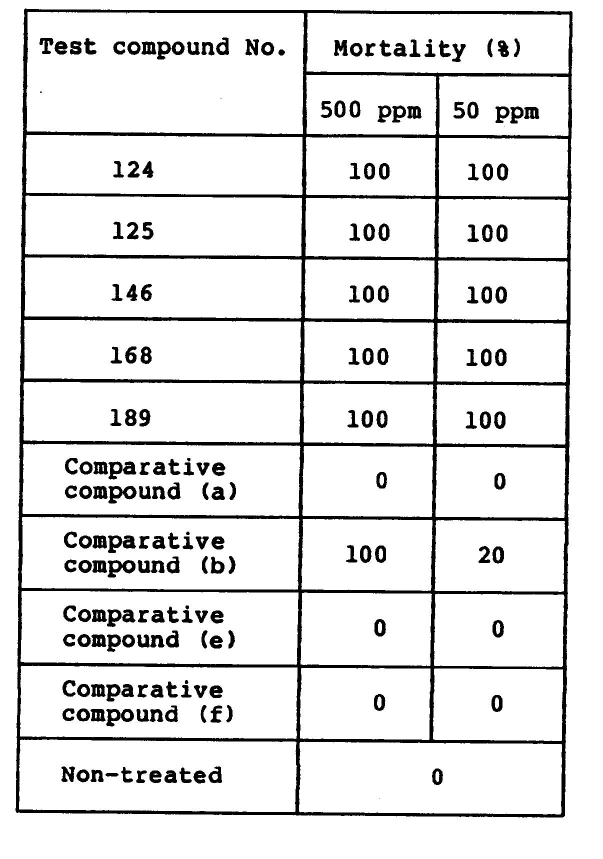

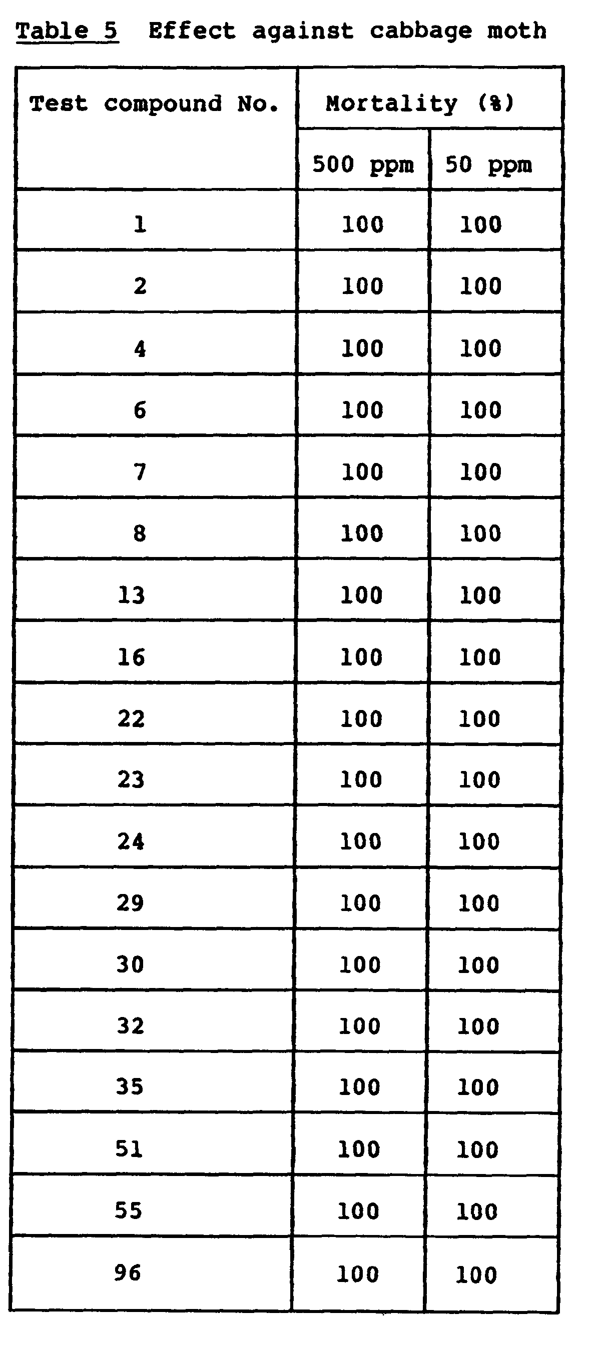

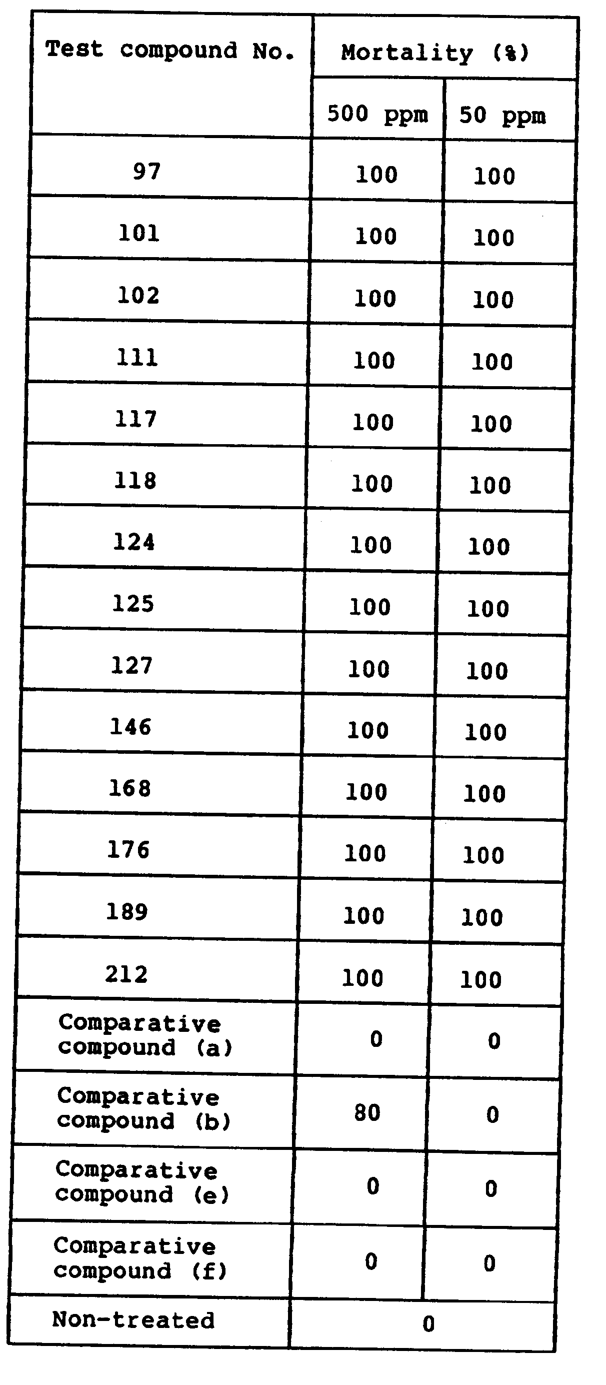

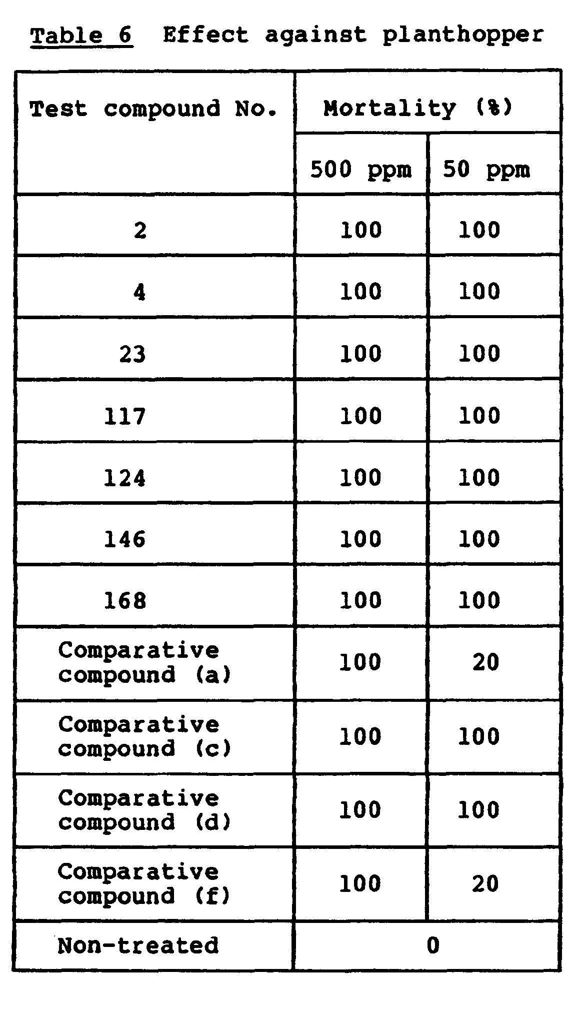

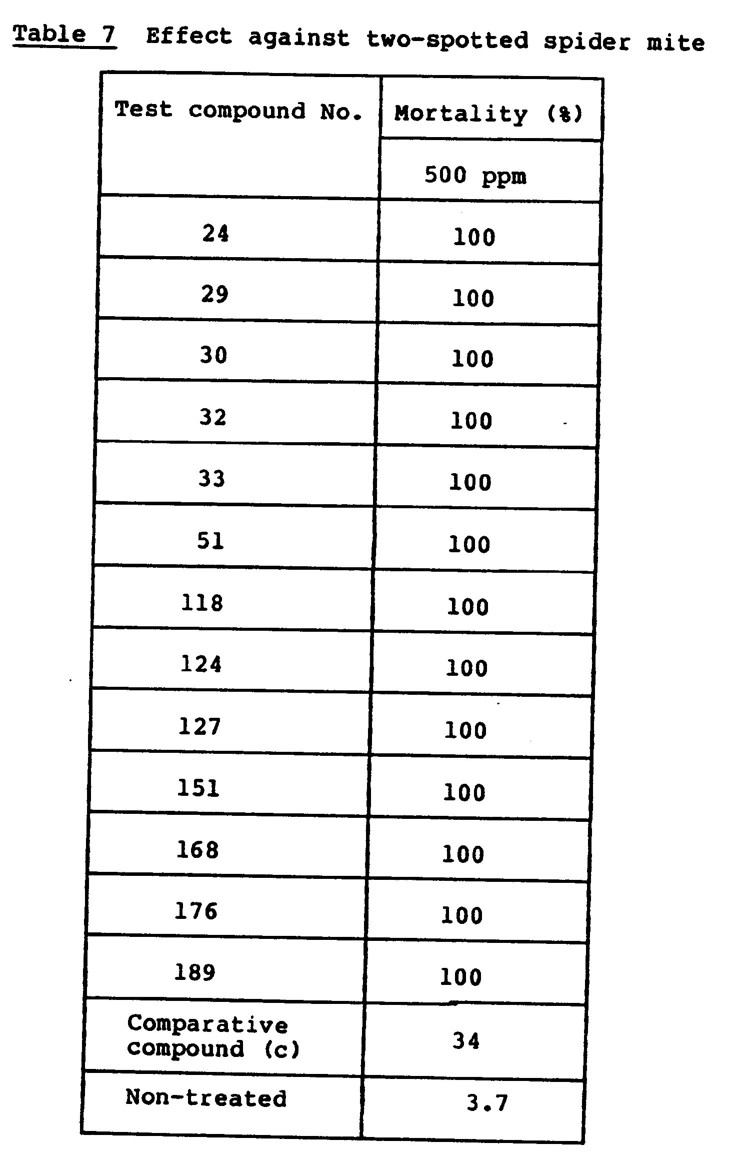

Comparative compound (a) is a compound of the folllwoing formula described in Japanese Laid-Open Patent Publication No. 140577/1986. 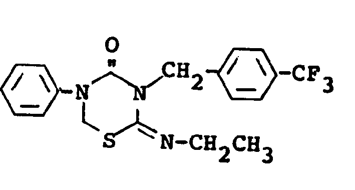 Comparative compound (b) is dichlorovos (DDVP) of the following formula:

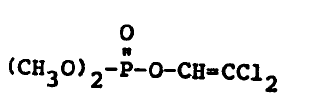

Comparative compound (c) is diazinone of the following formula:

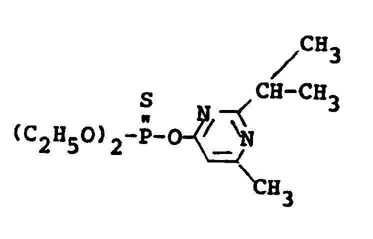

Comparative compound (d) is pyridafenthion of the following formula:

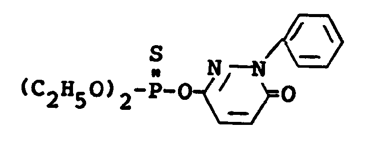

Comparative compound (e) is a compound of the following formula described in Japanese Laid-Open Patent Publication No. 3083/1979.

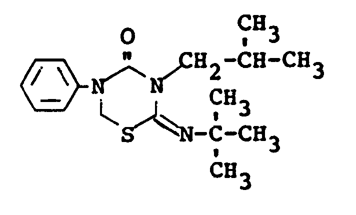

Comparative compound (f) is a compound of the following formula described in Japanese Laid-Open Patent Publication No. 140577/1986.

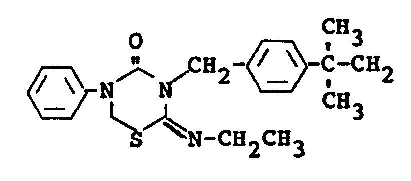

As is clearly from the foregoing statement, the tetrahydro-1,3,5-thiadiazin-4-ones of formula (I) and their salts show an excellent controlling effect on pests. Furthermore, agricultural chemical of this invention comprising the tetrahydro-1,3,5-thiadiazin-4-one of general formula (I) or its salt has excellent properties as an insecticidal and acaricidal agent and is useful.

## Claims (Claims for the following Contracting State(s) : s:CH,DE,FR,G8,IT,LI,NL)

1. A tetrahydro-1,3,5-thiadiazin-4-one of the following general formula (I) 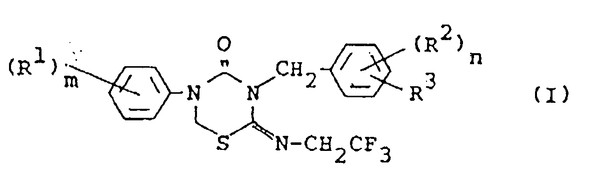 wherein each of R¹ and R² represents a halogen atom or an alkyl group having 1 to 4 carbon atoms, R³ represents a haloalkyloxy group having 1 to 4 carbon atoms, a haloalkenyloxy group having 2 to 4 carbon atoms, a haloalkyltio group having 1 to 4 carbon atoms, a haloalkyl group having 2 to 8 carbon atoms, a haloalkenyl group having 2 to 8 carbon atoms, an alkyloxycarbonyl group having 2 to 8 carbon atoms, phenoxycarbonyl, 2-chlorophenoxycarbonyl, 3-chlorophenoxycarbonyl, 4-chlorophenoxycarbonyl, 2-fluorophenoxycarbonyl, 3-fluorophenoxycarbonyl, 4-fluorophenoxycarbonyl, 2,4-dichlorophenoxycarbonyl, 3,4-dichlorophenoxycarbonyl, 3,5-dichlorophenoxycarbonyl, 2,4-difluorophenoxycarbonyl, 2,6-difluorophenoxycarbonyl, 4-trifluoro- methylphenoxycarbonyl, 4-trifluoromethoxyphenoxycarbonyl, 4-methoxyphenoxycarbonyl, 2-methylphenoxycarbonyl, 3-methylphenoxycarbonyl, 4-methylphenoxycarbonyl, 2,4-dimethylphenoxycarbonyl, 4-t-butylphenoxycarbonyl, 3-chloro-pytidyloxy, 3,5-dichloro-2-pyridyloxy, 5-trifluoromethyl-pyridyloxy and 3-chloro-5-trifluoromethyl-pyridyloxy groups at 4-position m is 0, 1, 2 or 3, and n is 0, 1, 2 or 3, or a salt thereof.

2. The compound of claim 1 in which m in general formula (I) is 0.

3. The compound of claim 1 in which R¹ in general formula (I) is a halogen atom or an alkyl group having 1 to 4 carbon atoms

4. The compound of claim 3 in which the halogen atom is a fluorine atom.

5. The compound of claim 4 in which the halogen atom is substituted at the 2-position.

6. The compound of claim 3 in which the alkyl group is a methyl group.

7. The compound of claim 6 in which the methyl group is substituted at the 3-position.

8. The compound of claim 6 in which the methyl group is substituted at the 4-position.

9. The compound of any of claims 1 to 8 in which R³ in general formula (1) is a trifluoromethoxy group.

10. The compound of any one of claims 1 to 8 in which R³ in general formula (I) is a pentafluoroethyl group.

11. A process for producing a tetrahydro-1,3,5-thiadiazin-4-one of the following general formula (I) 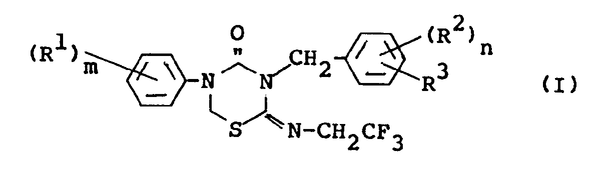 wherein R¹, R², R³, n and m are defined as in claim 1, or a salt thereof, which comprises reacting a compound represented by the following general formula (II) 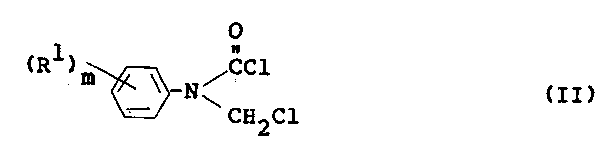 wherein R¹ and m are as defined above, with a compound of the following general formula (III) 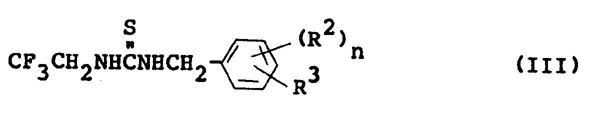 wherein R², R³ and n are as defined above.

12. An insecticidal and acaricidal composition comprising at least one tetrahydro-1,3,5-thiadiazin-4-one represented by the following general formula (I) 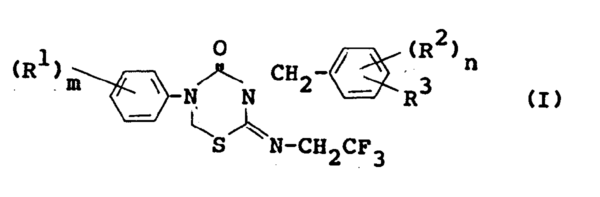 wherein R¹, R², R³, n and m are defined as in claim 1, or a salt thereof.

13. The insecticidal and acaricidal composition of claim 12 in which m in general formula (I) is 0.

14. The insecticidal and acaricidal composition of claim 12 in which R¹ in general formula (I) is a halogen atom or an alkyl group having 1 to 4 carbon atoms.

15. The insecticidal and acaricidal composition of claim 14 in which the halogen atom is a fluorine atom.

16. The insecticidal and acaricidal composition of claim 15 in which the fluorine atom is substituted at the 2-position.

17. The insecticidal and acaricidal composition of claim 14 in which the alkyl group is a methyl group.

18. The insecticidal and acaricidal composition of claim 17 in which the methyl group is substituted at the 3-position.

19. The insecticidal and acaricidal composition of claim 17 in which the methyl group is substituted at the 4-position.

20. The insecticidal and acaricidal composition of any-one of claims 12 to 19 in which R³ in general formula (I) is a trifluoromethoxy group.

21. The insecticidal and acaricidal composition of anyone of claima 12 to 19 in which R³ in general formula (I) is a pentafluoroethyl group.

## Claims (Claims for the following Contracting State(s) : ES)

1. A process for preparing a tetrahydro-1,3,5-thiadiazin-4-one of the following general formula (I) 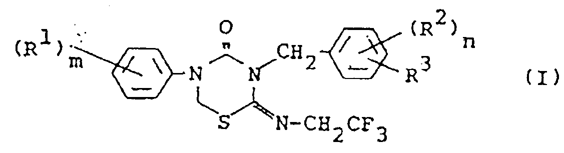 wherein each of R¹ and R² represents a halogen atom or an alkyl group having 1 to 4 carbon atoms, R³ represents a haloalkyloxy group having 1 to 4 carbon atoms, a haloalkenyloxy group having 2 to 4 carbon atoms, a haloalkyltio group having 1 to 4 carbon atoms, a haloalkyl group having 2 to 8 carbon atoms, a haloalkenyl group having 2 to 8 carbon atoms, an alkyloxycarbonyl group having 2 to 8 carbon atoms, phenoxycarbonyl, 2-chlorophenoxycarbonyl, 3-chlorophenoxycarbonyl, 4-chlorophenoxycarbonyl, 2-fluorophenoxycarbonyl, 3-fluorophenoxycarbonyl, 4-fluorophenoxycarbonyl, 2,4-dichlorophenoxycarbonyl, 3,4-dichlorophenoxycarbonyl, 3,5-dichlorophenoxycarbonyl, 2,4-difluorophenoxycarbonyl, 2,6-difluorophenoxycarbonyl, 4-trifluoro- methylphenoxycarbonyl, 4-trifluoromethoxyphenoxycarbonyl, 4-methoxyphenoxycarbonyl, 2-methylphenoxycarbonyl, 3-methylphenoxycarbonyl, 4-methylphenoxycarbonyl, 2,4-dimethylphenoxycarbonyl, 4-t-butylphenoxycarbonyl, 3-chloro-pyridyloxy, 3,5-dichloro-2-pyridyloxy, 5-trifluoromethyl-pyridyloxy and 3-chloro-5-trifluoromethyl-pyridyloxy groups at 4-position m is 0, 1, 2 or 3, and n is 0, 1, 2 or 3, or a salt thereof, which comprises reacting a compound represented by the following general formula (II) 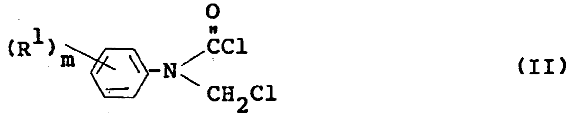 wherein R¹ and m axe as defined above, with a compound of the following general formula (III) 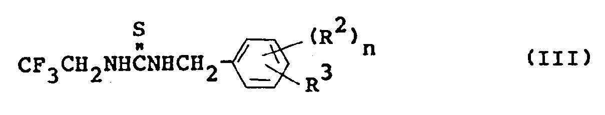 wherein R², R³ and n ate as defined above.

2. The process of claim 1 for preparing a compound of general formula (I) in which m is 0.

3. The process of claim 1 for preparing a compound of general formula (I) in which R¹ is a halogen atom or an alkyl group having 1 to 4 carbon atoms

4. The process of claim 3 in which the halogen atom is a fluorine atom.

5. The process of claim 4 in which the halogen atom is substituted at the 2-position.

6. The process of claim 3 in which the alkyl group is a methyl group.

7. The process of claim 6 in which the methyl group is substituted at the 3-position.

8. The process of claim 6 in which the methyl group is substituted at the 4-position.

9. The process of any of claims 1 to 8 for prepaparing a compound of general formula (I) in which R³ is a trifluoromethoxy group.

10. The process of any one of claims 1 to 8 for preparing a compound of general formula (I) in which R³ is a pentafluoroethyl group.

11. An insecticidal and acaricidal composition comprising at least one tetrahydro-1,3,5-thiadiazin-4-one represented by the following general formula (I) 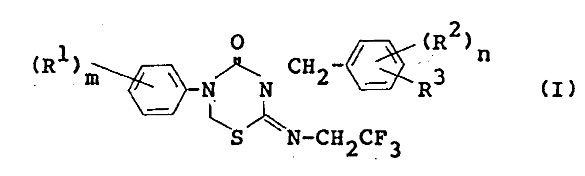 wherein R¹, R², R³, n and m are defined as in claim 1, or a salt thereof.

12. The insecticidal and acaricidal composition of claim II in which m in general formula (I) is 0.

13. The insecticidal and acaricidal composition of claim 11 in which R¹ in general formula (I) is a halogen atom or an alkyl group having 1 to 4 carbon atoms.

14. The insecticidal and acaricidal composition of claim 13 in which the halogen atom is a fluorine atom.

15. The insecticidal and acaricidal composition of claim 14 in which the fluorine atom is substituted at the 2-position.

16. The insecticidal and acaricidal composition of claim 13 in which the alkyl group is a methyl group.

17. The insecticidal and acaricidal composition of claim 16 in which the methyl group is substituted at the 3-position.

18. The insecticidal and acaricidal composition of claim 16 in which the methyl group is substituted at the 4-position.

19. The insecticidal and acaricidal composition of anyone of claims 11 to 18 in which R³ in general formula (I) is a trifluoromethoxy group.

20. The insecticidal and acaricidal composition of anyone of claims 11 to 18 in which R³ in general formula (I) is a pentafluoroethyl group.

## Patentansprüche (Patentansprüche für folgende(n) Vertragsstaat(en) : CH, DE, FR, GB, IT, LI, NL)

1. Tetrahydro-1,3,5-thiadiazin-4-on der folgenden allgemeinen Formel (I) 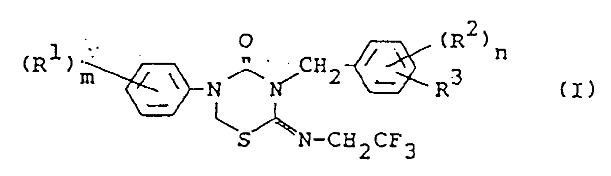 worin ein jedes von R¹ und R² für ein Halogenatom oder eine Alkylgruppe mit 1 bis 4 Kohlenstoffatomen steht, R³ eine Haloalkoxygruppe mit 1 bis 4 Kohlenstoffatomen, eine Haloalkenyloxygruppe mit 2 bis 4 Kohlenstoffatomen, eine Haloalkylthiogruppe mit 1 bis 4 Kohlenstoffatomen, eine Haloalkylgruppe mit 2 bis 8 Kohlenstoffatomen, eine Haloalkenylgruppe mit 2 bis 8 Kohlenstoffatomen, eine Alkyloxycarbonylgruppe mit 2 bis 8 Kohlenstoffatomen, Phenoxycarbonyl-, 2-Chlorphenoxycarbonyl-, 3-chlorphenoxycarbonyl-, 4-Chlorphenoxycarbonyl-, 2-Fluorphenoxycarbonyl-, 3-Fluorphenoxycarbonyl-, 4-Fluorphenoxycarbonyl-, 2,4-Dichlorphenoxycarbonyl-, 3,4-Dichlorphenoxycarbonyl-, 3,5-Dichlorphenoxycarbonyl-, 2,4-Difluorphenoxycarbonyl-, 2,6-Difluorphenoxycarbonyl-, 4-Trifluormethylphenoxycarbonyl-, 4-Trifluormethoxyphenoxycarbonyl-, 4-Methoxyphenoxycarbonyl-, 2-Methylphenoxycarbonyl-, 3-Methyl-phenoxycarbonyl-, 4-Methylphenoxycarbonyl-, 2,4-Dimethylphenoxycarbonyl-, 4-ter.-Butylphenoxycarbonyl-, 3-Chlorpyridyloxy-, 3,5-Dichlor-2-pyridyloxy-, 5-Tri-fluormethylpyridyloxy- und 3-Chlor-5-trifluormethylpyridyloxy-Gruppen in der 4-Stellung bedeutet, m für 0, 1, 2 oder 3 steht und n für 0, 1, 2 oder 3 steht, oder ein Salz hiervon.

2. Verbindung gemäß Anspruch 1, worin m in der allgemeinen Formel (I) für 0 steht.

3. Verbindung gemäß Anspruch 1, worin R¹ in der allgemeinen Formel (I) ein Halogenatom oder eine Alkylgruppe mit 1 bis 4 Kohlenstoffatomen ist.

4. Verbindung gemäß Anspruch 3, worin das Halogenatom ein Fluoratom ist.

5. Verbindung gemäß Anspruch 4, worin das Halogenatom als Substituent in der 2-Stellung vorliegt.

6. Verbindung gemäß Anspruch 3, worin die Alkylgruppe eine Methylgruppe ist.

7. Verbindung gemäß Anspruch 6, worin die Methylgruppe als Substituent in der 3-Stellung vorliegt.

8. Verbindung gemäß Anspruch 6, worin die Methylgruppe als Substituent in der 4-Stellung vorliegt.

9. Verbindung gemäß einem der Ansprüche 1 bis 8, worin R³ in der allgemeinen Formel (I) eine Trifluormethoxygruppe ist.

10. Verbindung gemäß einem der Ansprüche 1 bis 8, worin R³ in der allgemeinen Formel (I) eine Pentafluorethylgruppe ist.

11. Verfahren zur Herstellung eines Tetrahydro-1,3,5-thiadiazin-4-ons der folgenden allgemeinen Formel (I) 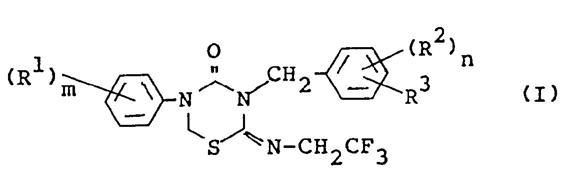 worin R¹, R², R³, n und m wie in Anspruch 1 definiert sind, oder eines Salzes hiervon, das die Umsetzung einer Verbindung der folgenden allgemeinen Formel (11) 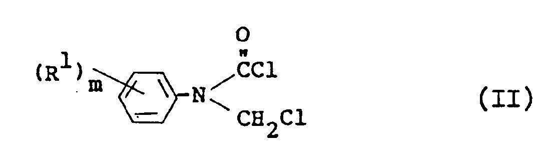 worin R¹ und m wie vorstehend definiert sind,mit einer Verbindung der folgenden allgemeinen Formel (111) 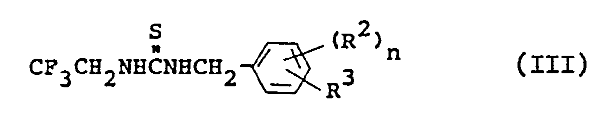 worin R², R³ und n wie vorstehend definiert sind, umfaßt.

12. Insektizide und akarizide Zusammensetzung, enthaltend zumindest ein Tetrahydro-1,3,5-thiadiazin-4- on der folgenden allgemeinen Formel (I) 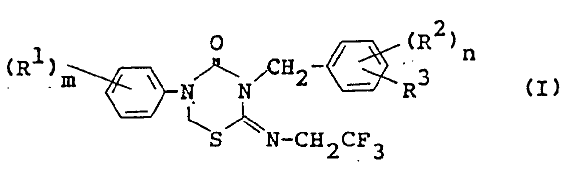 worin R¹, R², R³, n und m wie in Anspruch 1 definiert sind, oder ein Salz hiervon.

13. Insektizide und akarizide Zusammensetzung gemäß Anspruch 12, worin m in der allgemeinen Formel (I) für 0 steht.

14. Insektizide und akarizide Zusammensetzung gemäß Anspruch 12, worin R¹ in der allgemeinen Formel (I) ein Halogenatom oder eine Alkylgruppe mit 1 bis 4 Kohlenstoffatomen ist.

15. Insektizide und akarizide Zusammensetzung gemäß Anspruch 14, worin das Halogenatom ein Fluoratom ist.

16. Insektizide und akarizide Zusammensetzung gemäß Anspruch 15, worin das Fluoratom als Substituent in der 2-Stellung vorliegt.

17. Insektizide und akarizide Zusammensetzung gemäß Anspruch 14, worin die Alkylgruppe eine Methylgruppe ist.

18. Insektizide und akarizide Zusammensetzung gemäß Anspruch 17, worin die Methylgruppe als Substituent in der 3-Stellung vorliegt.

19. Insektizide und akarizide Zusammensetzung gemäß Anspruch 17, worin die Methylgruppe als Substituent in der 4-Stellung vorliegt.

20. Insektizide und akarizide Zusammensetzung gemäß einem der Ansprüche 12 bis 19, worin R³ in der allgemeinen Formel (I) eine Trifluormethoxygruppe ist.

21. Insektizide und akarizide Zummanensetzung gemäß einem der Ansprüche 12 bis 19, worin R³ in der allgemeinen Formel (I) eine Pentafluorethylgruppe ist.

## Patentansprüche (Patentansprüche für folgende(n) Vertragsstaat(en) : ES)

1. Verfahren zur Herstellung eines Tetrahydro-1,3,5-thiadiazin-4-ons der folgenden allgemeinen Formel (I) 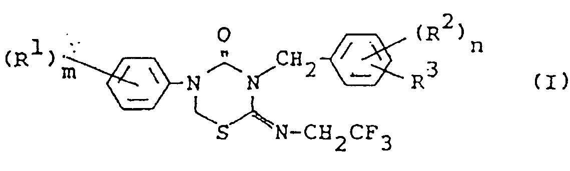 worin ein jedes von R¹ und R² für ein Halogenatom oder eine Alkylgruppe mit 1 bis 4 Kohlenstoffatomen steht, R³ eine Haloalkoxygruppe mit 1 bis 4 Kohlenstoffatomen, eine Haloalkenyloxygruppe mit 2 bis 4 Kohlenstoffatomen, eine Haloalkylthiogruppe mit 1 bis 4 Kohlenstoffatomen, eine Haloalkylgruppe mit 2 bis 8 Kohlenstoffatomen, eine Haloalkenylgruppe mit 2 bis 8 Kohlenstoffatomen, eine Alkyloxycarbonylgruppe mit 2 bis 8 Kohlenstoffatomen, Phenoxycarbonyl-, 2-Chlorphenoxycarbonyl-, 3-chlorphenoxycarbonyl-, 4-Chlorphenoxycarbonyl-, 2-Fluorphenoxycarbonyl-, 3-Fluorphenoxycarbonyl-, 4-Fluorphenoxycarbonyl-, 2,4-Dichlorphenoxycarbonyl-, 3,4-Dichlorphenoxycarbonyl-, 3,5-Dichlorphenoxycarbonyl-, 2,4-Difluorphenoxycarbonyl-, 2,6-Difluorphenoxycarbonyl-, 4-Trifluormethylphenoxycarbonyl-, 4-Trifluormethoxyphenoxycarbonyl-, 4-Methoxyphenoxycarbonyl-, 2-Methylphenoxycarbonyl-, 3-Methyl-phenoxycarbonyl-, 4-Methylphenoxycarbonyl-, 2,4-Dimethylphenoxycarbonyl-, 4-ter.-Butylphenoxycarbonyl-, 3-Chlorpyridyloxy-, 3,5-Dichlor-2-pyridyloxy-, 5-Tri-fluormethylpyridyloxy- und 3-Chlor-5-trifluormethylpyridyloxy-Gruppen in der4-Stellung bedeutet, m für 0, 1, 2 oder 3 steht und n für 0, 1, 2 oder 3 steht, oder ein Salz hiervon, das die Umsetzung einer Verbindung der folgenden allgemeinen Formel (II) 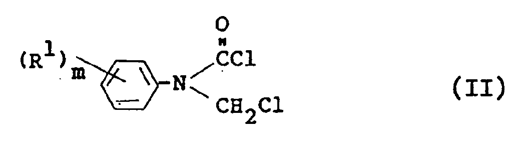 worin R¹ und m wie vorstehend definiert sind,mit einer Verbindung der folgenden allgemeinen Formel (III) 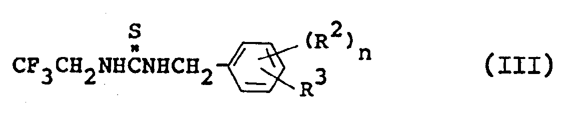 worin R², R³ und n wie vorstehend definiert sind, umfaßt.

2. Verfahren gemäß Anspruch 1 zur Herstellung einer Verbindung der allgemeinen Formel (1), worin m für 0 steht.

3. Verfahren gemäß Anspruch 1 zur Herstellung einer Verbindung der allgemeinen Formel (1), worin R¹ ein Halogenatom oder eine Alkylgruppe mit 1 bis 4 Kohlenstoffatomen ist.

4. Verfahren gemäß Anspruch 3, worin das Halogenatom ein Fluoratom ist.

5. Verfahren gemäß Anspruch 4, worin das Halogenatom als Substituent in der 2-Stellung vorliegt.

6. Verfahren gemäß Anspruch 3, worin die Alkylgruppe eine Methylgruppe ist.

7. Verfahren gemäß Anspruch 6, worin die Methylgruppe als Substituent in der 3-Stellung vorliegt.

8. Verfahren gemäß Anspruch 6, worin die Methylgruppe als Substituent in der 4-Stellung vorliegt.

9. Verfahren gemäß einem derAnsprüche 1 bis 8 zur Herstellung einer Verbindung derallgemeinen Formel (1), worin R³ eine Trifluormethoxygruppe bedeutet.

10. Verfahren gemäß einem der Ansprüche 1 bis 8 zur Herstellung einer Verbindung der allgemeinen Formel (I), worin R³ für eine Pentafluorethylgruppe steht.

11. Insektizide und akarizide Zusammensetzung, enthaltend zumindest ein Tetrahydro-1,3,5-thiadiazin-4- on der folgenden allgemeinen Formel (I) 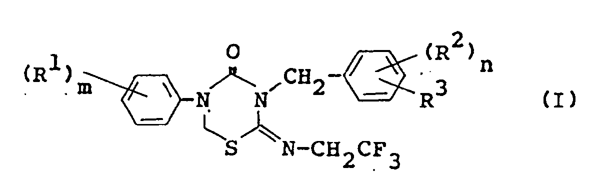 worin R¹, R², R³, n und m wie in Anspruch 1 definiert sind, oder ein Salz hiervon.

12. Insektizide und akarizide Zusammensetzung gemäß Anspruch 11, worin m in der allgemeinen Formel (I) für 0 steht.

13.Insektizide und akarizide Zusammensetzung gemäß Anspruch 11, worin R¹ in der allgemeinen Formel (I) ein Halogenatom oder eine Alkylgruppe mit 1 bis 4 Kohlenstoffatoment ist.

14. Insektizide und akarizide Zusammensetzung gemäß Anspruch 13, worin das Halogenatom ein Fluoratom ist.

15. Insektizide und akarizide Zusammensetzung gemäßAnspruch 14, worin das Fluoratom als Substituent in der 2-Stellung vorliegt.

16. Insektizide und akarizide Zusammensetzung gemäß Anspruch 13, worin die Alkylgruppe eine Methylgruppe ist.

17. Insektizide und akarizide Zusammensetzung gemäß Anspruch 16, worin die Methylgruppe als Substituent in der 3-Stellung vorliegt.

18. Insektizide und akarizide Zusammensetzung gemäß Anspruch 16, worin die Methylgruppe als Substituent in der 4-Stellung vorliegt.

19. Insektizide und akarizide Zusammensetzung gemäß einem der Ansprüche 11 bis 18, worin R³ in der allgemeinen Formel (I) eine Trifluormethoxygruppe ist.

20. Insektizide und akarizide Zusammensetzung gemäß einem der Ansprüche 11 bis 18, worin R³ in der allgemeinen Formel (I) eine Pentafluorethylgruppe ist.

## Revendications (Revendications pour l'(les) Etat(s) contractant(s) suivant(s) : suivants:CH, DE, FR, GB, IT, LI, NL)

1. Une tétrahydro-1,3,5-thiadiazine-4-one répondant à la formule générale (I) suivante : 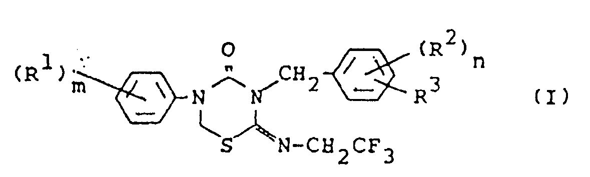 dans laquelle chacun de R¹ et de R² représente un atome d'halogène ou un groupe alcoyle ayant 1 à 4 atomes de carbone, R³ représente un groupe halogénoalcoyloxy ayant 1 à 4 atomes de carbone, un groupe halogénoalcényloxy ayant 2 à 4 atomes de carbone, un groupe halogénoalcoylthio ayant 1 à 4 atomes de carbone, un groupe halogénoalcoyle ayant 2 à 8 atomes de carbone, un groupe halogénoalcényle ayant 2 à 8 atomes de carbone, un groupe alcoyloxycarbonyle ayant 2 à 8 atomes de carbone, des groupes phénoxycar- bonylé, 2-chlorophénoxycarbonyle, 3-chlorophénoxycarbonyle, 4-chlorophénoxycarbonyle, 2-fluorophénoxy- carbonylé, 3-fluorophénoxycarbonyle, 4-fluorophénoxycarbonyle, 2,4-dichlorophénoxycarbonyle, 3,4-dichlorophénoxycarbonyle, 3,5-dichlorophénoxycarbonyle, 2,4-difluorophénoxycarbonyle, 2,6-difluorophénoxycarbonyle, 4-trifluorométhylphénoxycarbonyle, 4-trifluorométhoxyphénoxycarbonyle, 4-méthoxyphénoxycarbo- nyle, 2-méthylphénoxycarbonyle, 3-méthylphénoxycarbonyle, 4-méthylphénoxycarbonyle-2,4-diméthylphé- noxycarbonyle, 4-tert-butylphénoxycarbonyle, 3-chloropyridyloxy, 3,5-dichloro-2-pyridyloxy, 5-trifluorométhyl- pyridyloxy et 3-chloro-5-trifluorométhylpyridyloxy dans la position 4, m est 0, 1, 2 ou 3 et n est 0, 1, 2 ou 3, ou un de ses sels.

2. Le composé de la revendication 1, dans lequel m, dans la formule générale (I), est 0.

3. Le composé de la revendication 1, dans lequel R¹, dans la formule générale (I), est un atome d'halogène ou un groupe alcoyle ayant 1 à 4 atomes de carbone.

4. Le composé de la revendication 3, dans lequel l'atome d'halogène un un atome de fluor.

5. Le composé de la revendication 4, dans lequel l'atome d'halogène substitue la position 2.

6. Le composé de la revendication 3, dans lequel le groupe alcoyle est un groupe méthyle.

7. Le composé de la revendication 6, dans lequel le groupe méthyle substitue la position 3.

8. Le composé de la revendication 6, dans lequel le groupe méthyle substitue la position 4.

9. Le composé de l'une quelconque des revendications 1 à 8, dans lequel R³, dans la formule générale (I), est un groupe trifluorométhoxy.

10. Le composé de l'une quelconque des revendications 1 à 8, dans lequel R³, dans la formule générale (I), est un groupe pentafluoroéthyle.

11. Un procédé pour préparer une tétrahydro-1,3,5-thiadiazine-4-one répondant à la formule générale (I) suivante : 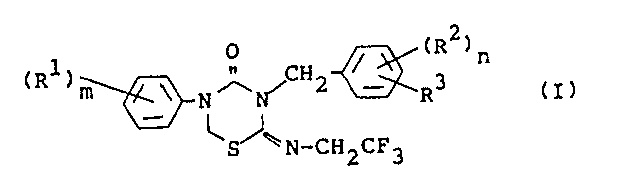 dans laquelle R¹, R², R³, n et m sont comme définis dans la revendication 1, ou un de ses sels, qui comprend la réaction d'un composé représenté par la formule générale (II) suivante 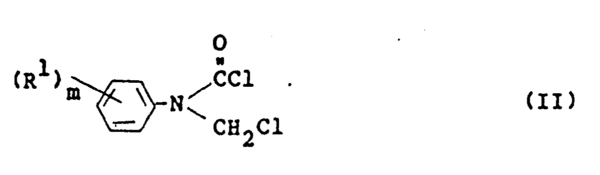 dans laquelle R¹ et m sont comme définis ci-dessus, avec un composé de formule générale (III) suivante 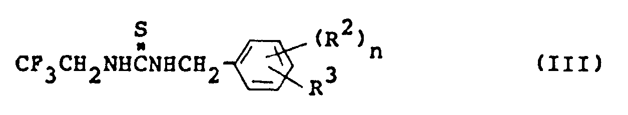 dans laquelle R², R³ et n sont comme définis ci-dessus.

12. Une composition insecticide et acaricide comprenant au moins une tétrahydro-1,3,5-thiadiazine-4-one représentée par la formule générale (I) suivante 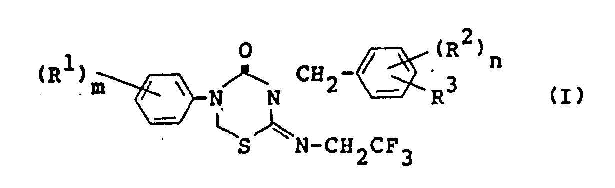 dans laquelle R¹, R², R³, n et m sont comme définis dans la revendication 1, ou un de ses sels.

13. La composition insecticide et acaricide de la revendication 12, dans laquelle m, dans la formule générale (I), est 0.

14. La composition insecticide et acaricide de la revendication 12, dans laquelle R¹, dans la formule générale (I), est un atome d'halogène ou un groupe alcoyle ayant 1 à 4 atomes de carbone.

15. La composition insecticide et acaricide de la revendication 14, dans laquelle l'atome d'halogène est un atome de fluor.

16. La composition insecticide et acaricide de la revendication 15, dans laquelle l'atome de fluor substitue la position 2.

17. La composition insecticide et acaricide de la revendication 14, dans laquelle le groupe alcoyle est un groupe méthyle.

18. La composition insecticide et acaricide de la revendication 17, dans laquelle le groupe méthyle substitue la position 3.

19. La composition insecticide et acaricide de la revendication 17, dans laquelle le groupe méthyle substitue la position 4.

20. La composition insecticide et acaricide de l'une quelconque des revendications 12 à 19, dans laquelle R³, dans la formule générale (I), est un groupe trifluorométhoxy.

21. La composition insecticide et acaricide de l'une quelconque des revendications 12 à 19, dans laquelle R³, dans la formule générale (I), est un groupe pentafluoroéthyle.

## Revendications (Revendications pour l'(les) Etat(s) contractant(s) suivant(s) : suivant: ES)

1. Un procédé pour préparer une tétrahydro-1,3,5-thiadiazine-4-one répondant à la formule générale (I) suivante : 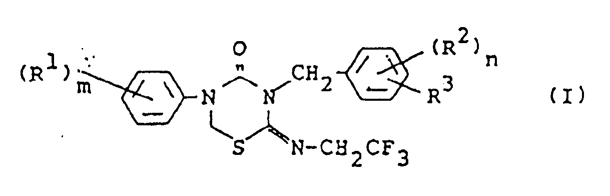 dans laquelle chacun de R¹ et de R² représente un atome d'halogène ou un groupe alcoyle ayant 1 à 4 atomes de carbone, R³ représente un groupe halogénoalcoyloxy ayant 1 à 4 atomes de carbone, un groupe halogénoalcényloxy ayant 2 :à 4 atomes de carbone, un groupe halogénoalcoylthio ayant 1 à 4 atomes de carbone, un groupe halogénoalcoyle ayant 2 à 8 atomes de carbone, un groupe halogénoalcényle ayant 2 à 8 atomes de carbone, un groupe alcoyloxycarbonyle ayant 2 à 8 atomes de carbone, des groupes phénoxycar- bonylé, 2-chlorophénoxycarbonyle, 3-chlorophénoxycarbonyle, 4-chlorophénoxycarbonyle, 2-fluorophénoxy- carbonylé, 3-fluorophénoxycarbonyle, 4-fluorophénoxycarbonyle, 2,4-dichlorophénoxycarbonyle, 3,4-dichlorophénoxycarbonyle, 3,5-dichlorophénoxycarbonyle, 2,4-difluorophénoxycarbonyle, 2,6-difluorophénoxycarbonyle, 4-trifluorométhylphénoxycarbonyle, 4-trifluorométhoxyphénoxycarbonyle, 4-méthoxyphénoxycarbo- nyle, 2-méthylphénoxycarbonyle, 3-méthylphénoxycarbonyle, 4-méthylphénoxycarbonyle, 2,4-diméthylphé- noxycarbonyle, 4-tert-butylphénoxycarbonyle, 3-chloropyridyloxy, 3,5-dichloro-2-pyridyloxy, 5-trifluorométhyl- pyridyloxy et 3-chloro-5-trifluorométhylpyridyloxy dans la position 4, m est 0, 1, 2 ou 3 et n est 0, 1, 2 ou 3, ou un de ses sels, qui comprend la réaction d'un composé représenté par la formule générale (II) suivante 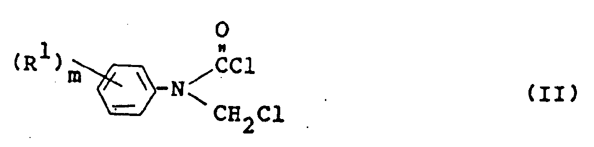 dans laquelle R¹ et m sont comme définis ci-dessus, avec un composé de formule générale (III) suivante 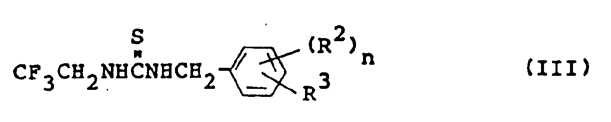 dans laquelle R², R³ et n sont comme définis ci-dessus.

2. Le procédé de la revendication 1 pour préparer un composé de formule générale (I) dans laquelle m est 0.

3. Le procédé de la revendication 1 pour préparer un composé de formule générale (I) dans laquelle R¹ est un atome d'halogène ou un groupe alcoyle ayant 1 à 4 atomes de carbone.

4. Le procédé de la revendication 3 où l'atome d'halogène est un atome de fluor.

5. Le procédé de la revendication 4 où l'atome d'halogène substitue la position 2.

6. Le procédé de la revendication 3 où le groupe alcoyle est un groupe méthyle.

7. Le procédé de la revendication 6 où le groupe méthyle substitue la position 3.

8. Le procédé de la revendication 6 où le groupe méthyle substitue la position 4.

9. Le procédé de l'une quelconque des revendications 1 à 8 pour la préparation d'un composé de formule générale (I) où R³ est un groupe trifluorométhoxy.

10. Le procédé de l'une quelconque des revendications 1 à 8 pour la préparation d'un composé de formule générale (I) où R³ est un groupe pentafluoroéthyle.

11. Une composition insecticide et acaricide comprenant au moins une tétrahydro-1,3,5-thiadiazine-4-one représentée par la formule générale (I) suivante 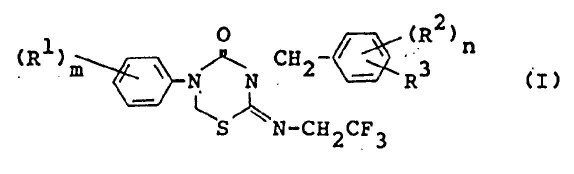 dans laquelle R¹, R², R³, n et m sont comme définis dans la revendication 1, ou un de ses sels.

12. La composition insecticide et acaricide de la revendication 11, dans laquelle m, dans la formule générale (I), est 0.

13. La composition insecticide et acaricide de la revendication 11, dans laquelle R¹, dans la formule générale (I), est un atome d'halogène ou un groupe alcoyle ayant 1 à 4 atomes de carbone.

14. La composition injecticide et acaricide de la revendication 13, dans laquelle l'atome d'halogène est un atome de fluor.

15. La composition insecticide et acaricide de la revendication 14, dans laquelle l'atome de fluor substitue la position 2.

16. La composition insecticide et acaricide de la revendication 13, dans laquelle le groupe alcoyle est un groupe méthyle.

17. La composition insecticide et acaricide de la revendication 16, dans laquelle le groupe méthyle substitue la position 3.

18. La composition insecticide et acaricide de la revendication 16, dans laquelle le groupe méthyle substitue la position 4.

19. La composition insecticide et acaricide de l'une quelconque des revendications 11 à 18, dans laquelle R³, dans la formule générale (I), est un groupe trifluorométhoxy.

20. La composition insecticide et acaricide de l'une quelconque des revendications 11 à 18, dans laquelle R³, dans la formule générale (I), est un groupe pentafluoroéthyle.
